# EUROPÄISCHE PATENTSCHRIFT

(11) **EP 0 777 658 B1**
(45) Veröffentlichungstag und Bekanntmachung des Hinweises auf die Patenterteilung: **15.12.1999**
(21) Anmeldenummer: 95923241.4
(22) Anmeldetag: 07.06.1995
(51) Int. Cl.: C07D 249/12, A01N 43/653

(54) **SUBSTITUIERTE TRIAZOLINONE ALS PFLANZENSCHUTZMITTEL**
SUBSTITUTED TRIAZOLINONES AS PLANT PROTECTIVE AGENTS
TRIAZOLINONES SUBSTITUEES UTILISEES SOUS FORME D'AGENTS PHYTOSANITAIRES

(30) Priorität: 16.08.1994 DE 4429006
(43) Veröffentlichungstag der Anmeldung: 11.06.1997
(73) Patentinhaber: BASF AKTIENGESELLSCHAFT, 67056 Ludwigshafen (DE)
(72) Erfinder: HEISTRACHER, Elisabeth, D-67061 Ludwigshafen (DE); VON DEM BUSSCHE-HÜNNEFELD, Christoph-Sweder, D-68199 Mannheim (DE); HAMPRECHT, Gerhard, D-69469 Weinheim (DE); KLINTZ, Ralf, D-67269 Grünstadt (DE); SCHÄFER, Peter, D-67308 Ottersheim (DE); WESTPHALEN, Karl-Otto, D-67346 Speyer (DE); GERBER, Matthias, D-67117 Limburgerhof (DE); WALTER, Helmut, D-67283 Obrigheim (DE)
(86) Internationale Anmeldenummer: EP9502167
(87) Internationale Veröffentlichungsnummer: WO9605179

(56) Entgegenhaltungen:
- WO-A-90/02120
- DE-A- 4 131 038

## Beschreibung

Die vorliegende Erfindung betrifft neue substituierte Triazolinone der allgemeinen Formel I 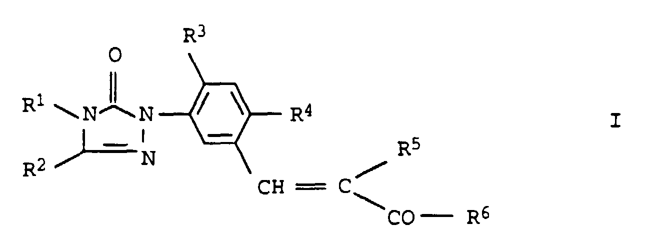 in der die Substituenten folgende Bedeutung haben:
- R¹: Wasserstoff, C₁-C₆-Alkyl oder C₁-C₆-Halogenalkyl;
- R²: Wasserstoff, C₁-C₆-Alkyl oder C₁-C₆-Halogenalkyl;
- R³: Wasserstoff oder Halogen;
- R⁴: Cyano, Halogen, C₁-C₆-Alkyl, C₁-C₆-Halogenalkyl, C₁-C₆-Alkoxy oder C₁-C₆-Halogenalkoxy;
- R⁵: Nitro, Cyano oder Halogen;
- R⁶: -OR⁷, -SR⁷, -N(R⁸)-R⁹ oder -N(R⁸)-OR¹⁰, wobei
- R⁷: für Wasserstoff, C₁-C₆-Alkyl, C₃-C₆-Alkenyl, C₃-C₆-Alkinyl, C₃-C₆-Cycloalkyl, C₁-C₆-Halogenalkyl, Cyano-C₁-C₆-alkyl, C₁-C₆-Alkoxy-C₁-C₆-alkyl, C₁-C₆-Alkylthio-C₁-C₆-alkyl, (C₁-C₆-Alkoxy)carbonyl-C₁-C₆-alkyl, (C₁-C₆-Alkylamino)carbonyl-C₁-C₆-alkyl, Di-(C₁-C₆-Alkyl)aminocarbonyl-C₁-C₆-alkyl, C₁-C₆-Alkyloximino-C₁-C₆-alkyl, Di-(C₁-C₆-alkoxy)-C₂-C₆-alkyl, Di-(C₁-C₆-alkylthio)-C₂-C₆-alkyl, C₃-C₆-Halogenalkenyl, Phenyl oder Benzyl, wobei die Phenylringe jeweils ein bis drei Reste tragen können, ausgewählt aus der Gruppe bestehend aus Cyano, Nitro, Halogen, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio und (C₁-C₆-Alkoxy)carbonyl;
- R⁸ und R⁹: unabhängig voneinander für (C₁-C₆-Alkyl)carbonyl, (C₁-C₆-Halogenalkyl)carbonyl oder eine der für R⁷ genannten Bedeutungen, oder
- R⁸ und R⁹: gemeinsam für eine vier- bis 6-gliedrige Kohlenstoffkette, in der eine nicht endständige Methyleneinheit durch Sauerstoff oder eine C₁-C₄-Alkyliminogruppe ersetzt sein kann und
- R¹⁰: für (C₁-C₆-Alkyl)carbonyl, (C₁-C₆-Halogenalkyl)carbonyl oder eine der für R⁷ genannten Bedeutungen stehen,
sowie die landwirtschaftlich brauchbaren Salze von I.

Außerdem betrifft die Erfindung
- die Verwendung der Verbindungen I als Herbizide und/oder zur Desikkation und/oder Defoliation von Pflanzen,
- herbizide Mittel und/oder Mittel zur Desikkation und/oder Defoliation von Pflanzen, welche die Verbindungen I als wirksame Substanzen enthalten,
- Verfahren zur Herstellung der Verbindungen I und von herbiziden Mitteln und/oder Mitteln zur Desikkation und/oder Defoliation von Pflanzen unter Verwendung der Verbindungen I, sowie
- Verfahren zur Bekämpfung von unerwünschtem Pflanzenwuchs und/oder zur Desikkation und/oder Defoliation von Pflanzen mit den Verbindungen I.

Herbizid wirksame Triazolinone sind bereits aus der WO 90/02120 und der WO 87/03782 bekannt. Ihre Wirkung ist jedoch nicht immer voll befriedigend.

Der Erfindung lag daher die Aufgabe zugrunde, Triazolinone mit verbesserten biologischen Eigenschaften bereitzustellen.

Demgemäß wurden die substituierten Triazolinone der Formel I gefunden. Ferner wurden herbizide Mittel gefunden, die die Verbindungen I enthalten und eine sehr gute herbizide Wirkung besitzen. Außerdem wurden Verfahren zur Herstellung dieser Mittel und Verfahren zur Bekämpfung von unerwünschtem Pflanzenwuchs mit den Verbindungen I gefunden.

Die erfindungsgemäßen Verbindungen I eignen sich des weiteren zur Defoliation und/oder Desikkation von Pflanzenteilen für z.B. Baumwolle, Kartoffel, Raps, Sonnenblume, Sojabohne oder Ackerbohnen, insbesondere für Baumwolle. Diesbezüglich wurden Mittel zur Desikkation und/oder Defoliation von Pflanzen, Verfahren zur Herstellung dieser Mittel und Verfahren zur Desikkation und/oder Defoliation von Pflanzen mit den Verbindungen I gefunden.

Die für die Substituenten R¹, R², R⁴ und R⁷ bis R¹⁰ oder als Reste an Phenylringen genannten organischen Molekülteile stellen - wie die Bedeutung Halogen - Sammelbegriffe für individuelle Auf zählungen der einzelnen Gruppenmitglieder dar. Sämtliche Kohlenstoffketten, also alle Alkyl-, Halogenalkyl-, Cyanoalkyl-, Alkoxy-, Halogenalkoxy-, Alkenyl-, Halogenalkenyl-, Alkinyl-, Alkoxycarbonyl-, Alkylthio- und Halogenalkylthio-Teile können geradkettig oder verzweigt sein, sofern nichts anderes angegeben ist. Mehrfach halogenierte Halogenalkyl-, Halogenalkoxy-, Halogenalkylthio- und Halogenalkenylreste können gleiche oder verschiedene Halogenatome tragen.

Im einzelnen stehen beispielsweise:
- Halogen für: Fluor, Chlor, Brom und Iod;
- C₁-C₆-Alkyl für: Methyl, Ethyl, n-Propyl, 1-Methylethyl, n-Butyl, 1-Methylpropyl, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, 1,1-Dimethylpropyl, 1,2-Dimethylpropyl, n-Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
- C₁-C₆-Halogenalkyl für: Chlormethyl, Dichlormethyl, Trichlormethyl, Fluormethyl, Difluormethyl, Trifluormethyl, Chorfluormethyl, Dichlorfluormethyl, Chlordifluormethyl, 2-Chlorethyl, 2-Fluorethyl, 2-Bromethyl, 2-Iodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, Pentafluorethyl, 2,2,2-Trichlorethyl, 3-Fluorpropyl, 2-Fluorpropyl, 2,2-Difluorpropyl, 2,3-Difluorpropyl, 3-Chlorpropyl, 2-Chlorpropyl, 2,3-Dichlorpropyl, 3-Brompropyl, 2-Brompropyl, 3,3,3-Trichlorpropyl, 3,3,3-Trifluorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl, Nonfluorbutyl, 5-Fluorpentyl, 5-Chlorpentyl, 5-Brompentyl, 5-Iodpentyl, Undecafluorpentyl, 6-Fluorhexyl, 6-Chlorhexyl, 6-Bromhexyl, 6-Jodhexyl und Dodecafluorhexyl;
- C₁-C₆-Alkoxy für Methoxy, Ethoxy, n-Propoxy, 1-Methylethoxy, n-Butoxy, 1-Methylpropoxy, 2-Methylpropoxy, 1,1-Dimethylethoxy, n-Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, n-Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy und 1-Ethyl-2-methylpropoxy;
- C₁-C₆-Halogenalkoxy für z.B. Difluormethoxy, Trifluormethoxy, Chlordifluormethoxy, Bromdifluormethoxy, 2-Fluorethoxy, 2,2-Difluorethoxy, 2,2,2-Trifluorethoxy, 2-Chlorethoxy, 2-Bromethoxy, 2-Iodethoxy, 2-Chlor-2-fluorethoxy, 2-Chlor-2,2-difluorethoxy, 2,2-Dichlor-2-fluorethoxy, 2,2,2-Trichlorethoxy, Pentafluorethoxy, 3-Fluorpropoxy, 2-Fluorpropoxy, 2,2-Difluorpropoxy, 2,3-Difluorpropoxy, 3-Chlorpropoxy, 2-Chlorpropoxy, 2,3-Dichlorpropoxy, 3-Brompropoxy, 2-Brom-propoxy, 3,3,3-Trichlorpropoxy, 3,3,3-Trifluorpropoxy, 2,2,3,3,3-Pentafluorpropoxy, Heptafluorpropoxy, 1-(Fluormethyl)-2-fluorethoxy, 1-(Chlormethyl)-2-chlorethoxy, 1-(Brommethyl)-2-bromethoxy, 4-Fluorbutoxy, 4-Chlorbutoxy, 4-Brombutoxy, 4-Iodbutoxy, Nonafluorbutoxy, 5-Fluorpentoxy, 5-Chlorpentoxy, 5-Brompentoxy, 5-Iodpentoxy, Undecafluorpentoxy, 6-Fluorhexoxy, 6-Chlorhexoxy, 6-Bromhexoxy, 6-Iodhexoxy und Dodecafluorhexoxy;
- C₁-C₆-Alkylcarbonyl für Methylcarbonyl, Ethylcarbonyl, Propylcarbonyl, 1-Methylethylcarbonyl, Butylcarbonyl, 1-Methylpropylcarbonyl, 2-Methylpropylcarbonyl, 1,1-Dimethylethylcarbonyl, Pentylcarbonyl, 1-Methylbutylcarbonyl, 2-Methylbutylcarbonyl, 3-Methylbutylcarbonyl, 1,1-Dimethylpropylcarbonyl, 1,2-Dimethylpropylcarbonyl, 2,2-Dimethylpropylcarbonyl, 1-Ethylpropylcarbonyl, Hexylcarbonyl, 1-Methylpentylcarbonyl, 2-Methylpentylcarbonyl, 3-Methylpentylcarbonyl, 4-Methylpentylcarbonyl, 1,1-Dimethylbutylcarbonyl, 1,2-Dimethylbutylcarbonyl, 1,3-Dimethylbutylcarbonyl, 2,2-Dimethylbutylcarbonyl, 2,3-Dimethylbutylcarbonyl, 3,3-Dimethylbutylcarbonyl, 1-Ethylbutylcarbonyl, 2-Ethylbutylcarbonyl, 1,1,2-Trimethylpropylcarbonyl, 1,2,2-Trimethylpropylcarbonyl, 1-Ethyl-1-methylpropylcarbonyl und 1-Ethyl-2-methylpropylcarbonyl;
- C₁-C₆-Halogenalkylcarbonyl für z.B. Chloracetyl, Dichloracetyl, Trichloracetyl, Fluoracetyl, Difluoracetyl, Triflouracetyl, Chlorfluoracetyl, Dichlorfluoractyl, Chlordifluoracetyl, 1-Fluorpropionyl, 2-Fluorpropionyl, 2,2-Difluorpropionyl, 3,3,3-Trifluorpropionyl, 3-Chlor-3-fluorpropionyl, 3-Chlor-3,3-difluorpropionyl, 3,3-Dichlor-3-fluorpropionyl, Trichlorpropionyl und Pentafluorpropionyl.

Im einzelnen haben die Substituenten R¹ bis R¹⁰ die folgenden Bedeutungen, und zwar jeweils für sich allein oder in Kombination:
- R¹: Wasserstoff, C₁-C₆-Alkyl oder C₁-C₆-Halogenalkyl, vorzugsweise Wasserstoff, Methyl oder C₁-C₆-Halogenalkyl, insbesondere Wasserstoff, Methyl, Fluormethyl, Difluormethyl, Trifluormethyl, 2-Fluorethyl, 2,2,2-Trifluorethyl oder Pentafluorethyl;
- R²: Wasserstoff, C₁-C₆-Alkyl oder C₁-C₆-Halogenalkyl, vorzugsweise Wasserstoff, C₁-C₆-Alkyl oder C₁-C₄-Halogenalkyl, insbesondere Wasserstoff, Methyl, Ethyl, n-Propyl, Isopropyl, n-Butyl, i-Butyl, sec.-Butyl, tert.-Butyl, Fluormethyl, Difluormethyl oder Trifluormethyl;
- R³: Wasserstoff oder Halogen;
- R⁴: Cyano, Halogen, C₁-C₆-Alkyl, C₁-C₆-Halogenalkyl, C₁-C₆-Alkoxy oder C₁-C₆-Halogenalkoxy, insbesondere Cyano, Fluor, Chlor, Brom, Methyl, Trifluormethyl, Methoxy oder Trifluormethoxy;
- R⁵: Nitro, Cyano oder Halogen;
- R⁷: - Wasserstoff oder C₁-C₆-Alkyl wie vorstehend genannt;
- C₃-C₆-Alkenyl wie Prop-1-en-1-yl, Prop-2-en-1-yl, 1-Methylethenyl, n-Buten-1-yl, n-Buten-2-yl, n-Buten-3-yl, 1-Methylprop-1-en-1-yl, 2-Methyl-prop-1-en-1-yl, 1-Methyl-prop-2-en-1-yl, 2-Methylprop-2-en-1-yl, n-Penten-1-yl, n-Penten-2-yl, n-Penten-3-yl, n-Penten-4-yl, 1-Methyl-but-1-en-1-yl, 2-Methylbut-1-en-1-yl, 3-Methyl-but-1-en-1-yl, 1-Methylbut-2-en-1-yl, 2-Methyl-but-2-en-1-yl, 3-Methyl-but-2-en-1-yl, 1-Methyl-but-3-en-1-yl, 2-Methyl-but-3-en-yl, 3-Methyl-but-3-en-1-yl, 1,1-Dimethyl-prop-2-en-1-yl, 1,2-Dimethyl-prop-1-en-1-yl, 1,2-Dimethyl-prop-2-en-1-yl, 1-Ethyl-prop-1-en-2-yl, 1-Ethyl-prop-2-en-1-yl, n-Hex-1-en-1-yl, n-Hex-2-en-1-yl, n-Hex-3-en-1-yl, n-Hex-4-en-1-yl, n-Hex-5-en-1-yl, 1-Methyl-pent-1-en-1-yl, 2-Methyl-pent-1-en-1-yl, 3-Methyl-pent-1-en-1-yl, 4-Methyl-pent-1-en-1-yl, 1-Methyl-pent-2-en-1-yl, 2-Methyl-pent-2-en-1-yl, 3-Methyl-pent-2-en-1-yl, 4-Methyl-pent-2-en-1-yl, 1-Methyl-pent-3-en-1-yl, 2-Methyl-pent-3-en-1-yl, 3-Methyl-pent-3-en-1-yl, 4-Methyl-pent-3-en-1-yl, 1-Methyl-pent-4-en-1-yl, 2-Methyl-pent-4-en-1-yl, 3-Methyl-pent-4-en-1-yl, 4-Methyl-pent-4-en-1-yl, 1,1-Dimethyl-but-2-en-1-yl, 1,1-Dimethyl-but-3-en-1-yl, 1,2-Dimethyl-but-1-en-1-yl, 1,2-Dimethyl-but-2-en-1-yl, 1,2-Dimethyl-but-3-en-1-yl, 1,3-Dimethyl-but-1-en-1-yl, 1,3-Dimethyl-but-2-en-1-yl, 1,3-Dimethyl-but-3-en-1-yl, 2,2-Dimethyl-but-3-en-1-yl, 2,3-Dimethyl-but-1-en-1-yl, 2,3-Dimethyl-but-2-en-1-yl, 2,3-Dimethyl-but-3-en-1-yl, 3,3-Dimethyl-but-1-en-1-yl, 3,3-Dimethyl-but-2-en-1-yl, 1-Ethyl-but-1-en-1-yl, 1-Ethyl-but-2-en-1-yl, 1-Ethyl-but-3-en-1-yl, 2-Ethyl-but-1-en-1-yl, 2-Ethyl-but-2-en-1-yl, 2-Ethyl-but-3-en-1-yl, 1,1,2-Trimethylprop-2-en-1-yl, 1-Ethyl-1-methyl-prop-2-en-1-yl, 1-Ethyl-2-methyl-prop-1-en-1-yl und 1-Ethyl-2-methyl-prop-2-en-1-yl;
- C₃-C₆-Alkinyl wie Prop-1-in-1-yl, Prop-2-in-1-yl, But-1-in-3-yl, n-But-1-in-1-yl, n-But-1-in-4-yl, n-But-2-in-1-yl, n-Pent-1-in-1-yl, n-Pent-1-in-3-yl, n-Pent-1-in-4-yl, n-Pent-1-in-5-yl, Pent-2-in-1-yl, Pent-2-in-4-yl, Pent-2-in-5-yl, 3-Methyl-but-1-in-3-yl, 3-Methyl-but-1-in-4-yl, n-Hex-1-in-1-yl, n-Hex-1-in-3-yl, n-Hex-1-in-4-yl, n-Hex-1-in-5-yl, n-Hex-1-in-6-yl, n-Hex-2-in-1-yl, N-Hex-2-in-4-yl, n-Hex-2-in-5-yl, n-Hex-2-in-6-yl, n-Hex-3-in-1-yl, n-Hex-3-in-2-yl, 3-Methylpent-1-in-1-yl, 3-Methyl-pent-1-in-3-yl, 3-Methyl-pent-1-in-4-yl, 3-Methyl-pent-1-in-5-yl, 4-Methyl-pent-1-in-1-yl, 4-Methyl-pent-2-in-4-yl und 4-Methyl-pent-2-in-5-yl;
- C₃-C₆-Cycloalkyl wie Cyclopropyl, Cyclobutyl, Cyclopentyl und Cyclohexyl;
- C₁-C₆-Halogenalkyl wie vorstehend genannt;
- Cyano-C₁-C₆-alkyl, z.B. Cyanomethyl, 1-Cyanoeth-1-yl, 2-Cyano-eth-1-yl, 1-Cyanoprop-1-yl, 2-Cyano-prop-1-yl, 3-Cyano-prop-1-yl, 1-Cyanoprop-2-yl, 2-Cyano-prop-2-yl, 1-Cyano-but-1-yl, 2-Cyanobut-1-yl, 3-Cyano-but-1-yl, 4-Cyano-but-1-yl, 1-Cyano-but-2-yl, 2-Cyano-but-2-yl, 1-Cyano-but-3-yl, 2-Cyano-but-3-yl, 1-Cyano-2-methyl-prop-3-yl, 2-Cyano-2-methyl-prop-3-yl, 3-Cyano-2-methyl-prop-3-yl, 2-Cyanomethylprop-2-yl, 1-Cyano-pent-1-yl, 2-Cyano-pent-1-yl, 3-Cyano-pent-1-yl, 4-Cyano-pent-1-yl, 5-cyano-pent-1-yl, 1-Cyano-pent-2-yl, 2-Cyano-pent-2-yl, 1-Cyano-pent-3-yl, 2-Cyano-pent-3-yl, 1-Cyano-pent-4-yl, 2-Cyano-pent-4-yl, 3-Cyano-pent-4-yl, 1-Cyano-2-ethyl-prop-3-yl, 1-Cyano-hex-1-yl, 2-Cyano-hex-1-yl, 3-Cyano-hex-1-yl, 4-Cyano-hex-1-yl, 5-Cyano-hex-1-yl, 6-Cyano-hex-1-yl, 1-Cyano-hex-2-yl, 2-Cyano-hex-2-yl, 1-Cyano-hex-3-yl, 2-Cyano-hex-3-yl, 1-Cyano-hex-4-yl, 2-Cyano-hex-4-yl, 3-Cyano-hex-4-yl, 1-Cyano-2-ethyl-but-3-yl, 1-Cyano-2-ethyl-but-4-yl oder 1-Cyano-2-propyl-prop-3-yl;
- C₁-C₆-Alkoxy-C₁-C₆-alkyl, z.B. Methoxymethyl, Ethoxymethyl, n-Propoxymethyl, (1-Methylethoxy)methyl, n-Butoxymethyl, (1-Methylpropoxy)methyl, (2-Methylpropoxy)methyl, (1,1-Dimethylethoxy)methyl, n-Pentoxymethyl, (1-Methylbutoxy)methyl, (2-Methylbutoxy)methyl, (3-Methylbutoxy)methyl, (2,2-Dimethylpropoxy)methyl, (1-Ethoxypropoxy)methyl, n-Hexoxymethyl, (1,1-Dimethylpropoxy)methyl, (1,2-Dimethylpropoxy)methyl, (1-Methylpentoxy)methyl, (2-Methylpentoxy)methyl, (3-Methylpentoxy)methyl, (4-Methylpentoxy)methyl, (1,1-Dimethylbutoxy)methyl, (1,2-Dimethylbutoxy)methyl, (1,3-Dimethylbutoxy)methyl, (2,2-Dimethylbutoxy)methyl, (2,3-Dimethylbutoxy)methyl, (3,3-Dimethylbutoxy)methyl, (1-Ethylbutoxy)methyl, (1-Ethyl-1-methylpropoxy)methyl, (2-Ethylbutoxy)methyl, (1,1,2-Trimethylpropoxy)methyl, (1,2,2-Trimethylpropoxy)methyl, (1-Ethyl-1-methylpropoxy)methyl, (1-Ethyl-2-methylpropoxy)methyl, Methoxyethyl, Ethoxyethyl, n-Propoxyethyl, (1-Methylethoxy)ethyl, n-Butoxyethyl, (1-Methylpropoxy)ethyl, (2-Methylpropoxy)ethyl, (1,1-Dimethylethoxy)ethyl, n-Pentoxyethyl, (1-Methylbutoxy)ethyl, (2-Methylbutoxy)ethyl, (3-Methylbutoxy)ethyl, (2,2-Dimethylpropoxy)ethyl, (1-Ethylpropoxy)ethyl, n-Hexoxyethyl, (1,1-Dimethylpropoxy)ethyl, (1,2-Dimethylpropoxy)ethyl, (1-Methylpentoxy)ethyl, (2-Methylpentoxy)ethyl, (3-Methylpent-oxy)ethyl, (4-Methylpentoxy)ethyl, (1,1-Dimethylbutoxy)ethyl, (1,2-Dimethylbutoxy)ethyl, (1,3-Dimethylbutoxy)ethyl, (2,2-Dimethylbutoxy)ethyl, (2,3-Dimethylbutoxy)ethyl, (3,3-Dimethylbutoxy)ethyl, (1-Ethylbutoxy)ethyl, (2-Ethylbutoxy)ethyl, (1,1,2-Trimethylpropoxy)ethyl, (1,2,2-Trimethylpropoxy)ethyl, (1-Ethyl-1-methyl-propoxy)ethyl, (1-Ethyl-2-methylpropoxy) ethyl, 2-(Methoxy)propyl, 3-(Methoxy)propyl oder 2-(Ethoxy)propyl;
- C₁-C₆-Alkylthio-C₁-C₆-alkyl, z.B. Methylthiomethyl, Ethylthiomethyl, n-Propylthiomethyl, (1-Methylethylthio)methyl, n-Butylthiomethyl, (1-Methylpropylthio)methyl, (2-Methylpropylthio)methyl, (1,1-Dimethylethylthio)methyl, n-Pentylthiomethyl, (1-Methylbutylthio)methyl, (2-Methylbutylthio)methyl, (3-Methylbutylthio)methyl, (2,2-Dimethylpropylthio)methyl, (1-Ethylpropylthio)methyl, n-Hexylthiomethyl, (1,1-Dimethylpropylthio)methyl, (1,2-Dimethylpropylthio)methyl, (1-Methylpentylthio)methyl, (2-Methylpentylthio)methyl, (3-Methylpentylthio)methyl, (4-Methylpentylthio)methyl, (1,1-Dimethylbutylthio)methyl, (1,2-Dimethylbutylthio)methyl, (1,3-Dimethylbutylthio)methyl, (2,2-Dimethylbutylthio)methyl, (2,3-Dimethylbutylthio)methyl, (3,3-Dimethylbutylthio)methyl, (1-Ethylbutylthio)methyl, (2-Ethylbutylthio)methyl, (1,1,2-Trimethylpropylthio)methyl, (1,2,2-Trimethylpropylthio)methyl, (1-Ethyl-1-methylpropylthio)methyl, (1-Ethyl-2-methylpropylthio)methyl, Methylthioethyl, Ethylthioethyl, n-Propylthioethyl, (1-Methylethylthio)ethyl, n-Butylthioethyl, (1-Methylpropylthio)ethyl, (2-Methylpropylthio)ethyl, (1,1-Dimethylethylthio)ethyl, n-Pentylthioethyl, (1-Methylbutylthio)ethyl, (2-Methylbutylthio)ethyl, (3-Methylbutylthio)ethyl, (2,2-Dimethylpropylthio)ethyl, (1-Ethylpropylthio)ethyl, n-Hexylthioethyl, (1,1-Dimethylpropylthio)ethyl, (1,2-Dimethylpropylthio)ethyl, (1-Methylpentylthio)ethyl, (2-Methylpentylthio)-ethyl, (3-Methylpentylthio)ethyl, (4-Methylpentylthio)ethyl, (1,1-Dimethylbutylthio)ethyl, (1,2-Dimethylbutylthio)ethyl, (1,3-Dimethylbutylthio)ethyl, (2,2-Dimethylbutylthio)ethyl, (2,3-Dimethylbutylthio)ethyl, (3,3-Dimethylbutylthio)ethyl, (1-Ethylbutylthio)ethyl, (2-Ethylbutylthio)ethyl, (1,1,2-Trimethylpropylthio)ethyl, (1,2,2-Trimethylpropylthio)ethyl, (1-Ethyl-1-methylpropylthio)ethyl, (1-Ethyl-2-methylpropylthio)ethyl, 2-(Methylthio)propyl, 3-(Methylthio)propyl oder 2-(Ethylthio)propyl;
- (C₁-C₆-Alkoxy)carbonyl-C₁-C₆-alkyl, z.B. Methoxycarbonylmethyl, Ethoxycarbonylmethyl, n-Propoxycarbonylmethyl, (1-Methylethoxycarbonyl)methyl, n-Butoxycarbonylmethyl, (1-Methylpropoxycarbonyl)methyl, (2-Methylpropoxycarbonyl)methyl, (1,1-Dimethylethoxycarbonyl)methyl, n-Pentoxycarbonylmethyl, (1-Methylbutoxycarbonyl)methyl, (2-Methylbutoxycarbonyl)methyl, (3-Methylbutoxycarbonyl)methyl, (1,1-Dimethylpropoxycarbonyl)methyl, (1,2-Dimethylpropoxycarbonyl)methyl, (2,2-Dimethylpropoxycarbonyl)methyl, (1-Ethylpropoxycarbonyl)methyl, n-Hexoxycarbonylmethyl, (1-Methylpentoxycarbonyl)methyl, (2-Methylpentoxycarbonyl)methyl, (3-Methylpentoxycarbonyl)methyl, (4-Methylpentoxycarbonyl)methyl, (1,1-Dimethylbutoxycarbonyl)methyl, (1,2-Dimethylbutoxycarbonyl)methyl, (1,3-Dimethylbutoxycarbonyl)methyl, (2,2-Dimethylbutoxycarbonyl)methyl, (2,3-Dimethylbutoxycarbonyl)methyl, (3,3-Dimethylbutoxycarbonyl)methyl, (1-Ethylbutoxcarbonyl)methyl, (2-Ethylbutoxycarbonyl)methyl, (1,1,2-Trimethylpropoxycarbonyl)methyl, (1,2,2-Trimethylpropoxycarbonyl)methyl, (1-Ethyl-1-methylpropoxycarbonyl)methyl, (1-Ethyl-2-Methylpropylcarbonyl)methyl, Methoxycarbonylethyl, Ethoxycarbonylethyl, n-Propoxycarbonylethyl, (1-Methylethoxycarbonyl)ethyl, n-Butoxcarbonylethyl, (1-Methylpropoxycarbonyl)ethyl, (2-Methylpropoxycarbonyl)ethyl, (1,1-Dimethylethoxycarbonyl)ethyl, n-Pentoxycarbonylethyl, (1-Methylbutoxycarbonyl)ethyl, (2-Methylbutoxycarbonyl)ethyl, (3-Methylbutoxycarbonyl)ethyl, (1,1-Dimethylpropoxycarbonyl)ethyl, (1,2-Dimethylpropoxycarbonyl)ethyl, (2,2-Dimethylpropoxycarbonyl)-ethyl, (1-Ethylpropoxycarbonyl)ethyl, n-Hexoxycarbonylethyl, (1-Methylpentoxycarbonyl)ethyl, (2-Methylpentoxycarbonyl)-ethyl, (3-Methylpentoxycarbonyl)ethyl, (4-Methylpentoxycarbonyl)ethyl, (1,1-Dimethylbutoxycarbonyl)ethyl, (1,2-Dimethylbutoxycarbonyl)ethyl, (1,3-Dimethylbutoxycarbonyl)ethyl, (2,2-Dimethylbutoxycarbonyl)ethyl, (2,3-Dimethylbutoxycarbonyl)ethyl, (3,3-Dimethylbutoxycarbonyl)ethyl, (1-Ethylbutoxycarbonyl)ethyl, (2-Ethylbutoxycarbonyl)ethyl, (1,1,2-Trimethylpropoxycarbonyl)ethyl, (1,2,2-Trimethylpropoxycarbonyl)ethyl, (1-Ethyl-1-methyl-propoxycarbonyl)ethyl, (1-Ethyl-2-methylpropylcarbonyl)ethyl, 3-(Methoxycarbonyl)propyl, 2-(Methoxycarbonyl)propyl oder 2-Ethoxycarbonyl)propyl;
- (C₁-C₆-Alkylamino)carbonyl-C₁-C₆-alkyl, z.B. Methylaminocarbonylmethyl, Ethylaminocarbonylmethyl, n-Propylaminocarbonylmethyl, (1-Methylethylaminocarbonyl)methyl, n-Butylaminocarbonylmethyl, (1-Methylpropylaminocarbonyl)methyl, (2-Methylpropylaminocarbonyl)methyl, (1,1-Dimethylethylaminocarbonyl)methyl, n-Pentylaminocarbonylmethyl, (1-Methylbutylaminocarbonyl)methyl, (2-Methylbutylaminocarbonyl)methyl, (3-Methyl-butylaminocarbonyl)methyl, (1,1-Dimethylpropylaminocarbonyl)methyl, (1,2-Dimethylpropylaminocarbonyl)methyl, (2,2-Dimethylpropylaminocarbonyl)methyl, (1-Ethylpropylaminocarbonyl)methyl, (n-Hexylaminocarbonyl)methyl, (1-Methylpentylaminocarbonyl)methyl, (2-Methylpentylaminocarbonyl)methyl, (3-Methylpentylaminocarbonyl)methyl, (1,2-Dimethylbutylaminocarbonyl)methyl, (1,3-Dimethylbutylaminocarbonyl)methyl, (2,2-Dimethylbutylaminocarbonyl)methyl, (2,3-Dimethylbutylaminocarbonyl)methyl, (3,3-Dimethylbutylaminocarbonyl)methyl, (1-Ethylbutylaminocarbonyl)methyl, (2-Ethylbutylaminocarbonyl)methyl, (1,1,2-Trimethylpropylaminocarbonyl)methyl, (1,2,2-Trimethylpropylaminocarbonyl)methyl, (1-Ethyl-1-methyl-propylcarbonyl)methyl, (1-Ethyl-2-methylpropylaminocarbonyl)methyl, Methyl-aminocarbonylethyl, Ethylaminocarbonylethyl, n-Propylaminocarbonylethyl, (1-Methylethylaminocarbonyl)ethyl, n-Butylaminocarbonylethyl, (1-Methylpropylaminocarbonyl)-ethyl, (2-Methylpropylaminocarbonyl)ethyl, (1,1-Dimethylethylaminocarbonyl)ethyl, n-Pentylaminocarbonylethyl, (1-Methylbutylaminocarbonyl)ethyl, (2-Methylbutylaminocarbonyl)ethyl, (3-Methylbutylaminocarbonyl)ethyl, (1,1-Dimethylpropylaminocarbonyl)ethyl, (1,2-Dimethylpropylaminocarbonyl)ethyl, (2,2-Dimethylpropylaminocarbonyl)ethyl, (1-Ethylpropylaminocarbonyl)ethyl, (n-Hexylaminocarbonyl)ethyl, (1-Methylpentylaminocarbonyl)ethyl, (2-Methylpentylaminocarbonyl)ethyl, (3-Methylpentylaminocarbonyl)ethyl, (1,2-Dimethylbutylaminocarbonyl)ethyl, (1,3-Dimethylbutylaminocarbonyl)ethyl, (2,2-Dimethylbutylaminocarbonylethyl, (2,3-Dimethylbutylaminocarbonyl)ethyl, (3,3-Dimethylethylbutylaminocarbonyl)ethyl, (1-Ethylbutylaminocarbonyl)ethyl, (2-Ethylbutylaminocarbonyl)ethyl, (1,1,2-Trimethylpropylaminocarbonyl)-ethyl, (1,2,2-Trimethylpropylaminocarbonyl)-ethyl, (1-Ethyl-1-methylpropylcarbonyl)ethyl, (1-Ethyl-2-methylpropylaminocarbonyl)ethyl, 3-(Methylaminocarbonyl)propyl oder 2-(Methylaminocarbonyl)propyl;
- Di-(C₁-C₆-Alkyl)aminocarbonyl-(C₁-C₆-alkyl), z.B. N,N-Dimethylaminocarbonylmethyl, N,N-Diethylaminocarbonylmethyl, N,N-Diisopropylaminocarbonylmethyl, N,N-Dibutylaminocarbonylmethyl, N,N-Di-(1-methylpropyl)aminocarbonylmethyl, N,N-Di-(2-Methylpropyl)-aminocarbonylmethyl, N,N-Di-(1,1-Dimethylethyl)aminocarbonylmethyl, N-Ethyl-N-Methylaminocarbonylmethyl, N-Methyl-N-propylaminocarbonylmethyl, N-Methyl-N-(1-Methylethyl)aminocarbonylmethyl, N-Butyl-N-methylaminocarbonylmethyl, N-Methyl-N-(1-methylpropyl)aminocarbonylmethyl, N-Methyl-N-(2-methylpropyl)aminocarbonylmethyl, N-(1,1-Dimethylethyl)-N-methylaminocarbonylmethyl, N-Ethyl-N-propylaminocarbonylmethyl, N-Ethyl-N-(1-methylethyl)aminocarbonylmethyl, N-Butyl-N-ethylaminocarbonylmethyl, N-Ethyl-N-(1-methylpropyl)aminocarbonylmethyl, N-Ethyl-N-(2-methylpropyl)aminocarbonylmethyl, N-Ethyl-N-(1,1-dimethylethyl)aminocarbonylmethyl, N-(1-Methylethyl)-N-propylaminocarbonylmethyl, N-Butyl-N-propylaminocarbonylmethyl, N-(1-Methylpropyl)-N-propylaminocarbonylmethyl, N-(2-Methylpropyl)-N-propylaminocarbonylmethyl, N-(1,1-Dimethylethyl)-N-propylaminocarbonylmethyl, N-Butyl-N-(1-methylethyl)aminocarbonylmethyl, N-(1-Methylethyl)-N-(1-methylpropyl)aminocarbonylmethyl, N-(1-Methylethyl)-N-(2-methylpropyl)aminocarbonylmethyl, N-(1,1-Dimethylethyl)-N-(1-methylethyl)aminocarbonylmethyl, N-Butyl-N-(1-methylpropyl)aminocarbonylmethyl, N-Butyl-N-(2-methylpropyl)aminocarbonylmethyl, N-Butyl-N-(1,1-dimethylethyl)aminocarbonylmethyl, N-(1-Methylpropyl)-N-(2-methylpropyl)aminocarbonylmethyl, N-(1,1-Dimethylethyl)-N-(1-methylpropyl)aminocarbonylmethyl, N-(1,1-Dimethylethyl)-N-(2-methylpropyl)aminocarbonylmethyl, N,N-Dimethylaminocarbonylethyl, N,N-Diethylaminocarbonylethyl, N,N-Diisopropylaminocarbonylethyl, N,N-Dibutylaminocarbonylethyl, N,N-Dibutylaminocarbonylethyl, N,N-Di-(1-methylpropyl)aminocarbonylethyl, N,N-Di-(2-methylpropyl)aminocarbonylethyl, N,N-Di-(1,1-Dimethylethyl)aminocarbonylethyl, N-Ethyl-N-methylaminocarbonylethyl, N-Methyl-N-propylaminocarbonylethyl, N-Methyl-N-(1-methylethyl)aminocarbonylethyl, N-Butyl-N-methylaminocarbonylethyl, N-Methyl-N-(1-methylpropyl)aminocarbonylethyl, N-Methyl-N-(2-methylpropyl)aminocarbonylethyl, N-(1,1-Dimethylethyl)-N-methylaminocarbonylethyl, N-Ethyl-N-propylaminocarbonylethyl, N-Ethyl-N-(1-methylethyl)aminocarbonylethyl, N-Butyl-N-ethylaminocarbonylethyl, N-Ethyl-N-(1-methylpropyl)aminocarbonylethyl, N-Ethyl-N-(2-methylpropyl)aminocarbonylethyl, N-Ethyl-N-(1,1-dimethylethyl)aminocarbonylethyl, N-(1-Methylethyl)-N-propylaminocarbonylethyl, N-Butyl-N-propylaminocarbonylethyl, N-(1-Methylpropyl)-N-propylaminocarbonylethyl, N-(2-Methylpropyl)-N-propylaminocarbonylethyl, N-(1,1-Di-methylethyl)-N-propylaminocarbonylethyl, N-Butyl-N-(1-methylethyl)aminocarbonylethyl, N-(1-Methylethyl)-N-(1-Methylpropyl)aminocarbonylethyl, N-(1-Methylethyl)-N-(2-methylpropyl)aminocarbonylethyl, N-(1,1-Dimethylethyl)-N-(1-methylethyl)aminocarbonylethyl, N-Butyl-N-(1-methylpropyl)aminocarbonylethyl, N-Butyl-N-(2-methylpropyl)aminocarbonylethyl, N-Butyl-N-(1,1-dimethylethyl)aminocarbonylethyl, N-(1-Methylpropyl)-N-(2-methylpropyl)aminocarbonylethyl, N-(1,1-Dimethylethyl)-N-(1-methylpropyl)aminocarbonylethyl, N-(1 1-Dimethylethyl)-N-(2-methylpropyl)aminocarbonylethyl, 3-N,N-(Dimethylaminocarbonyl)propyl, 3-N,N-(Diethylaminocarbonyl)propyl, 2-N,N-Dimethylaminocarbonylpropyl oder 1-N,N-Dimethylaminocarbonylpropyl;
- C₁-C₆-Alkyloximino-C₁-C₆-alkyl, z.B. Methoximinomethyl, Ethoximinomethyl, n-Propoximinomethyl, (1-Methylethoximino)methyl, n-Butoximinoethyl, (1-Methylpropoximimo)methyl, (2-Methylpropoximino)methyl, (1,1-Dimethylethoximino)methyl, n-Pentoximino-methyl, (1-Methylbutoximino)methyl, (2-Methylbutoximino)methyl, (3-Methylbutoximino)methyl, (2,2-Dimethylpropoximino)methyl, (1-Ethylpropoximino)methyl, n-Hexoximinomethyl, (1,1-Dimethylpropoximino)methyl, (1,2-Dimethylpropoximino)methyl, (1-Methylpentoximino)methyl, (2-Methylpentoximino)methyl, (3-Methylpentoximino)methyl, (4-Methylpentoximino)methyl, (1,1-Dimethylbutoximino)methyl, (1,2-Dimethylbutoximino)methyl, (1,3-Dimethylbutoximino)methyl, (2,2-Dimethylbutoximino)methyl, (2,3-Dimethylbutomino)methyl, (3,3-Dimethylbutoximino)methyl, (1-Ethylbutoximino)methyl, (2-Ethylbutoximino)methyl, (1,1,2-Trimethylpropoximino)methyl, (1,2,2-Trimethylpropoximino)methyl, (1-Ethyl-1-methylpropoximino)methyl, (1-Ethyl-2-methylpropoximino)methyl, Methoximinoethyl, Ethoximinoethyl, N-Propoximinoethyl, (1-Methylethoximino)ethyl, n-Butoximino-ethyl, (1-Methylpropoximino)ethyl, (2-Methylpropoximino)ethyl, (1,1-Dimethylethoximino)ethyl, n-Pentoximinoethyl, (1-Methylbutoximino)ethyl, (2-Methylbutoximino)ethyl, (3-Methylbutoximino)ethyl, (2,2-Dimethylpropoximino)ethyl, (1-Ethylpropoximino)ethyl, n-Hexoximino-ethyl, (1,1-Dimethylpropoximino)ethyl, (1,2-Dimethylpropoximino)ethyl, (1-Methylpentoximino)ethyl, (2-Methylpentoximino)ethyl, (3-Methylpentoximino)ethyl, (4-Methylpentoximino)ethyl, (1,1-Dimethylbutoximino)ethyl, (1,2-Dimethylbutoximino)ethyl, (1,2-Dimethylbutoximino)ethyl, (1,3-Dimethylbutoximino)ethyl, (2,2-Dimethylbutoximino)ethyl, (2,3-Dimethylbutoximino)ethyl, (3,3-Dimethylbutoximino)ethyl, (1-Ethyl-butoximino)ethyl, (2-Ethylbutoximino)ethyl, (1,1,2-Trimethylpropoximino)ethyl, (1,2,2-Trimethylpropoximino)ethyl, (1-Ethyl-1-methylpropoximino)ethyl, (1-Ethyl-2-methylpropoximino)ethyl, 2-(Methoximino)propyl, 3-(Methoximino)propyl oder 2-(Ethoximino)propyl;
- Di(C₁-C₆-alkoxy)-C₂-C₆-alkyl, z.B. 2,2-Dimethoxyethyl, 2,2-Diethoxyethyl, 2,2-Di-(n-propoxy)ethyl, 2,2-Di-(1-methylethoxy)ethyl, 2,2-Dibutoxyethyl, 2,2-Di-(1-methyl-propoxy)ethyl, 2,2-Di-(2-methylpropoxy)ethyl, 2,2-Di-(1,1-dimethylethoxy)ethyl, 2-(Ethoxy)-2-(methoxy)ethyl, 2-(Methoxy)-2-(propoxy)ethyl, 2-(Methoxy)-2-(1-methylethoxy)ethyl, 2-(Butoxy)-2-(methoxy)ethyl, 2-(Methoxy)-2-(1-methylpropoxy)ethyl, 2-(Methoxy)-2-(2-methylpropoxy)ethyl, 2-(1,1-Dimethylethoxy)-2-(methoxy)ethyl, 2-(Ethoxy)-2-(propoxy)ethyl, 2-(Ethoxy)-2-(1-methylethoxy)ethyl, 2-(Butoxy)-2-(ethoxy)-ethyl, 2-(Ethoxy)-2-(1-methyl-propoxy)ethyl, 2-(Ethoxy)-2-(2-methylpropoxy)ethyl, 2-(Ethoxy)-2-(1,1-Dimethylethoxy)ethyl, 2-(1-Methyl-ethoxy)-2-(propoxy)ethyl, 2-(Butoxy)-2-(propoxy)ethyl,2-(1-Methylpropoxy)-2-(propoxy)ethyl, 2-(2-Methyl-propoxy)-2-(propoxy)-ethyl, 2-(1,1-Dimethylethoxy)-2-(propoxy)ethyl, 2-(Butoxy)-2-(1-methylethoxy)ethyl, 2-(1-Methylethoxy)-2-(1-methylpropoxy)ethyl, 2-(1-Methylethoxy)-2-(2-methylpropoxy)ethyl, 2-(1,1-Dimethylethoxy)-2-(1-methylethoxy)ethyl, 2-(Butoxy)-2-(1-methylpropoxy)ethyl, 2-(Butoxy)-2-(2-methylpropoxy)ethyl, 2-(Butoxy)-2-(1,1-Dimethylethoxy)ethyl, 2-(1-Methyl-propoxy)-2-(2-Methyl-propoxy)ethyl, 2-(1,1-Dimethyl-ethoxy)-2-(1-methylpropoxy)ethyl oder 2-(1,1-Dimethyl-ethoxy)-2-(2-methylpropoxy)ethyl;
- Di- (C₁-C₆-alkylthio)-C₂-C₆-alkyl, z.B. 2,2-(Dimethylthio)ethyl, 2,2-(Diethylthio)ethyl, 2,2-Di(n-propylthio)ethyl, 2,2-Di-(1-methylethylthio)ethyl, 2,2-(Dibutylthio)ethyl, 2,2-Di-(1-methylpropylthio)ethyl, 2,2-Di-(2-methylpropylthio)ethyl, 2,2-Di(1,1-dimethylethylthio)ethyl, 2-(Ethylthio)-2-(methylthio)ethyl, 2-(Methylthio)-2-(propylthio)ethyl, 2-(Methylthio)-2-(1-methylethylthio)ethyl, 2-(Butylthio)-2-(methylthio)ethyl, 2- (Methylthio)-2-(1-methylpropylthio)ethyl, 2-(Methylthio)-2-(2-methylpropylthio)ethyl, 2-(1,1-Dimethylethyl-thio)-2-(methylthio)ethyl, 2-(Ethylthio)-2-(propylthio)-ethyl, 2-(Ethylthio)-2-(1-methylethylthio)ethyl, 2-(Butylthio)-2-(ethylthio)ethyl, 2-(Ethylthio)-2-(1-methylpropylthio)ethyl, 2-(Ethylthio)-2-(2-methylpropylthio)ethyl, 2-(Ethylthio)-2-(1,1-dimethylethylthio)ethyl, 2-(1-Methylethylthio)-2-(propylthio)ethyl, 2-(Butylthio)-2-(propylthio)-ethyl, 2-11-Methylpropylthio)-2-(propylthio)ethyl, 2-(2-Methylpropylthio)-2-(propylthioethyl), 2-(1,1-Dimethyl-ethylthio)-2-(propylthio)ethyl, 2-(Butylthio)-2-(1-methylethylthio)ethyl, 2-(1-Methylethylthio)-2-(1-methylpropylthio)ethyl, 2-(1-Methylethylthio)-2-(2-methylpropylthio)ethyl, 2-(1,1-Dimethylethylthio)-2-(1-methylethylthio)ethyl, 2-(Butylthio)-2-(1-methylpropylthio)ethyl, 2-(Butylthio)-2-(2-methylpropylthio)ethyl, 2-(Butylthio)-2-(1,1-dimethylethylthio)ethyl, 2-(1-Methylpropylthio)-2-(2-methylpropylthio)ethyl, 2-(1,1-Dimethylethylthio)-2-(1-methylpropylthio)ethyl oder 2-(1,1-Dimethylethylthio)-2-(2-methylpropylthio)ethyl;
- C₃-C₆-Halogenalkenyl, z.B. 2-Chlorprop-2-enyl, 3-Chlor-prop-2-enyl, 2,3-Dichlorprop-2-enyl, 3,3-Dichlorprop-2-enyl, 2,3,3-Trichlorprop-2-enyl, 2,3-Dichlorbut-2-enyl, 2-Bromprop-2-enyl, 3-Bromprop-2-enyl, 2,3-Dibromprop-2-enyl, 3,3-Dibromprop-2-enyl, 2,3,3-Tribromprop-2-enyl oder 2,3-Dibrombut-2-enyl;
- Phenyl oder Benzyl, wobei der Phenylring jeweils ein bis drei Reste tragen kann:
   - Cyano, Nitro, Halogen, C₁-C₆-Alkyl, C₁-C₆-Halogenalkyl, C₁-C₆-Alkoxy, C₁-C₆-Halogenalkoxy,
   - C₁-C₆-Alkylthio wie Methylthio, Ethylthio, n-Propylthio, 1-Methylethylthio, n-Butylthio, 1-Methyl-propylthio, 2-Methylpropylthio, 1,1-Dimethylethylthio, n-Pentylthio, 1-Methylbutylthio, 2-Methylbutylthio, 3-Methylbutylthio, 1,1-Dimethylpropylthio, 1,2-Dimethylproyplthio, 2,2-Dimethylpropylthio, 1-Ethylpropylthio, n-Hexylthio, 1-Methylpentylthio, 2-Methylpentylthio, 3-Methylpentylthio, 4-Methylpentylthio, 1,1-Dimethylbutylthio, 1,2-Dimethylbutylthio, 1,3-Dimethylbutlythio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropylthio und 1-Ethyl-2-methylpropylthio,
   - C₁-C₆-Halogenalkylthio, z.B. Difluormethylthio, Trifluormethylthio, Chlordifluormethylthio, Bromdifluormethylthio, 2-Fluorethylthio, 2,2-Difluorethylthio, 2,2,2-Trifluorethylthio, 2-Chlorethylthio, 2-Bromethylthio, 2-Iodethylthio, 2,2,2-Trichlorethylthio, 2-Chlor-2-fluorethylthio, 2-Chlor-2,2-difluorethylthio, 2,2-Dichlor-2-fluorethylthio, 2,2,2-Trichlorethylthio, Pentafluorethylthio, 3-Fluorpropylthio, 2-Fluorpropylthio, 2,2-Difluorpropylthio, 2,3-Difluorpropylthio, 3-Chlorpropylthio, 2-Chlorpropylthio, 2,3-Dichlorpropylthio, 3-Brompropylthio, 2-Brompropylthio, 3,3,3-Trichlorpropylthio, 3,3,3-Trifluorpropylthio, 2,2,3,3,3-Pentfluorpropylthio, Heptafluorpropylthio, 1-(Fluormethyl)-2-fluorethylthio, 1-(Chlormethyl)-2-chlorethylthio, 1-(Brommethyl)-2-bromethylthio, 4-Fluorbutylthio, 4-Chlorbutylthio oder 4-Brombutylthio,
   - (C₁-C₆-Alkoxy)carbonyl, z.B. Methoxycarbonyl, Ethoxycarbonyl, n-Propoxycarbonyl, 1-Methylethoxycarbonyl, n-Butoxycarbonyl, 1-Methylpropoxycarbonyl, 2-Methylpropoxycarbonyl, 1,1-Dimethylethoxycarbonyl, n-Pentoxycarbonyl, 1-Methylbutoxycarbonyl, 2-Methylbutoxycarbonyl, 3-Methylbutoxycarbonyl, 1,1-Dimethylpropoxycarbonyl, 1,2-Dimethylpropoxycarbonyl, 2,2-Dimethylpropoxycarbonyl, 1-Ethylpropoxycarbonyl, Hexoxycarbonyl, 1-Methylpentoxycarbonyl, 2-Methylpentoxycarbonyl, 3-Methylpentoxycarbonyl, 4-Methylpentoxycarbonyl, 1,1-Dimethylbutoxycarbonyl, 1,2-Dimethylbutoxycarbonyl, 1,3-Dimethylbutoxycarbonyl, 2,2-Dimethylbutoxycarbonyl, 2,3-Dimethylbutoxycarbonyl, 3,3-Dimethylbutoxycarbonyl, 1-Ethylbutoxycarbonyl, 2-Ethylbutoxycarbonyl, 1,1,2-Trimethylpropoxycarbonyl, 1,2,2-Trimethylpropoxycarbonyl, 1-Ethyl-1-methylpropoxycarbonyl oder 1-Ethyl-2-methylpropyl-carbonyl.

Insbesondere bedeutet R⁷:
- Wasserstoff,
- C₁-C₄-Alkyl, insbesondere Methyl, Ethyl, n-Propyl, 1-Methylethyl, 1-Methylpropyl oder 2-Methylpropyl;
- C₃-C₄-Alkenyl, insbesondere Prop-2-en-2-yl, Prop-2-en-1-yl, But-2-en-2-yl, But-2-en-1-yl, 2-Methyl-prop-2-en-1-yl oder But-1-en-3-yl;
- C₃-C₄-Alkinyl, insbesondere Prop-2-in-1-yl, n-Bu-1-tin-3-yl oder n-But-2-in-1-yl;
- C₃-C₆-Cycloalkyl, insbesondere Cyclopropyl, Cyclopentyl oder Cyclohexyl;
- C₁-C₄-Halogenalkyl, insbesondere Fluormethyl, Difluormethyl, Trifluormethyl, 2-Fluorethyl, 2,2-Difluorethyl, 2,2,2-Trifluorethyl, 2-Chlorethyl oder 2,2,2-Trichlorethyl;
- C₁-C₄-Cyanoalkyl, insbesondere Cyanomethyl oder 1-Cyanoeth-1-yl;
- C₁-C₄-Alkoxy-C₁-C₄-alkyl, insbesondere Methoxymethyl oder Methoxyethyl;
- C₁-C₄-Alkylthio-C₁-C₄-alkyl, insbesondere Methylthiomethyl oder Methylthioethyl;
- C₁-C₄-Alkoxycarbonyl-C₁-C₄-alkyl, insbesondere Methoxycarbonylmethyl, Ethoxycarbonylmethyl, 1-Methylethoxycarbonylmethyl, 2-Methylpropoxycarbonylmethyl, 1-(Methoxycarbonyl)-eth-1-yl, 1-(Ethoxycarbonyl)-eth-1-yl, 1-(1-Methylethoxycarbonyl)-eth-1-yl, 1-(Methoxycarbonyl)-prop-1-yl, 1-(Ethoxycarbonyl)-prop-1-yl, 1-(Methoxycarbonyl)-but-1-yl oder 1-(Ethoxycarbonyl)-but-1-yl;
- (C₁-C₄-Alkylamino)carbonyl-(C₁-C₄)-alkyl, insbesondere Methylaminocarbonylmethyl, Ethylaminocarbonylmethyl, 1-Methylethylaminocarbonylmethyl, 2-Methylpropylaminocarbonylmethyl, 1-(Methylaminocarbonyl)-eth-1-yl, 1-(Ethylaminocarbonyl)-eth-1-yl, 1-(1-Methylethylaminocarbonyl)eth-1-yl, 1-(Methylaminocarbonyl)-prop-1-yl, 1-(Ethylaminocarbonyl)-prop-1-yl, 1-(Methylaminocarbonyl)-but-1-yl oder 1-(Ethylaminocarbonyl)-but-1-yl;
- Di-(C₁-C₄-Alkyl)aminocarbonyl-(C₁-C₄-alkyl), insbesondere N,N-Dimethylaminocarbonylmethyl, N,N-Diethylaminocarbonylmethyl, N-Methyl-N-ethylaminocarbonylmethyl, N-Isopropyl-N-methylaminocarbonylmethyl, 1-(N,N-Dimethylaminocarbonyl)-1-ethyl, 1-(N,N-Diethylaminocarbonyl)-eth-1-yl, 1-(N-Methyl-N-ethylaminocarbonyl)-eth-1-yl, 1-(N,N-Dimethylaminocarbonyl)-1-propyl oder 1-(N,N-Diethylaminocarbonyl)-prop-1-yl;
- Alkyloximinoalkyl mit insgesamt höchstens 6 C-Atomen, insbesondere Methoximinomethyl, Ethoximinomethyl, Methoximinoethyl oder Ethoximinoethyl;
- Dialkoxyalkyl mit insgesamt höchstens 8 C-Atomen, insbesondere 2,2-Dimethoxyethyl oder 2,2-Diethoxyethyl;
- Dialkylthioalkyl mit insgesamt höchstens 8 C-Atomen, insbesondere 2,2-(Dimethylthio)ethyl oder 2,2-(Diethylthio)-ethyl;
- C₃-C₄-Halogenalkenyl, insbesondere 2-Chlor-2-propenyl;
- unsubstituiertes oder einfach substituiertes Phenyl oder Benzyl, insbesondere Phenyl, 2-Fluorphenyl, 3-Fluorphenyl, 4-Fluorphenyl, 2-Chlorphenyl, 3-Chlorphenyl, 4-Chlorphenyl, 2-Methylphenyl, 3-Methylphenyl, 4-Methylphenyl, 2-Trifluormethylphenyl, 3-Trifluormethylphenyl, 4-Trifluormethylphenyl, 2-Methoxyphenyl, 3-Methoxyphenyl, 4-Methoxyphenyl, 2-Methoxycarbonylphenyl, 3-Methoxycarbonylphenyl, 4-Methoxycarbonylphenyl oder Benzyl;

R⁸, R⁹, R¹⁰ unabhängig voneinander
- (C₁-C₆-Alkyl)carbonyl wie vorstehend genannt,
- (C₁-C₆-Halogenalkyl)carbonyl wie vorstehend genannt oder
- einen der für R⁷ genannten Reste.

Bevorzugt sind
- (C₁-C₄-Alkyl)carbonyl, insbesondere Methylcarbonyl, Ethylcarbonyl oder Propylcarbonyl,
- (C₁-C₄-Halogenalkyl)carbonyl, insbesondere Chloracetyl, Trichloracetyl, Fluoracetyl oder Trifluoracetyl;
- Wasserstoff;
- C₁-C₄-Alkyl, insbesondere Methyl, Ethyl, n-Propyl, 1-Methylethyl, 1-Methylpropyl oder 2-Methylpropyl;
- C₃-C₄-Alkenyl, insbesondere Prop-2-en-2-yl, Prop-2-en-1-yl, But-2-en-2-yl, But-2-en-1-yl, 2-Methyl-prop-2-en-1-yl oder But-1-en-3-yl;
- C₃-C₄-Alkinyl, insbesondere Prop-2-in-1-yl, n-Bu-1-tin-3-yl oder n-But-2-in-1-yl;
- C₃-C₆-Cycloalkyl, insbesondere Cyclopropyl, Cyclopentyl oder Cyclohexyl;
- C₁-C₄-Halogenalkyl, insbesondere Fluormethyl, Difluormethyl, Trifluormethyl, 2-Fluorethyl, 2,2-Difluorethyl, 2,2,2-Trifluorethyl, 2-Chlorethyl oder 2,2,2-Trichlorethyl;
- C₁-C₄-Cyanoalkyl, insbesondere Cyanomethyl oder 1-Cyano-eth-1-yl;
- C₁-C₄-Alkoxy-C₁-C₄-alkyl, insbesondere Methoxymethyl oder Methoxyethyl;
- C₁-C₄-Alkylthio-C₁-C₄-alkyl, insbesondere Methylthiomethyl oder Methylthioethyl;
- C₁-C₄-Alkoxycarbonyl-C₁-C₄-alkyl, insbesondere einen der hier- für bei R⁷ einzeln aufgeführten Reste;
- (C₁-C₄-Alkylamino)carbonyl-(C₁-C₄)-alkyl, insbesondere einen der hierfür bei R⁷ einzeln aufgeführten Reste;
- Di-(C₁-C₄-Alkyl)aminocarbonyl-(C₁-C₄-alkyl), insbesondere einen der hierfür bei R⁷ einzeln aufgeführten Reste;
- Alkyloximinoalkyl mit insgesamt höchstens 6 C-Atomen, insbesondere einen der hierfür bei R⁷ einzeln aufgeführten Reste;
- Dialkoxyalkyl mit insgesamt höchstens 8 C-Atomen, insbesondere 2,2-Dimethoxyethyl oder 2,2-Diethoxyethyl;
- Dialkylthioalkyl mit insgesamt höchstens 8 C-Atomen, insbesondere 2,2-(Dimethylthio)ethyl oder 2,2-(Diethylthio)-ethyl;
- C₃-C₄-Halogenalkenyl, insbesondere 2-Chlor-2-propenyl;
- unsubstituiertes oder einfach substituiertes Phenyl oder Benzyl, insbesondere wie bei R⁷ hierfür einzeln aufgeführt.

R⁸ und R⁹ können auch zusammen eine 4- bis 6-gliedrige Alkylenkette bedeuten, in der eine Methyleneinheit durch Sauerstoff oder durch eine C₁-C₄-Alkyliminogruppe ersetzt sein kann, und auf diese Weise mit dem Stickstoff, an den die beiden Substituenten gebunden sind, einen 5- bis 7-gliedrigen Heterocyclus bilden, beispielsweise 1-Pyrrolidinyl, 1-Piperidinyl, 1-Hexahydroazepinyl, 4-Morpholinyl oder 4-Methylhexahydropyrazin-1-yl.

Im Hinblick auf die Verwendung der erfindungsgemäßen substituierten Triazolinone der Formel I als herbizide und/oder defoliant/ desikkant wirksame Verbindungen haben die Variablen vorzugsweise folgende Bedeutung:
- R¹: einen Rest aus der Gruppe H, CH₃, CH₂F, CHF₂, CF₃, CH₂CH₂F, CH₂CF₃, CF₂CF₃;
- R²: einen Rest aus der Gruppe H, CH₃, C₂H₅, n-C₃H₇, i-C₃H₇, n-C₄H₉, i-C₄H₉, s-C₄H₉, t-C₄H₉, CH₂F, CHF₂, CF₃;
- R³: einen Rest aus der Gruppe H, F, Cl, Br, I;
- R⁴: einen Rest aus der Gruppe F, Cl, Br, CN, CH₃, CF₃, OCH₃, OCF₃;
- R⁵: einen Rest aus der Gruppe Cl, Br, I, F, NO₂, CN;
- R⁶: einen Rest aus der Gruppe 6.01 - 6.310 (Tabelle 1),
wobei die Reste R¹, R², R³, R⁴, R⁵ und R⁶ beliebig miteinander kombiniert werden können.

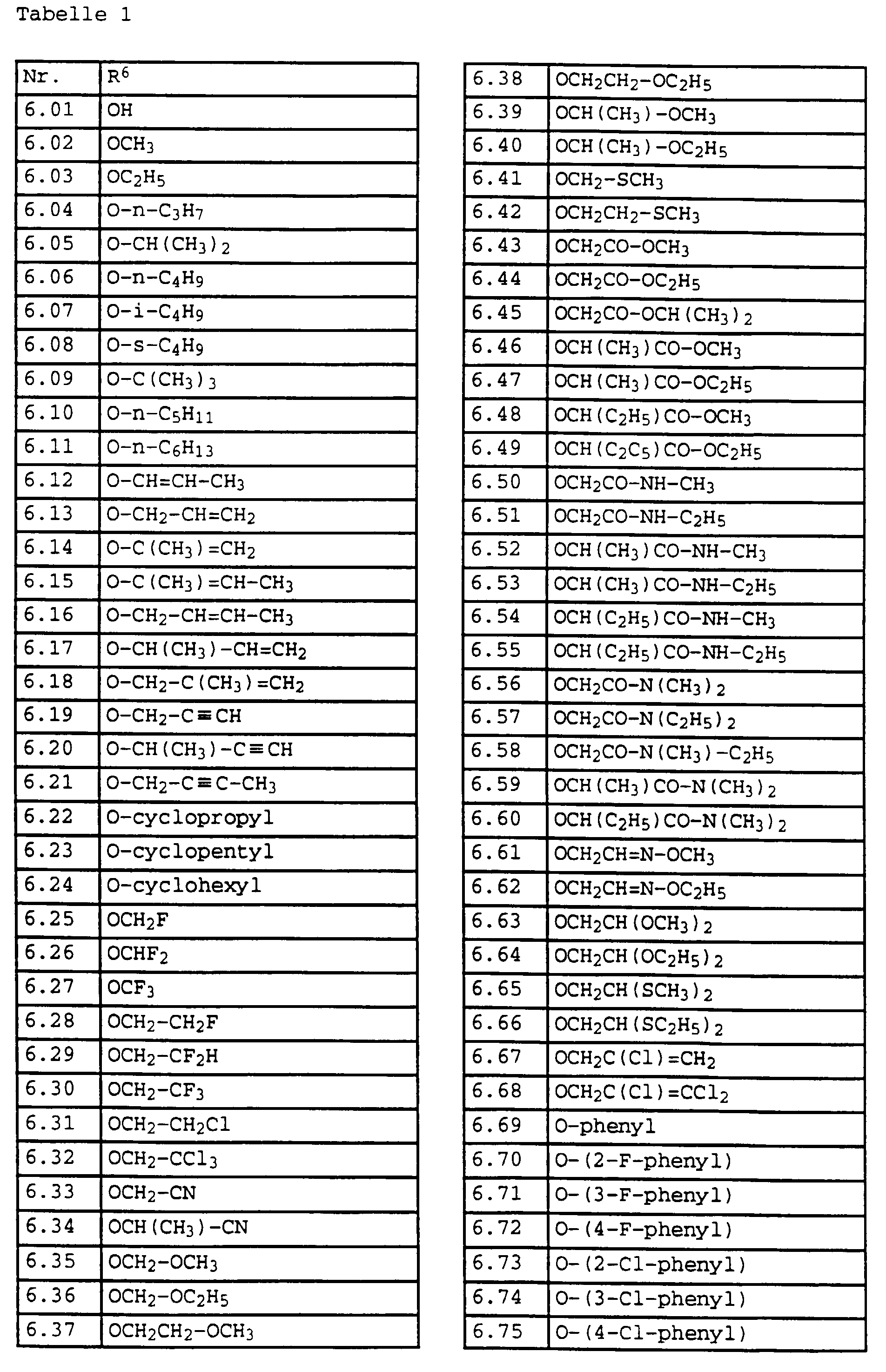

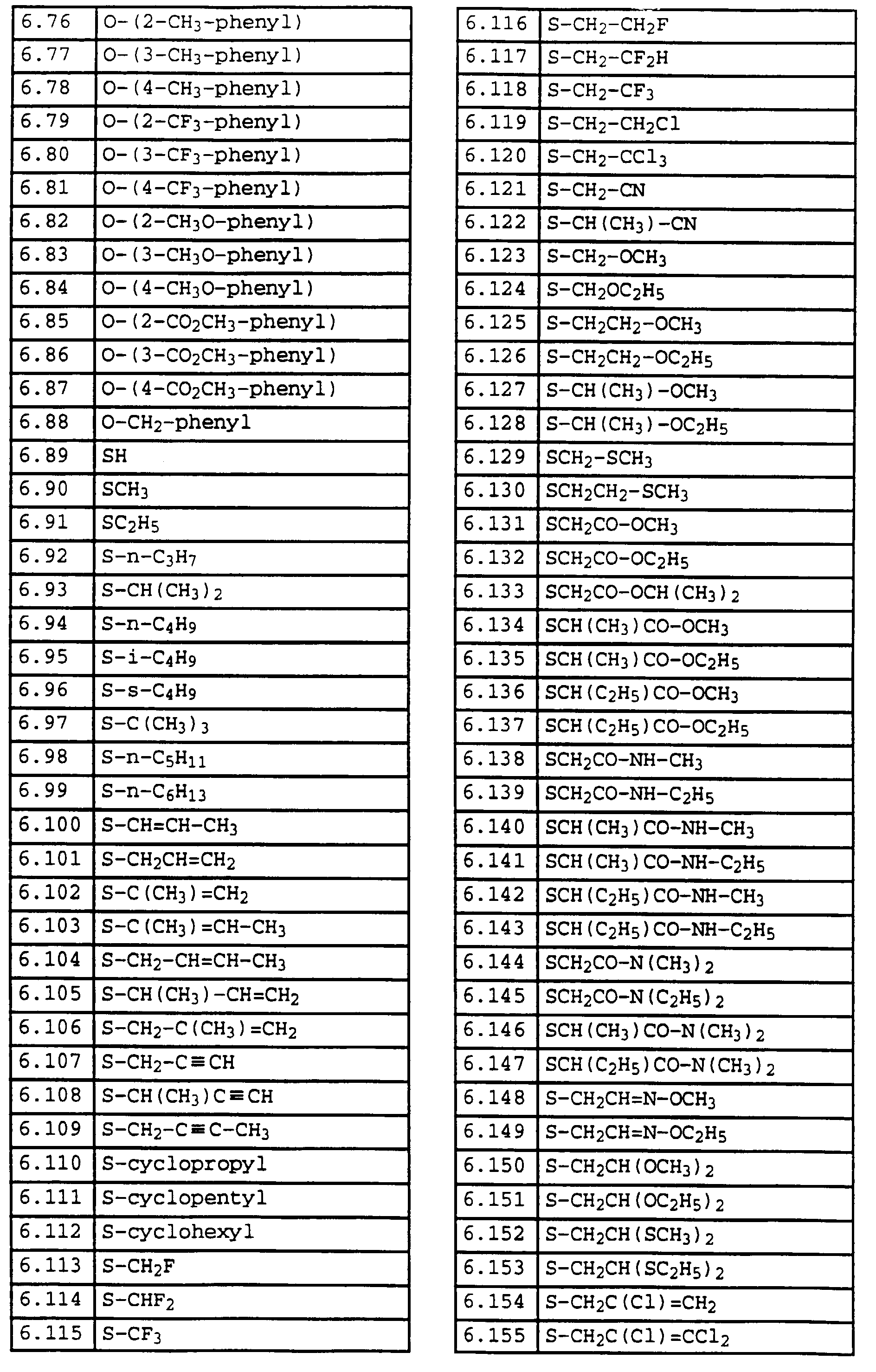

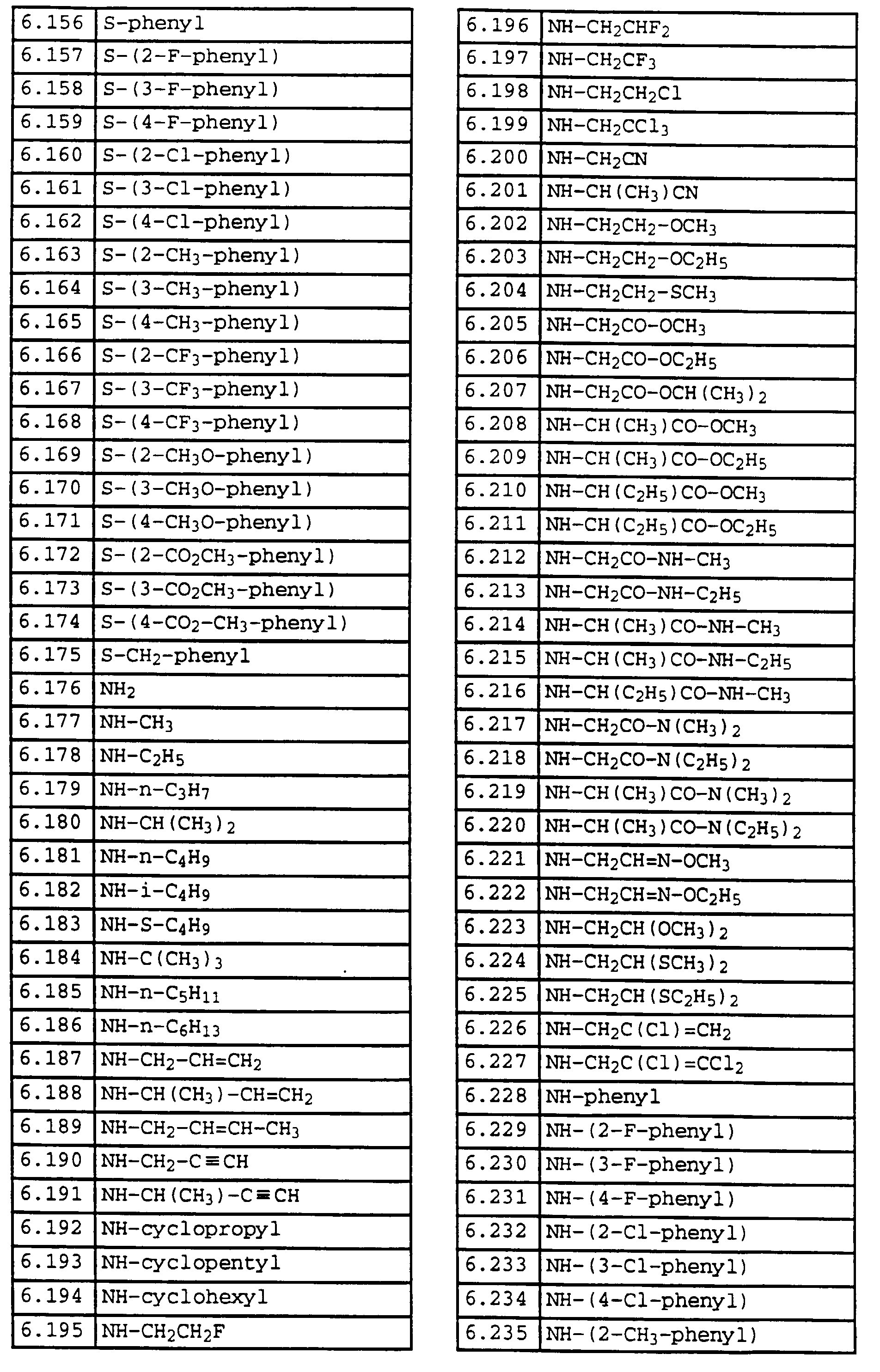

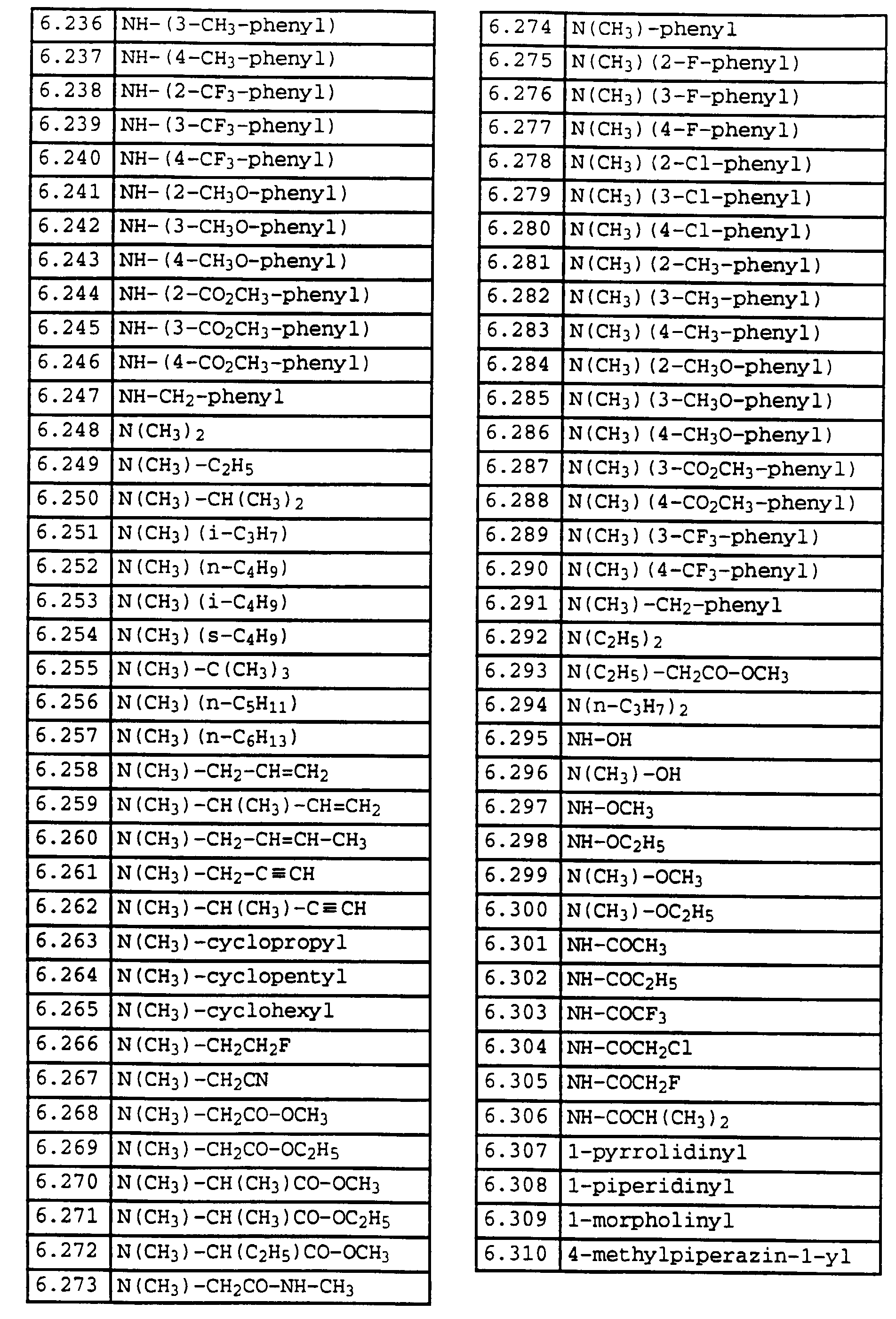

Die substituierten Triazolinone der Formel I können in Form ihrer landwirtschaftlich brauchbaren Salze vorliegen, wobei es auf die Art des Salzes in der Regel nicht ankommt. Im allgemeinen kommen die Salze von solchen Basen in Betracht, die die herbizide Wirkung von I nicht negativ beeinträchtigen.

Als basische Salze eignen sich besonders diejenigen der Alkalimetalle, vorzugsweise Natrium- und Kaliumsalze, die der Erdalkalimetalle, vorzugsweise Calcium-, Magnesiumsalze und die Übergangsmetalle, vorzugsweise Zink- und Eisensalze, sowie die Ammoniumsalze, die ein bis vier C₁-C₄-Alkyl, Hydroxy-C₁-C₄-alkyl-substituenten und/oder einen Phenyl- oder Benzylsubstituenten tragen können, vorzugsweise Diisopropylammonium-, Tetramethylammonium-, Tetrabutylammonium-, Trimethylbenzylammonium- und Trimethyl-(2-hydroxyethyl)-ammoniumsalze, die Phosphoniumsalze, Sulfoniumsalze und Sulfoxoniumsalze, vorzugsweise Tri-(C₁-C₄-alkyl)sulfoxoniumsalze.

In Hinblick auf die Verwendung der substituierten Triazolone I als Herbizide sind die in Tabelle 2 aufgeführten Verbindungen Ia (= I mit R¹ = CHF₂, R² = CH₃, R⁶ = OR⁷) ganz besonders bevorzugt:

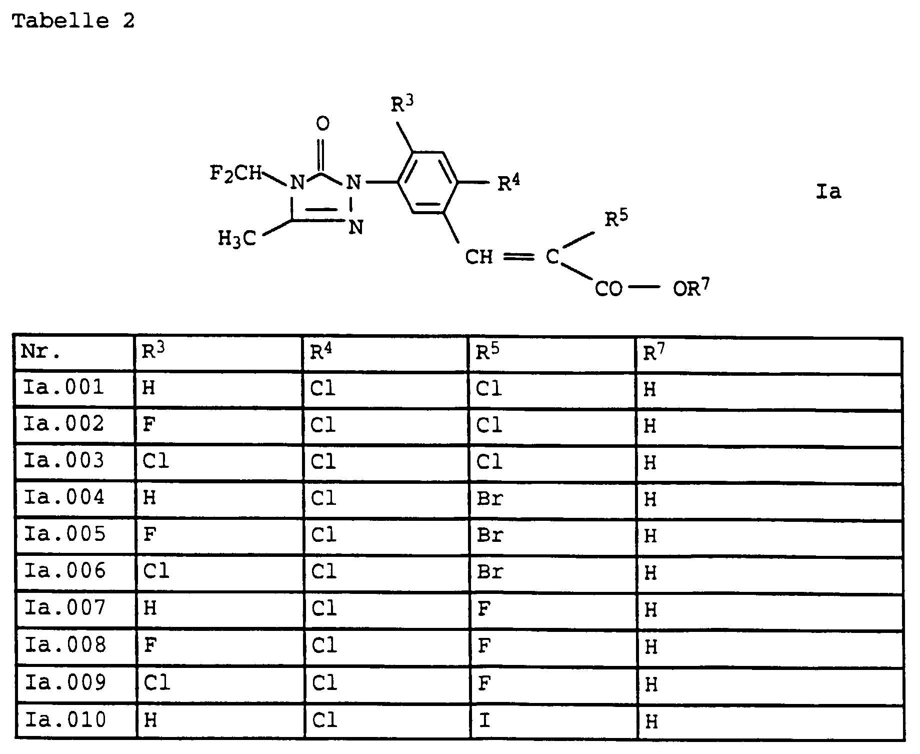

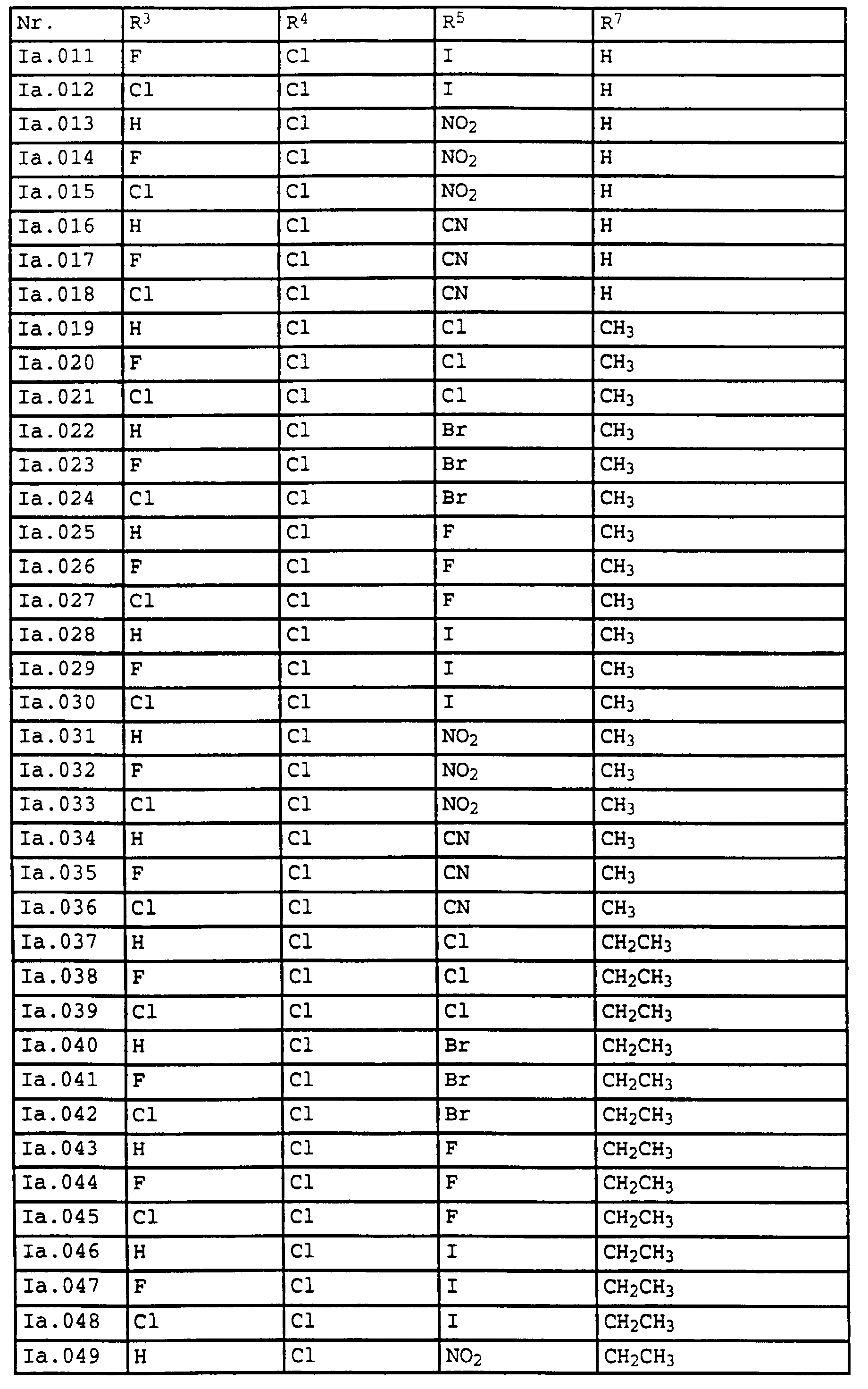

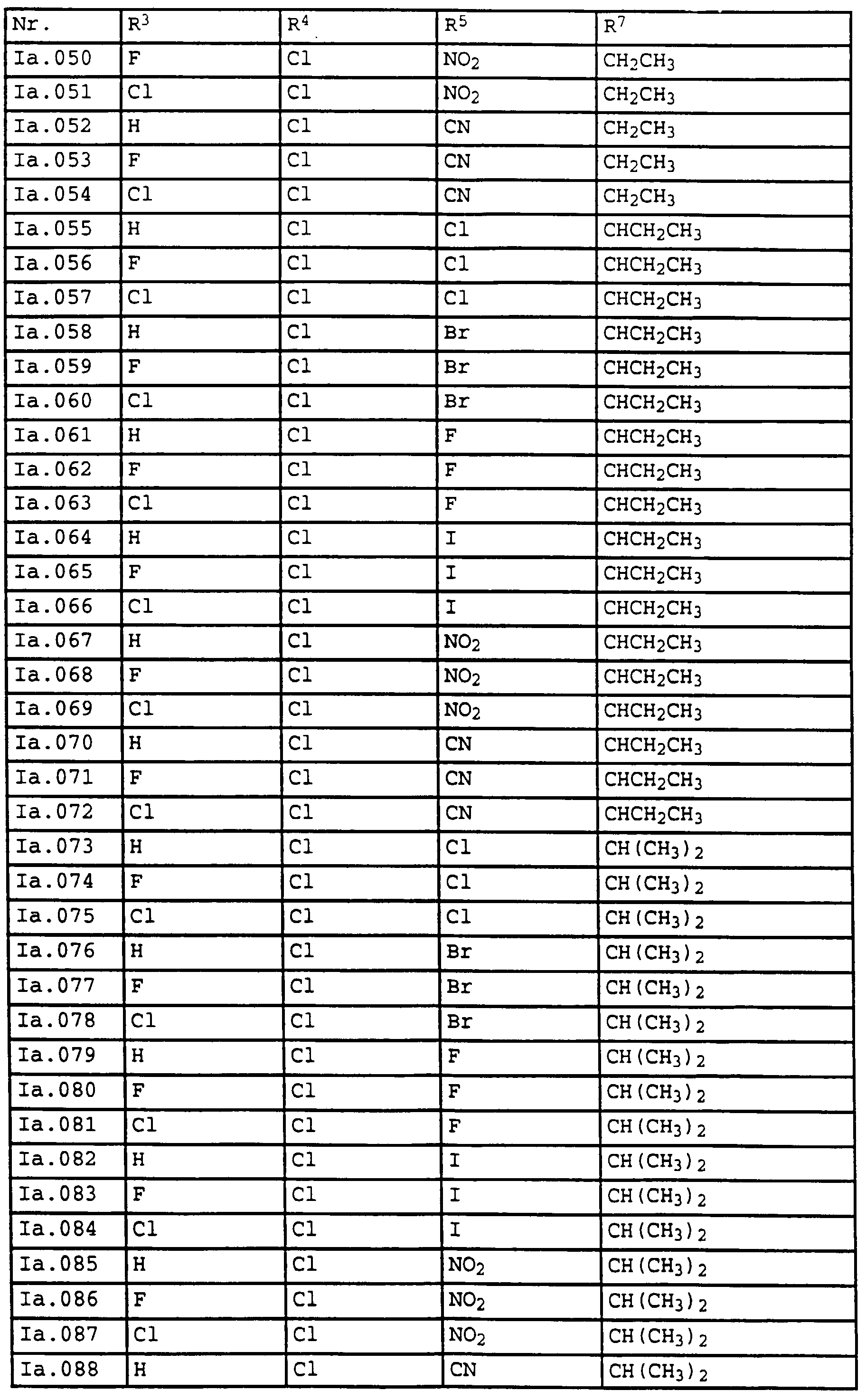

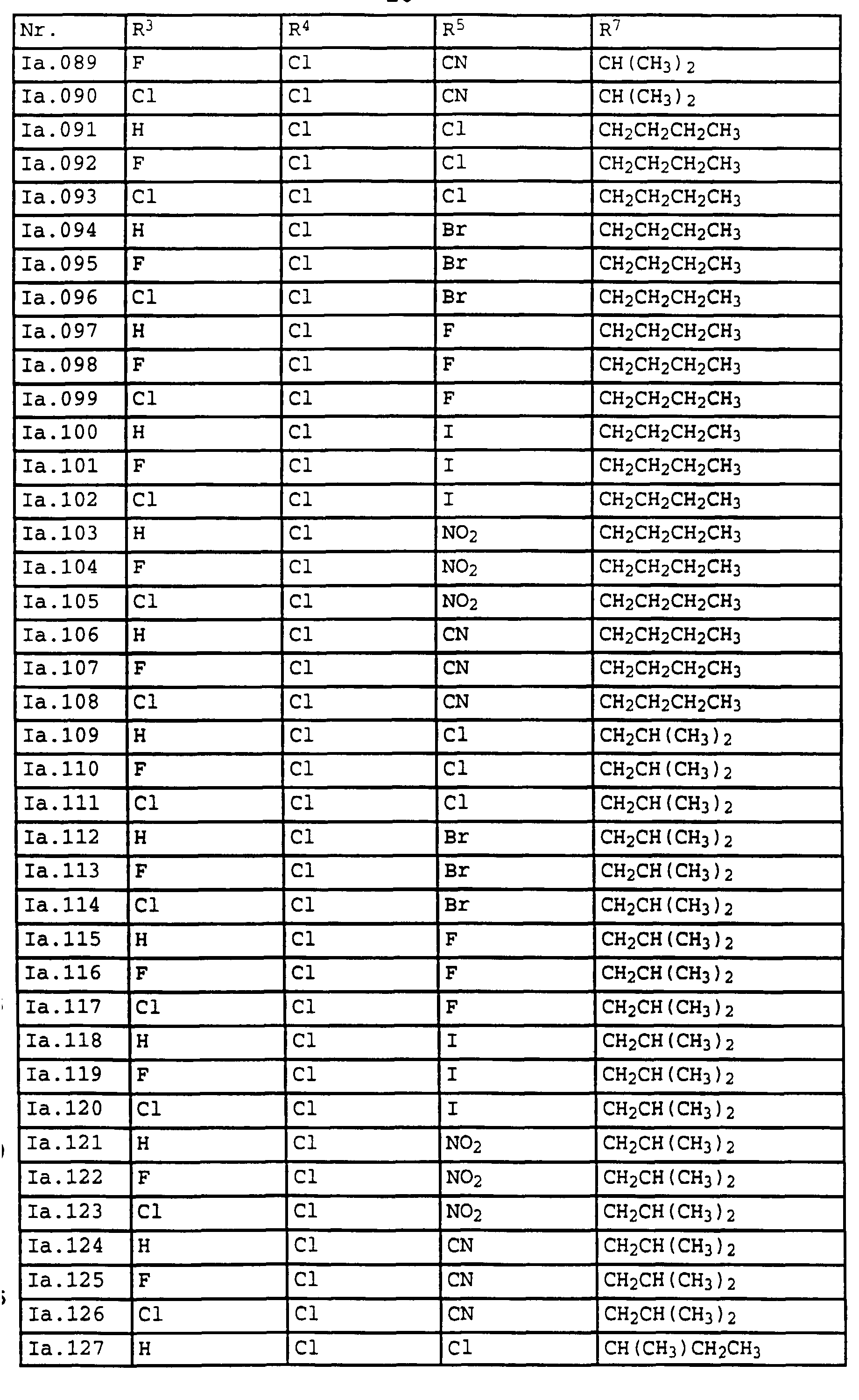

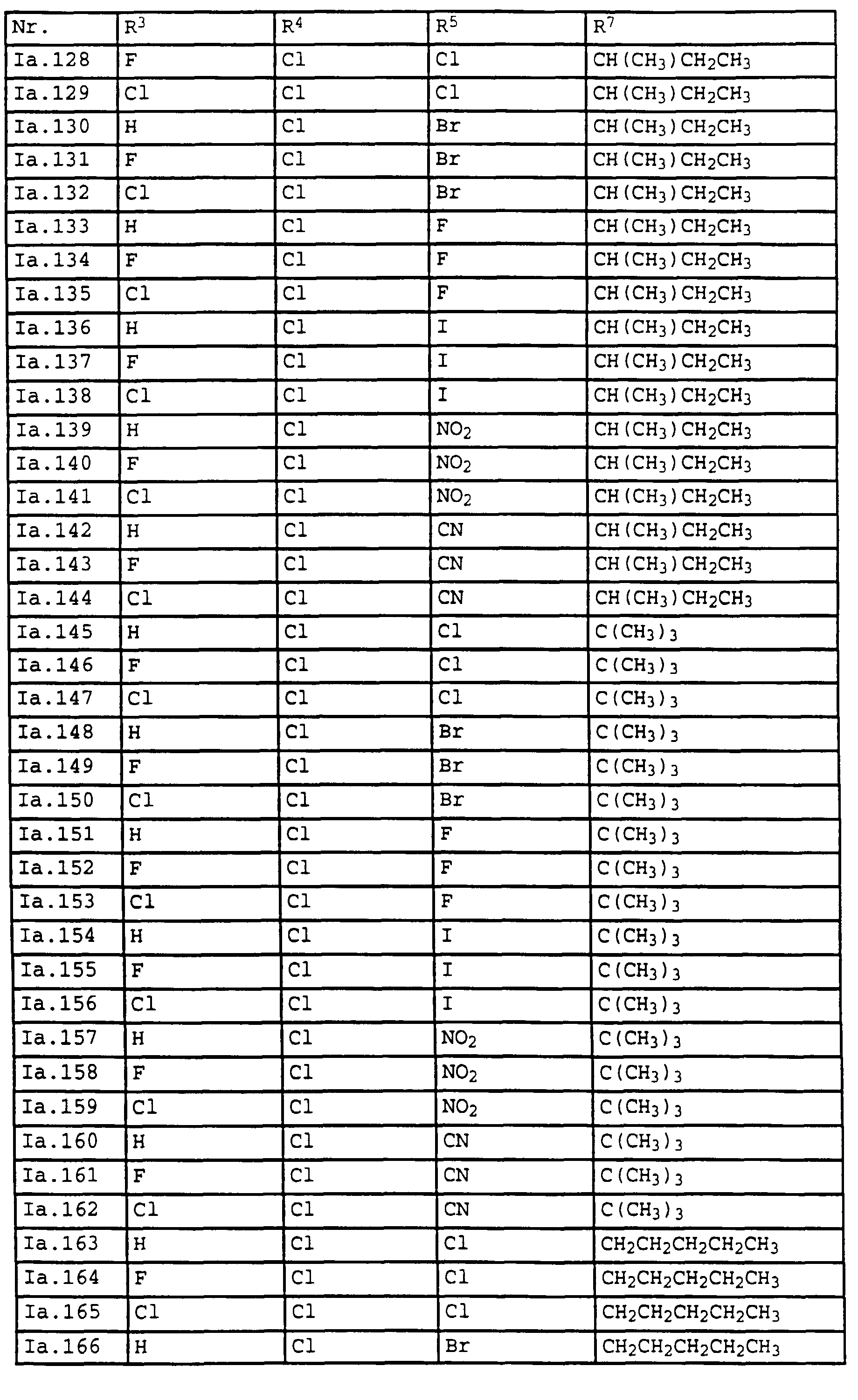

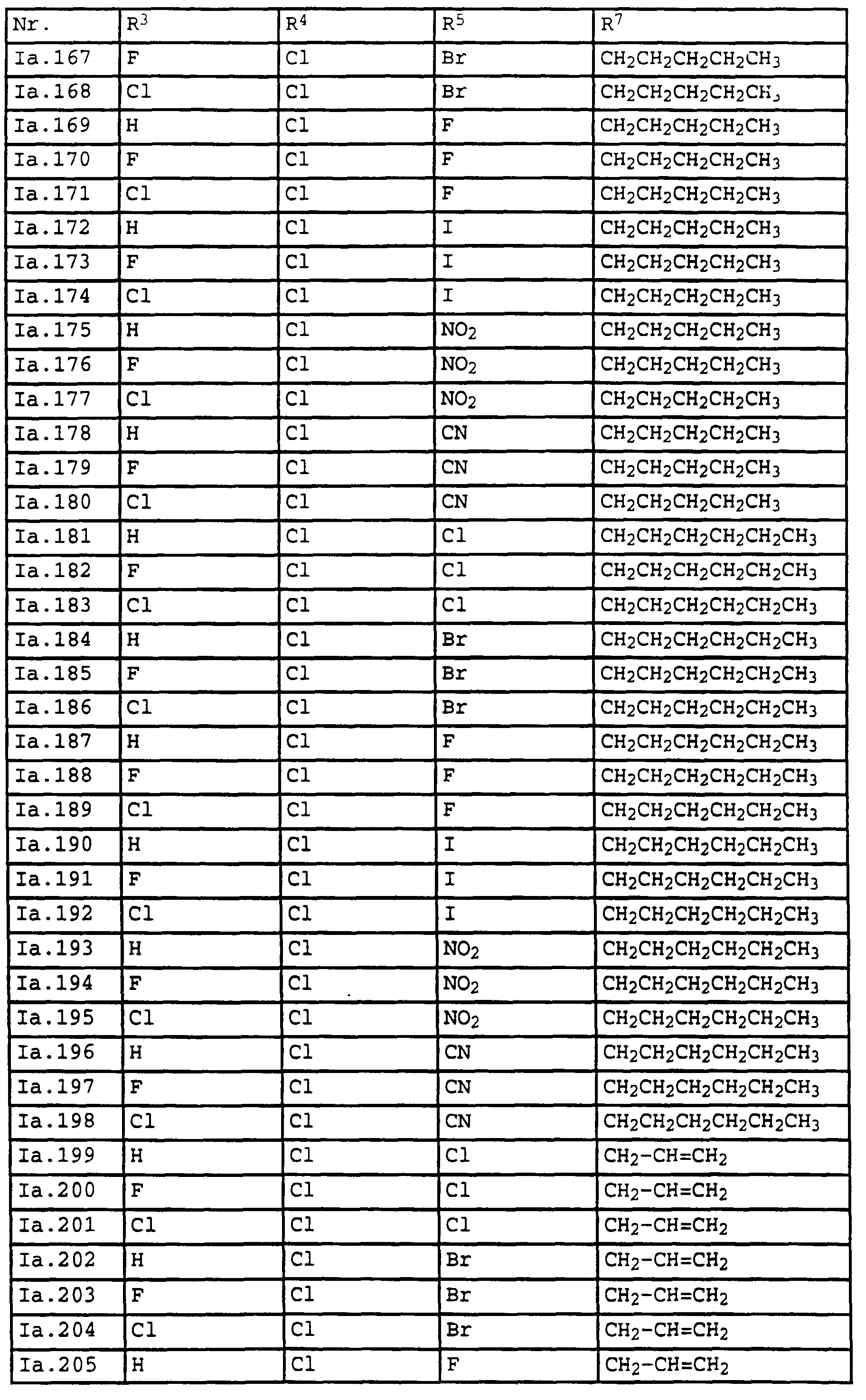

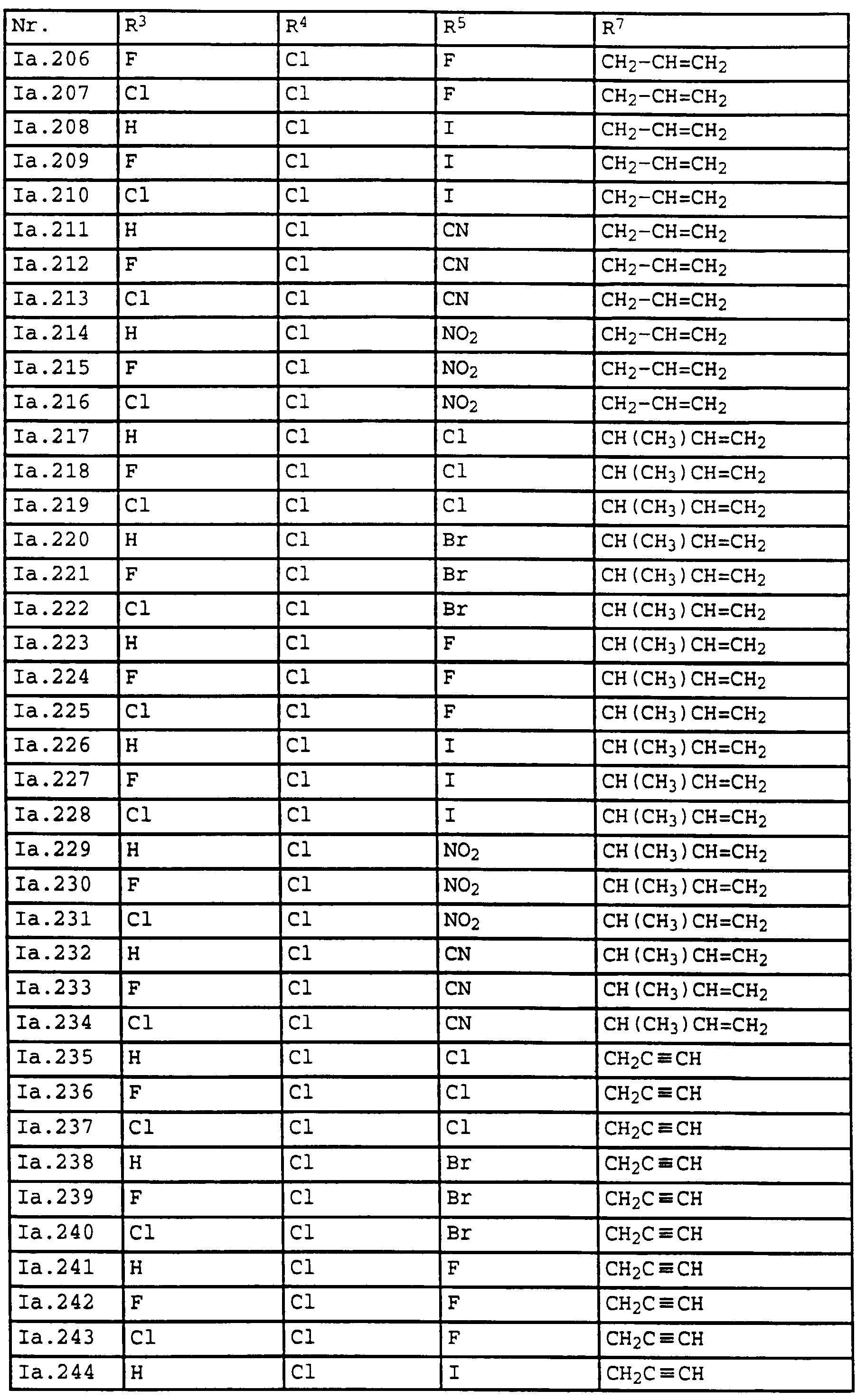

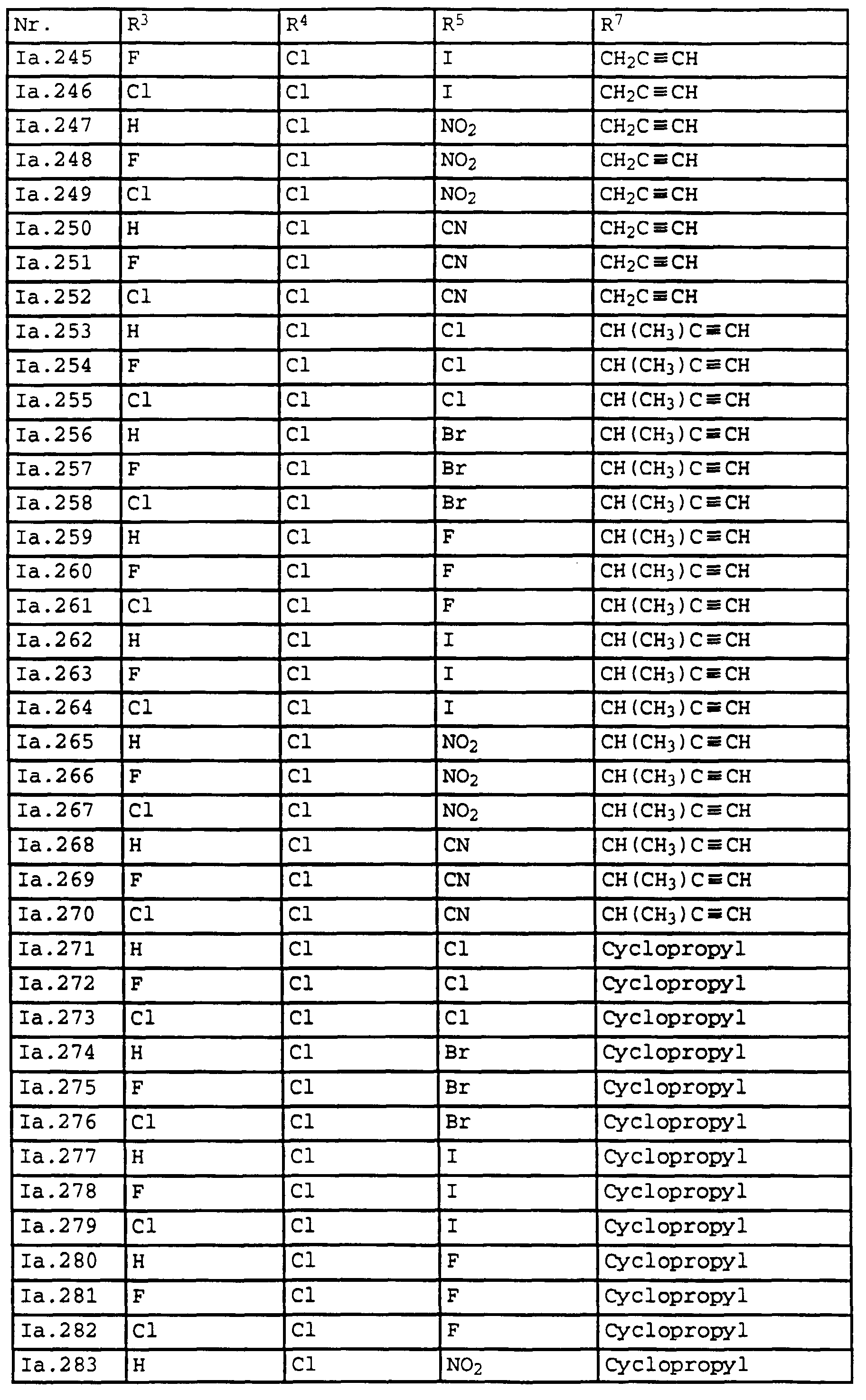

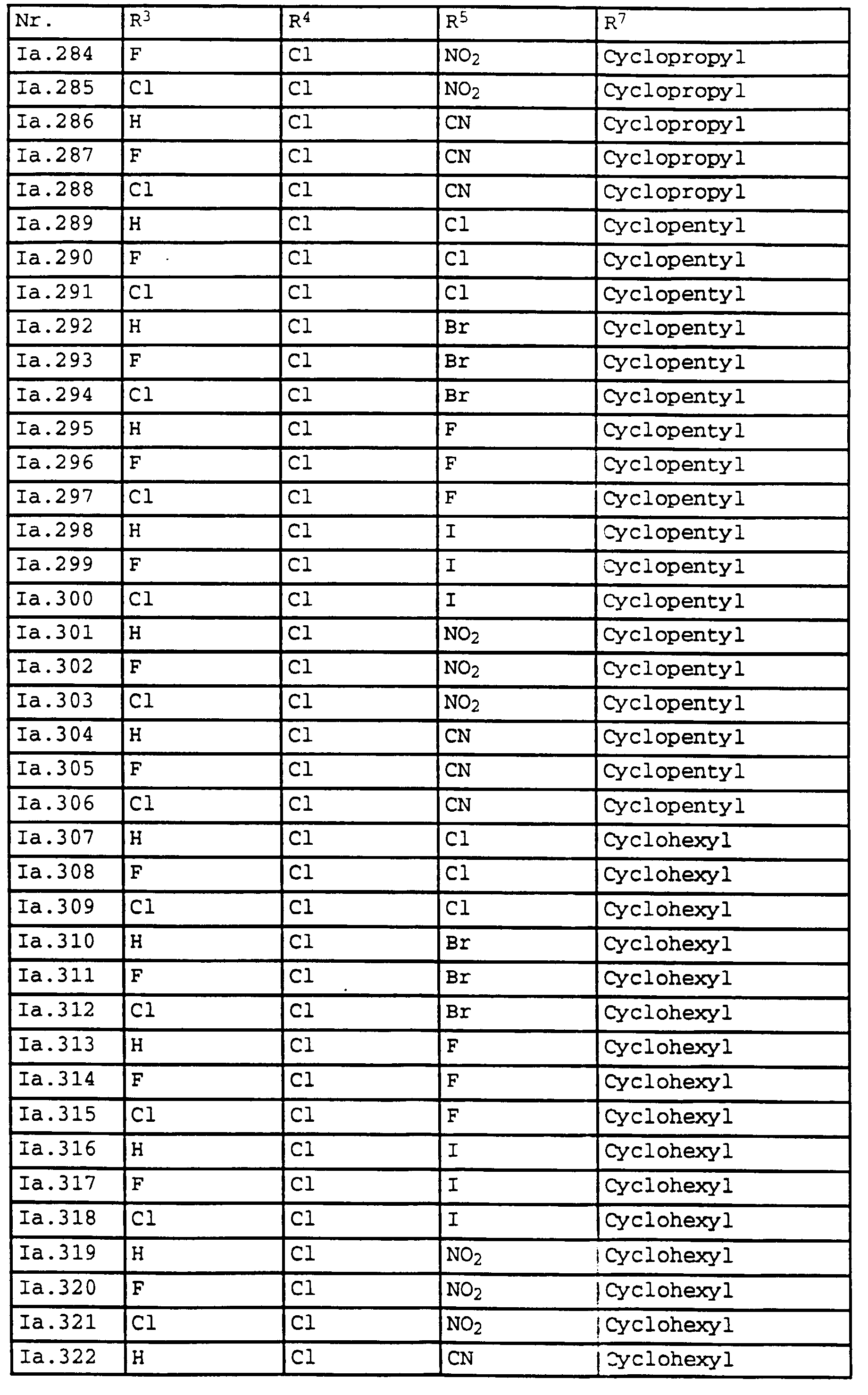

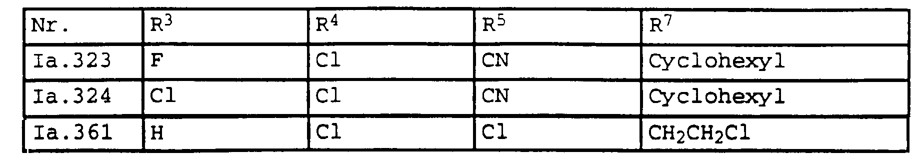

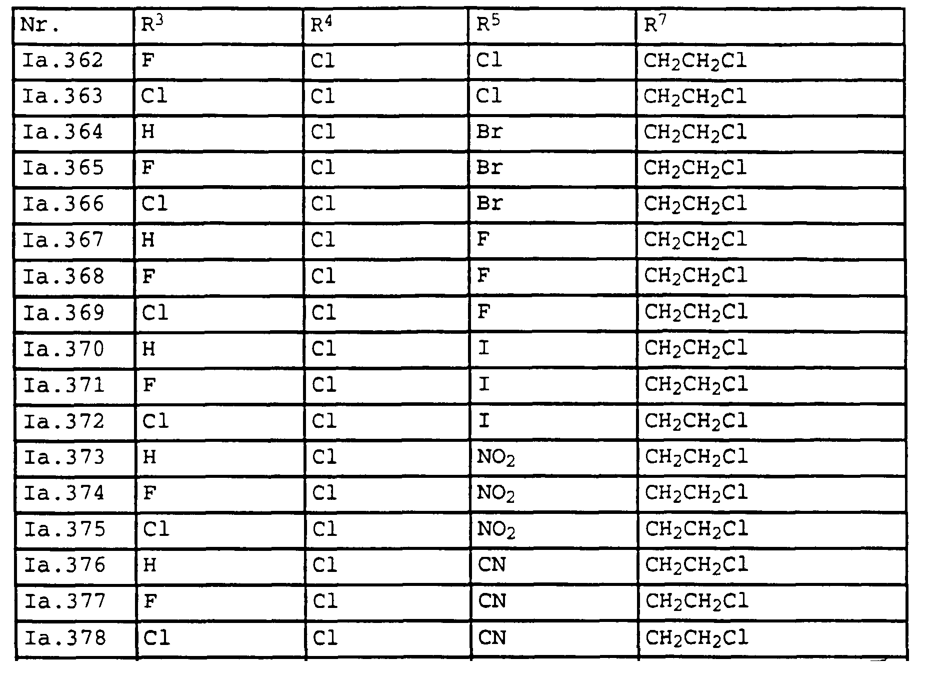

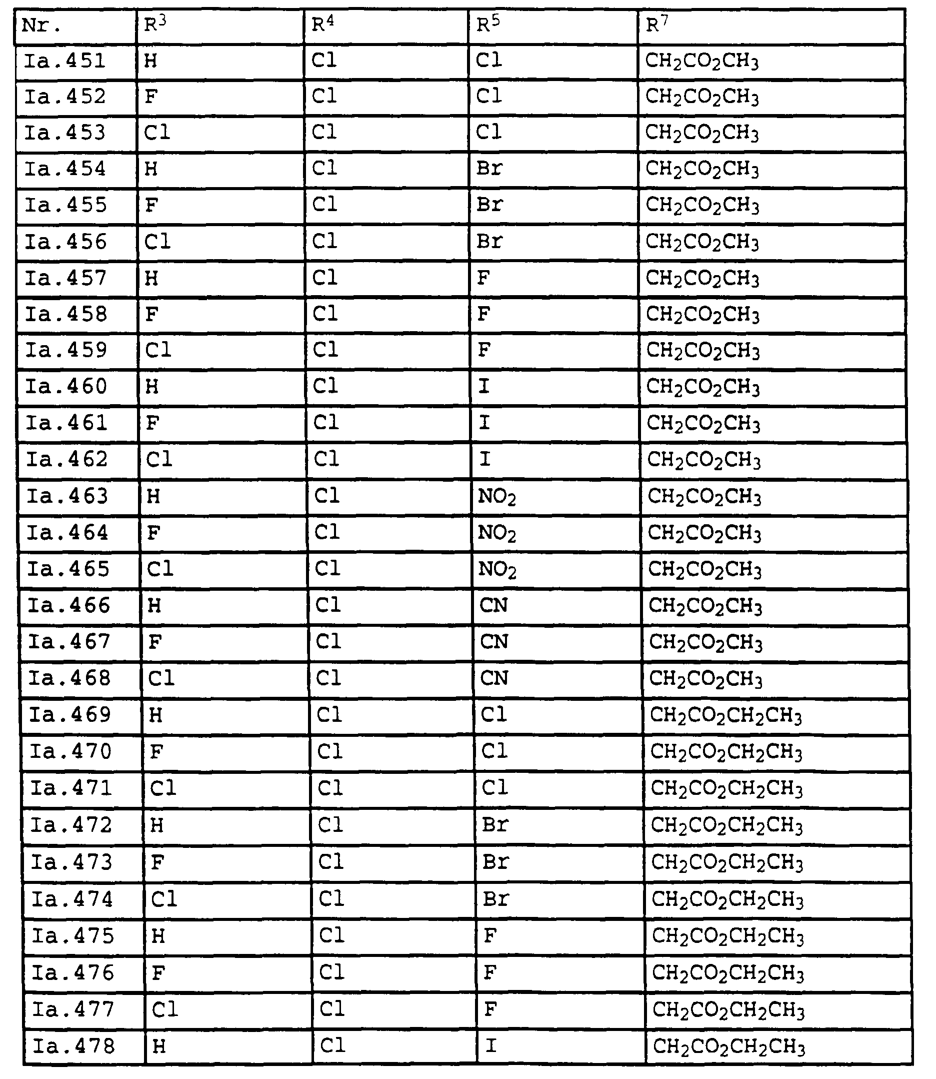

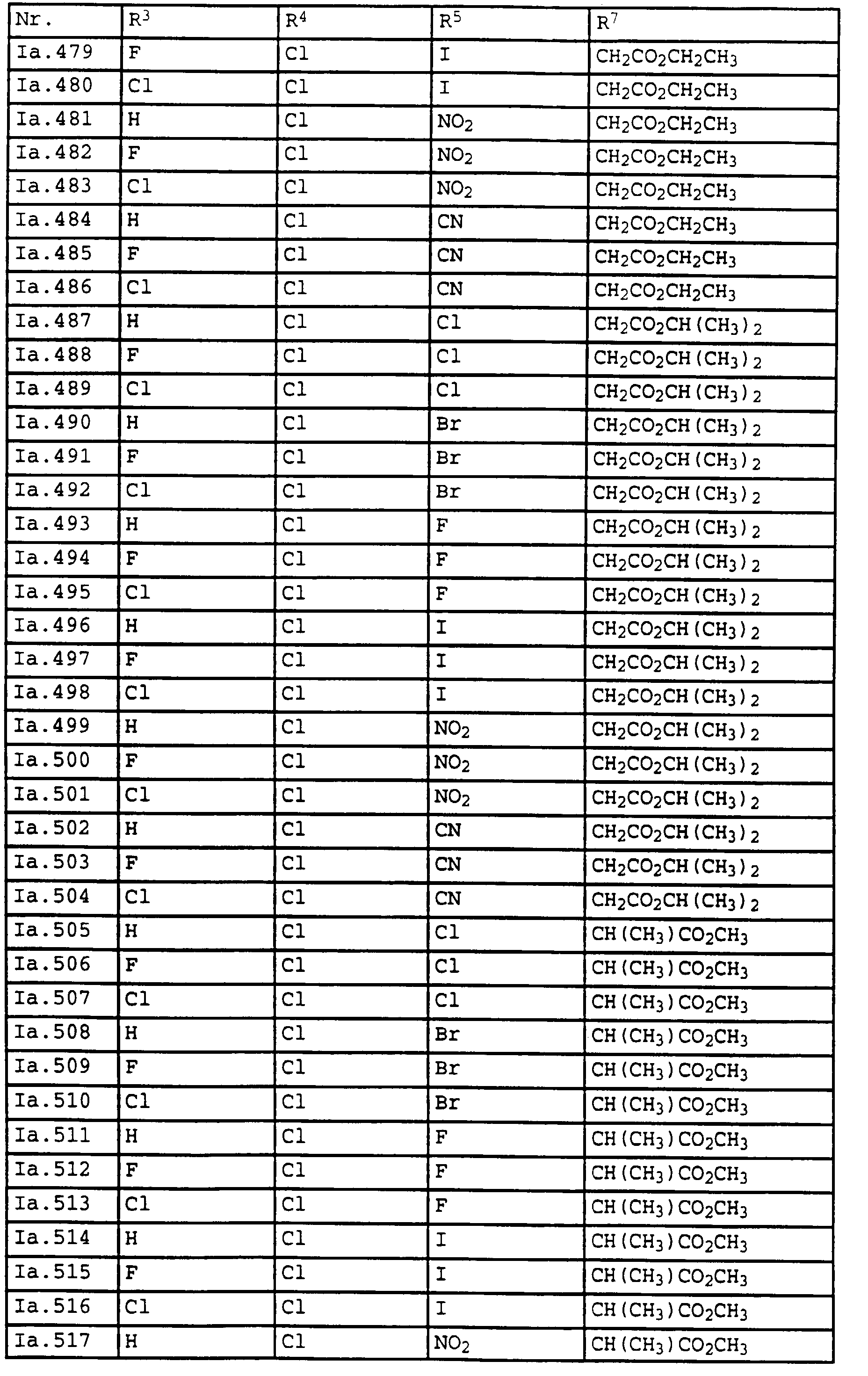

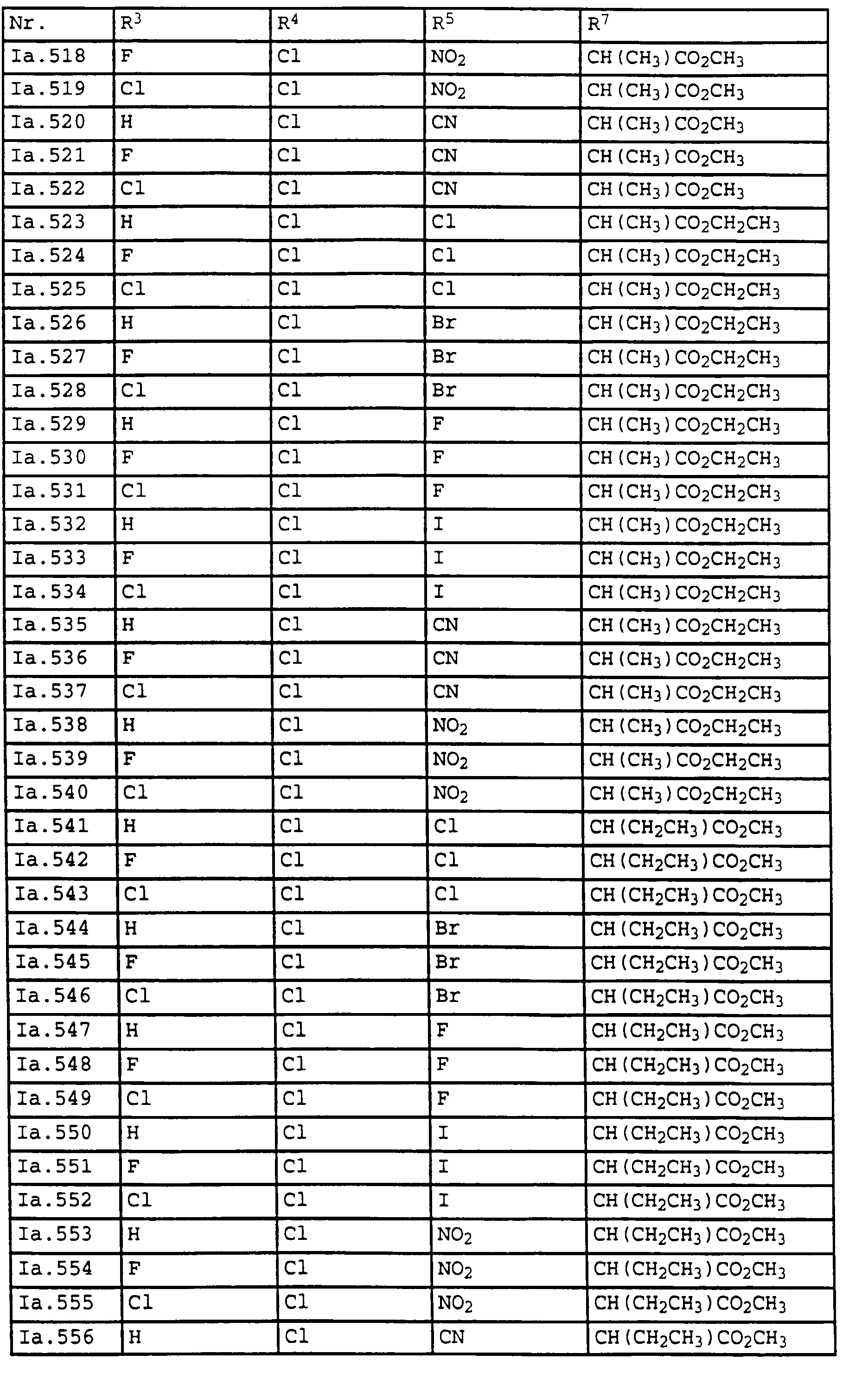

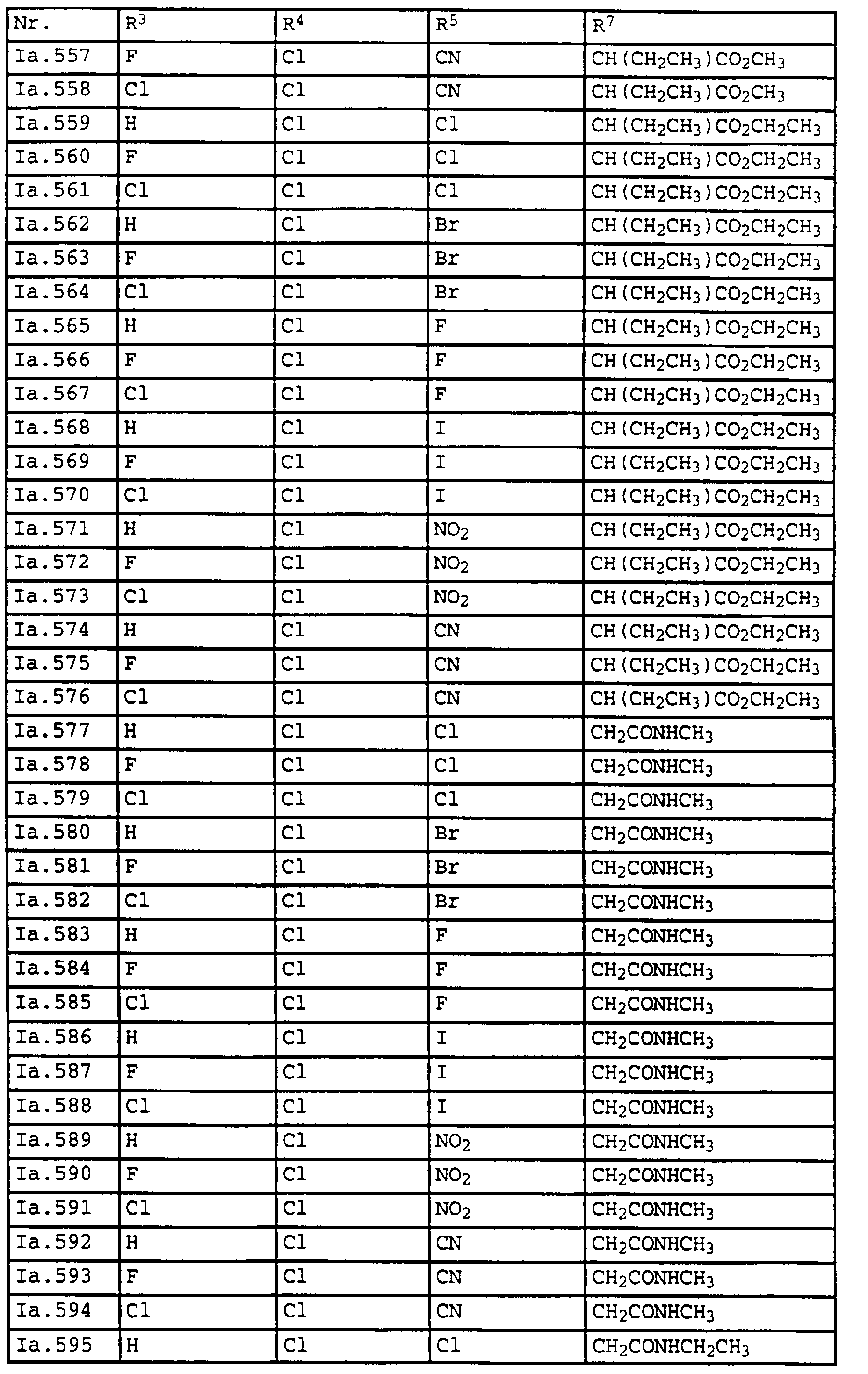

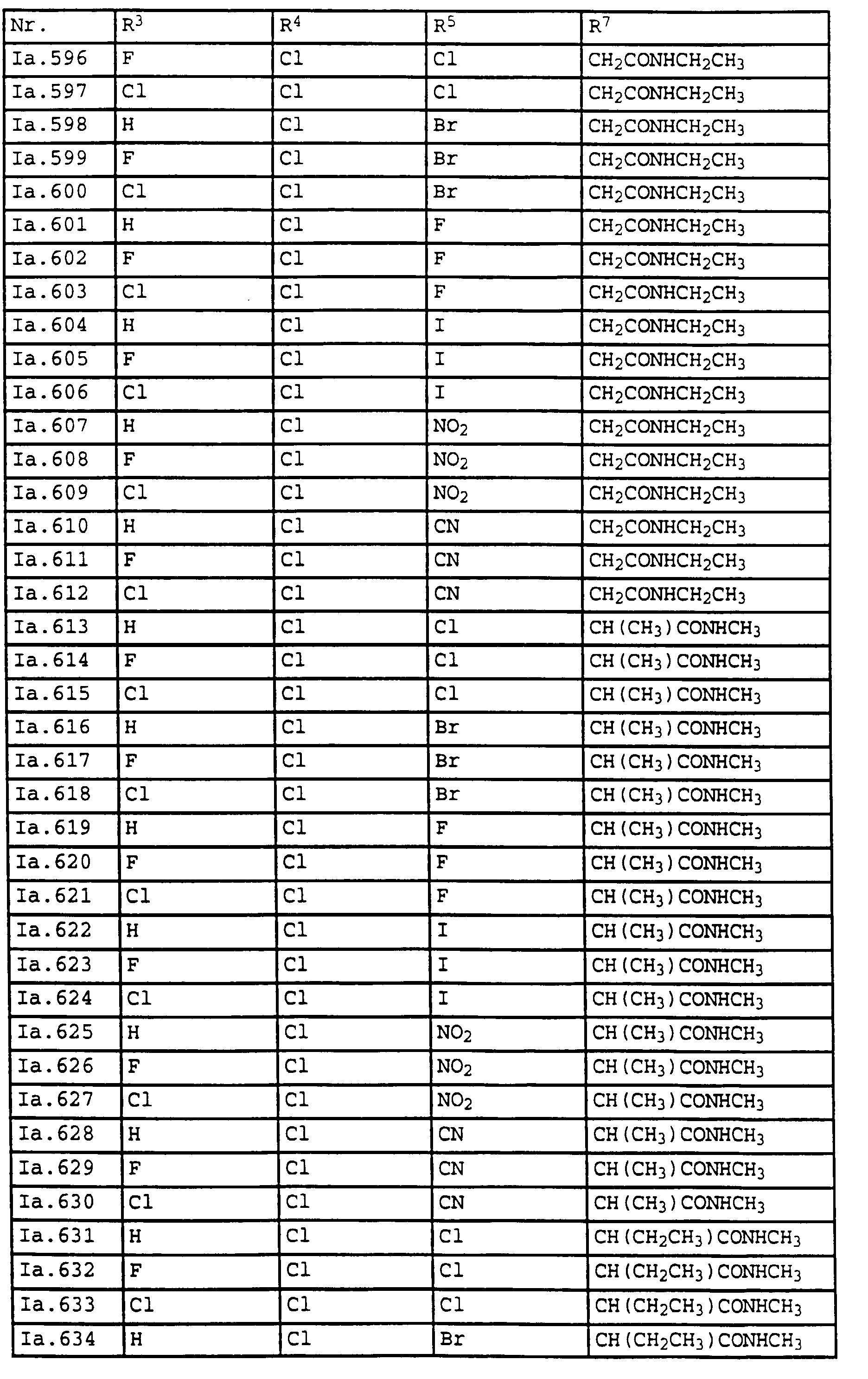

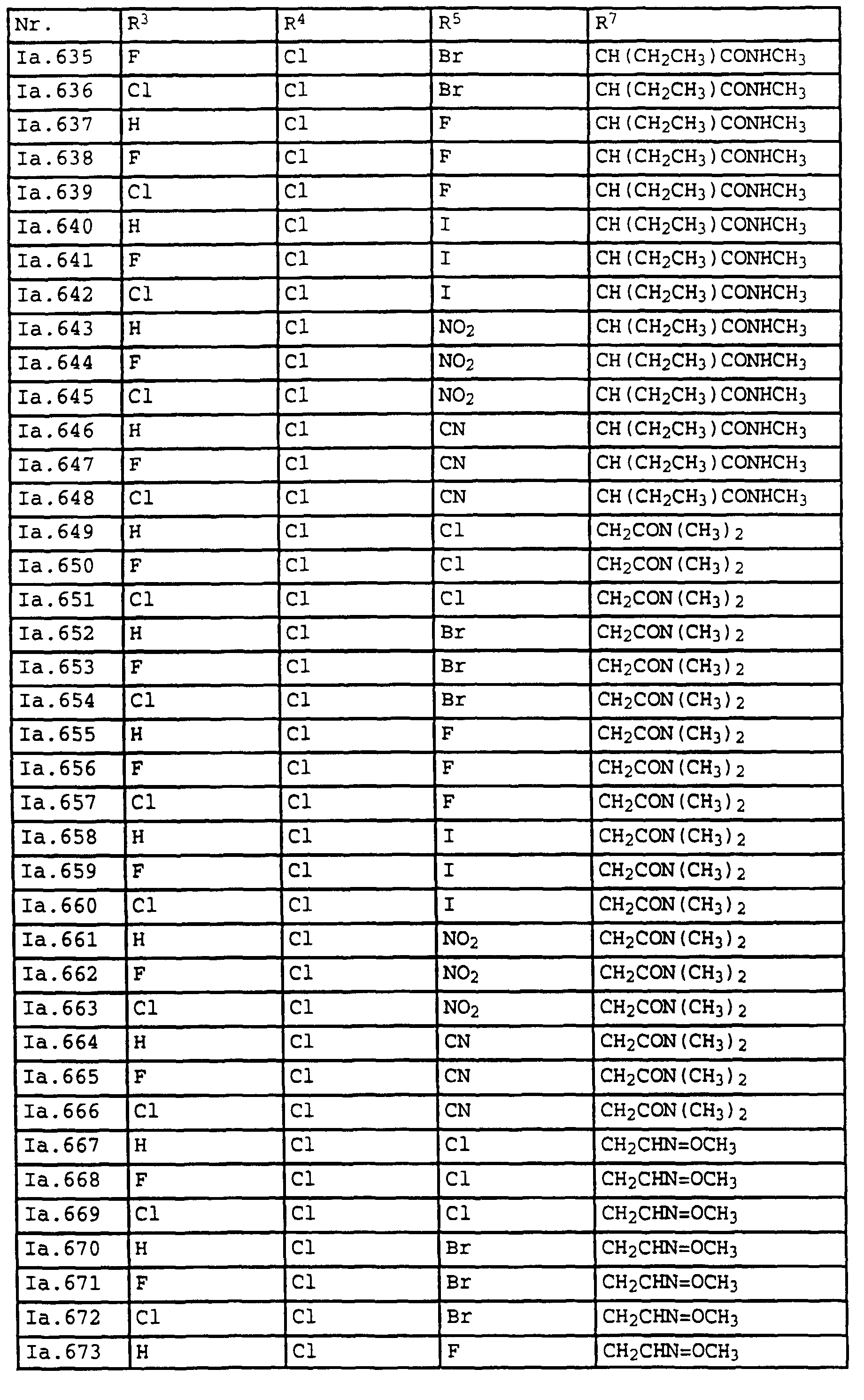

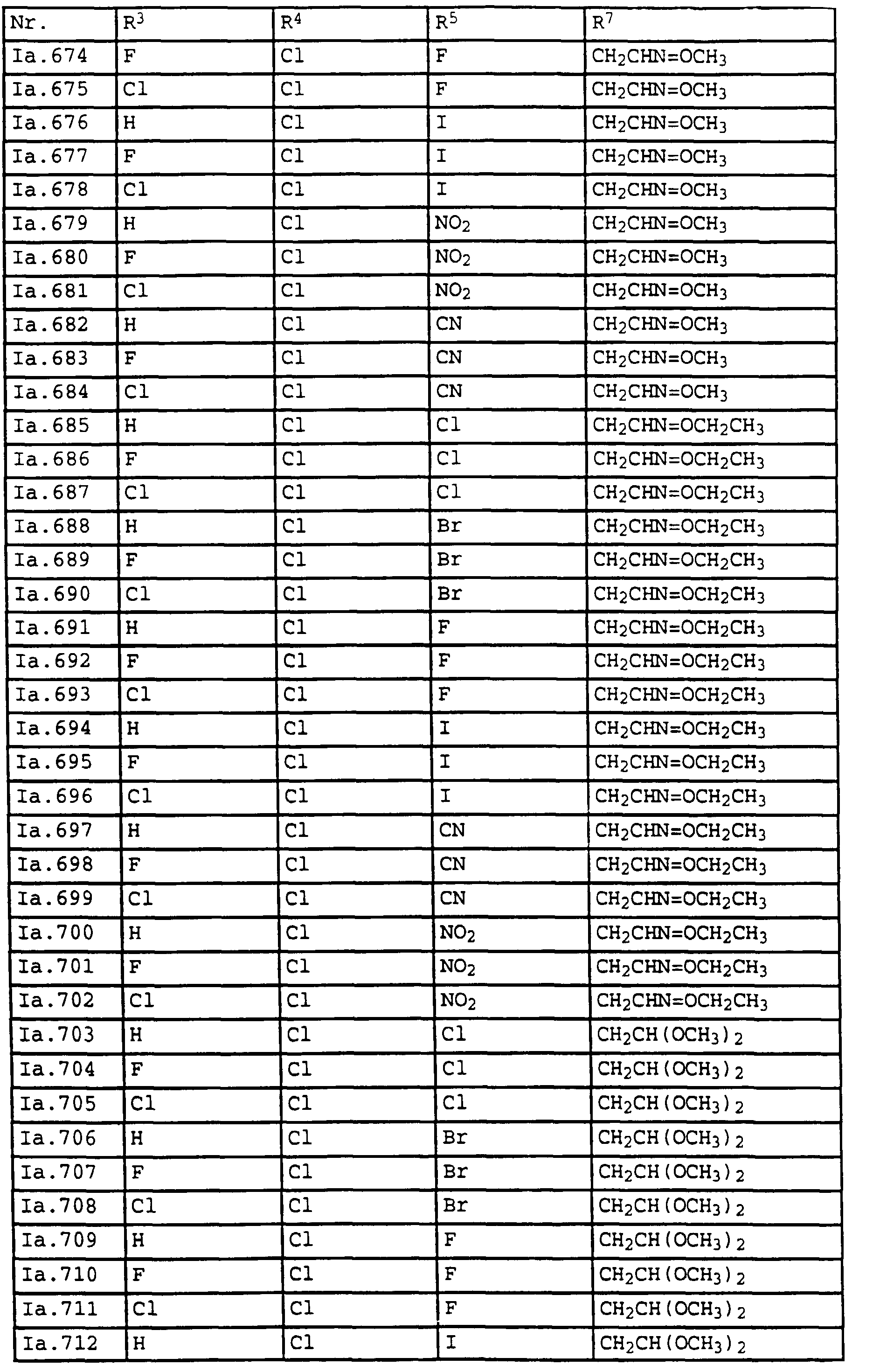

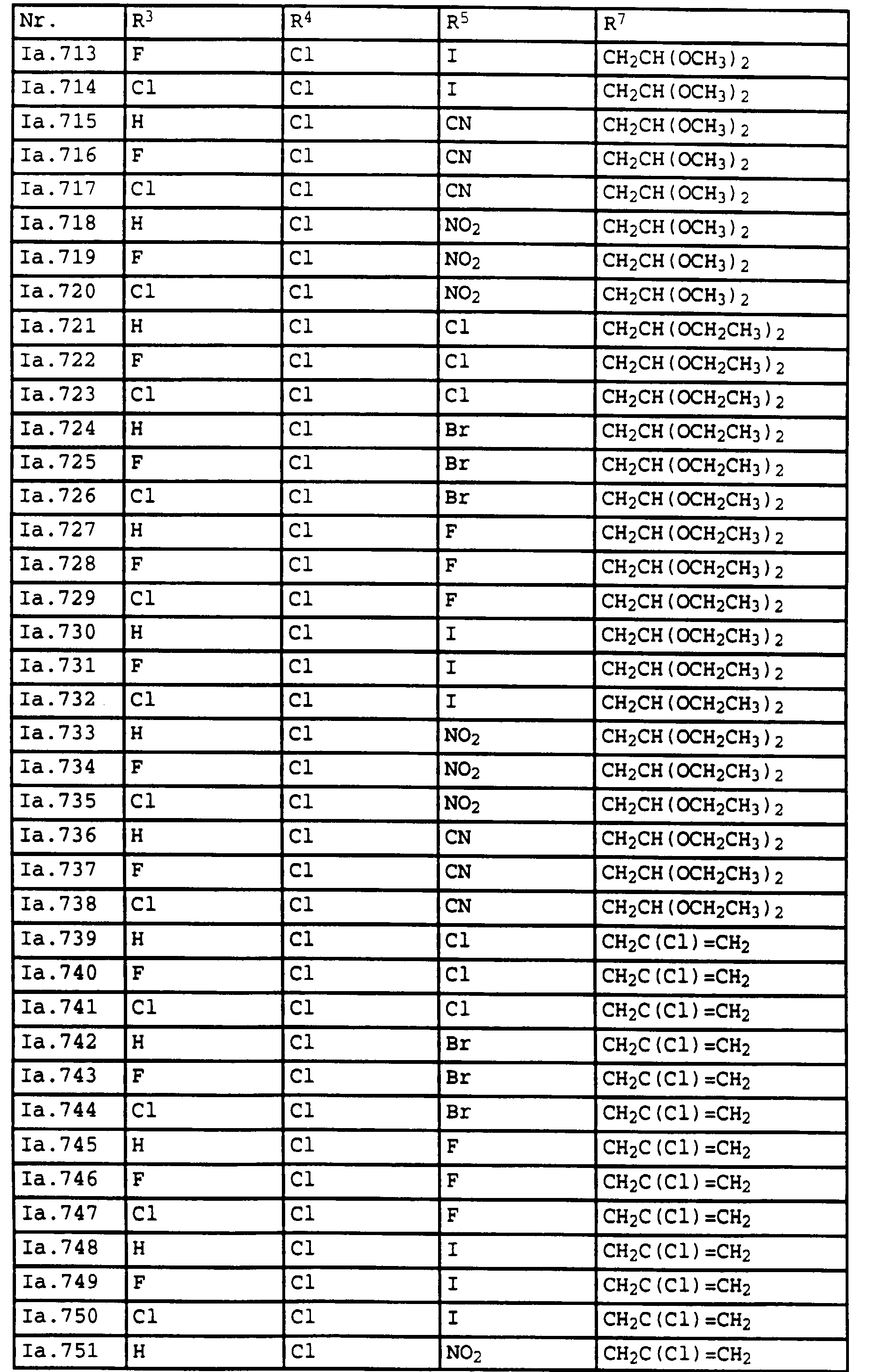

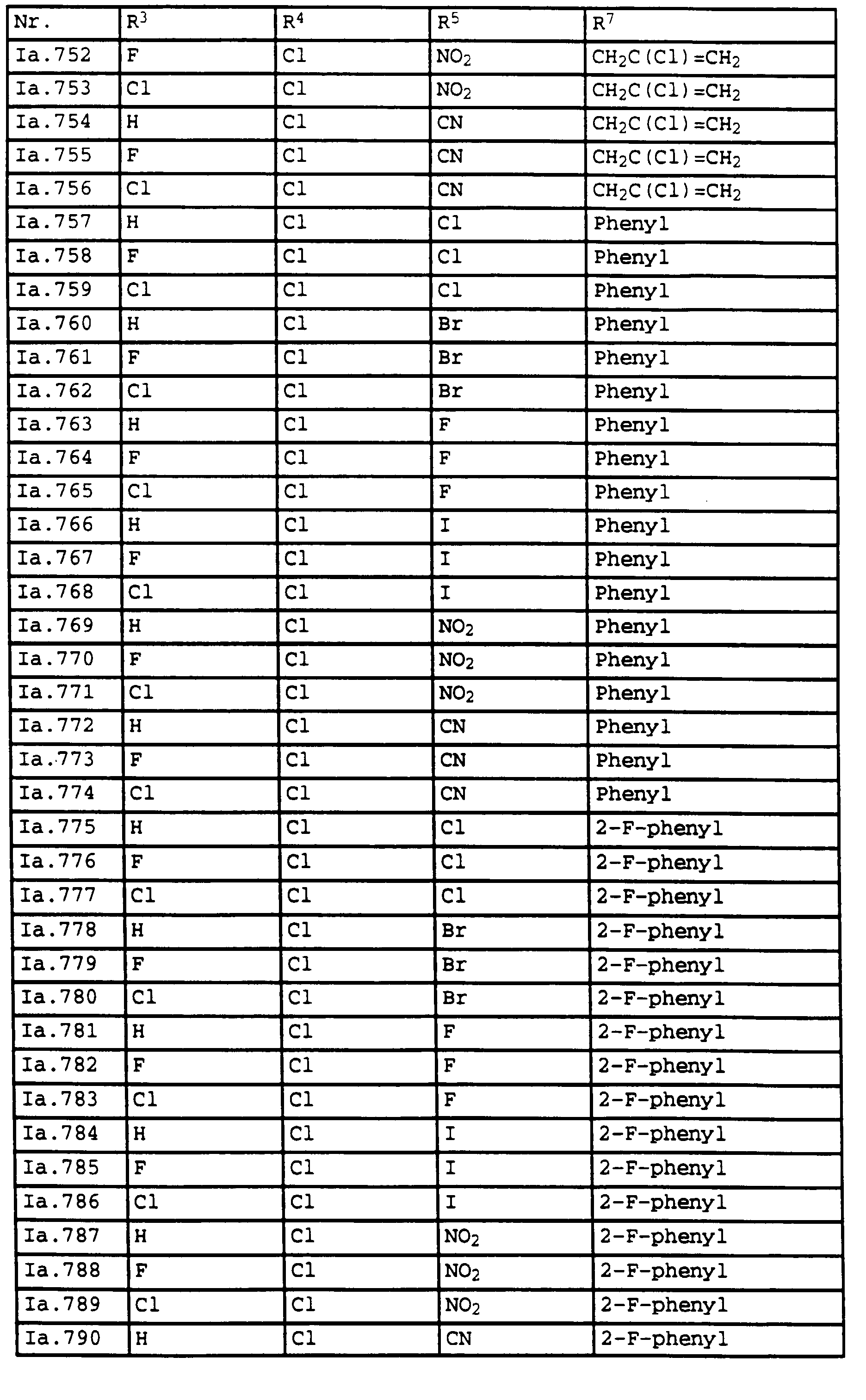

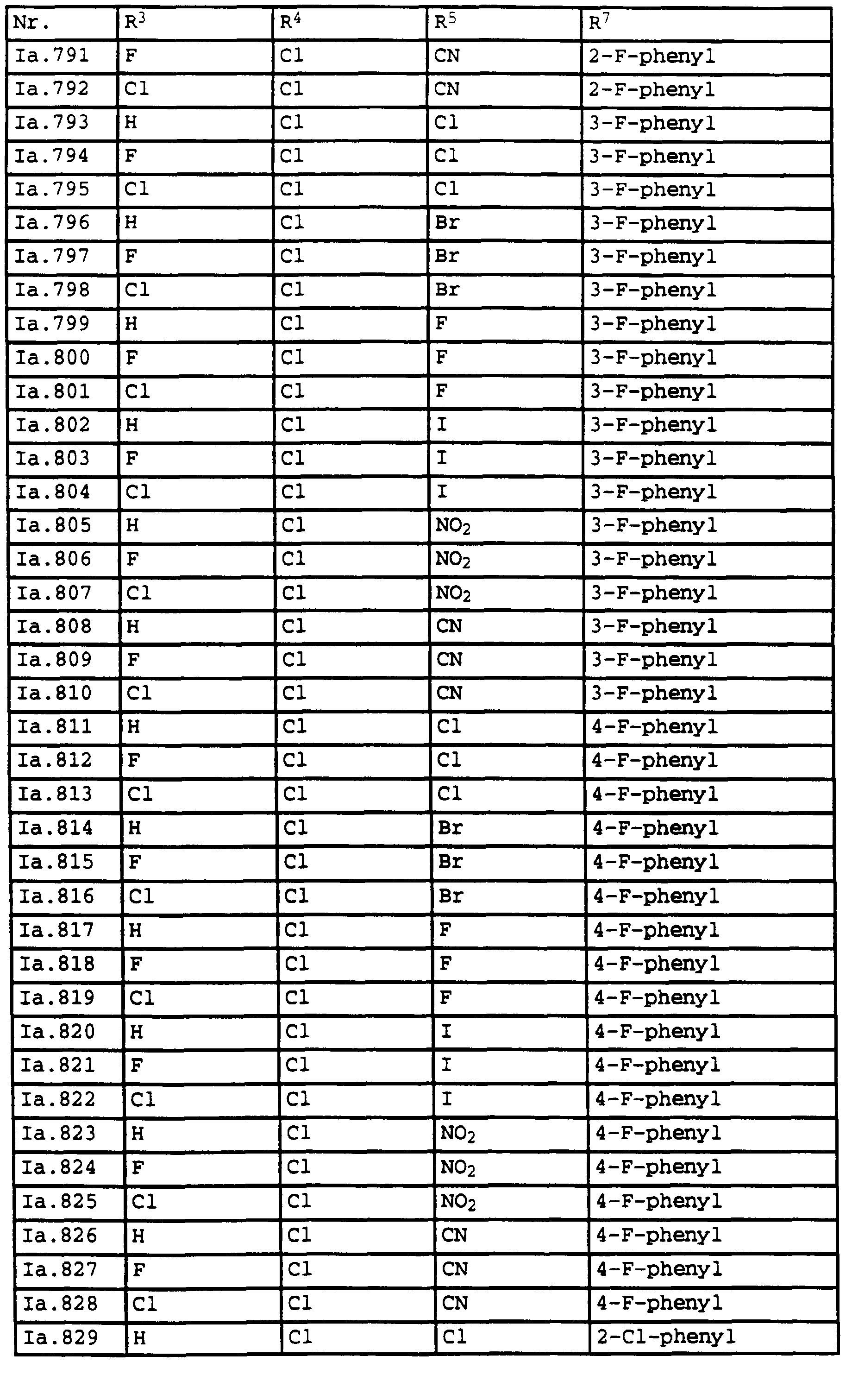

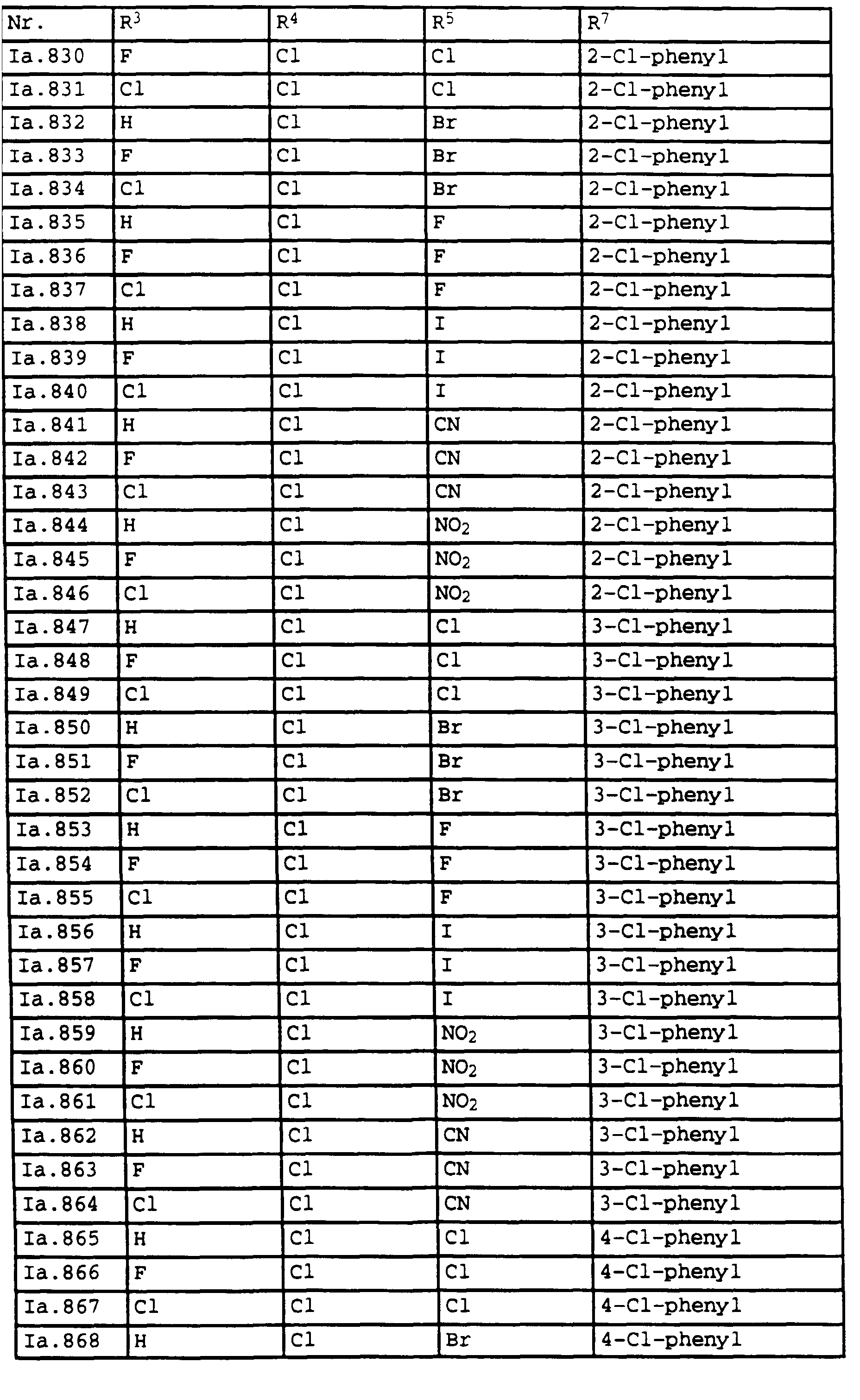

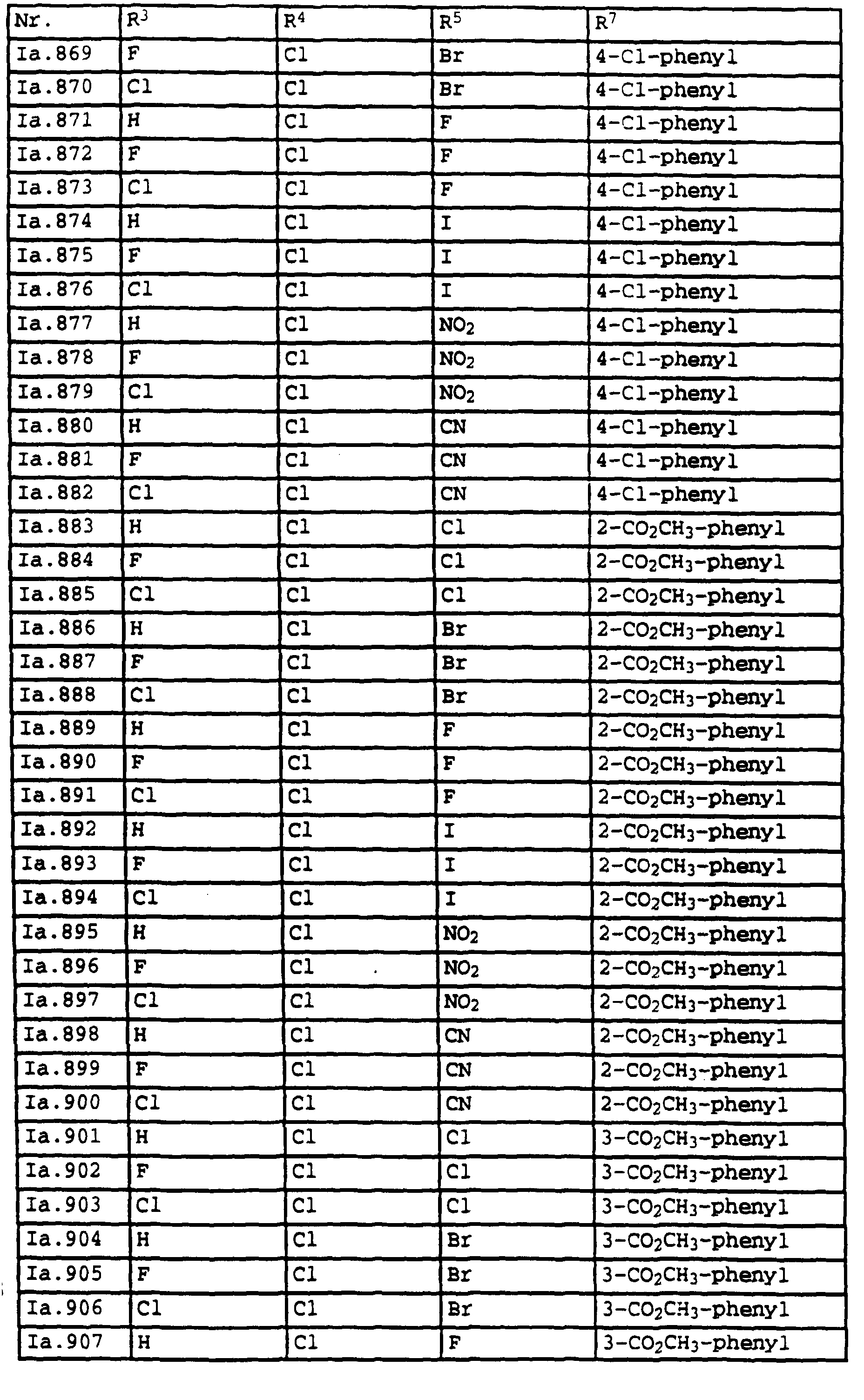

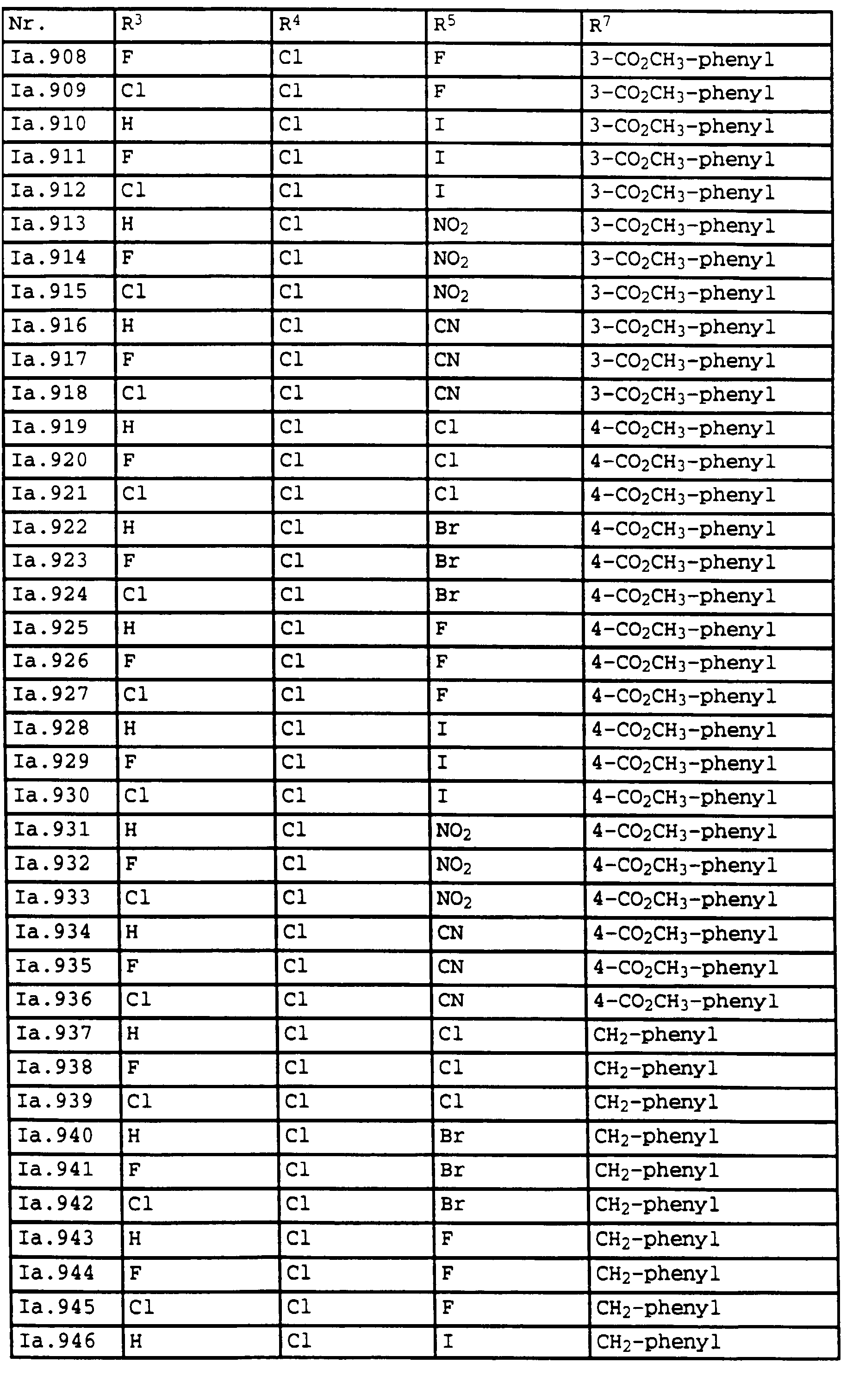

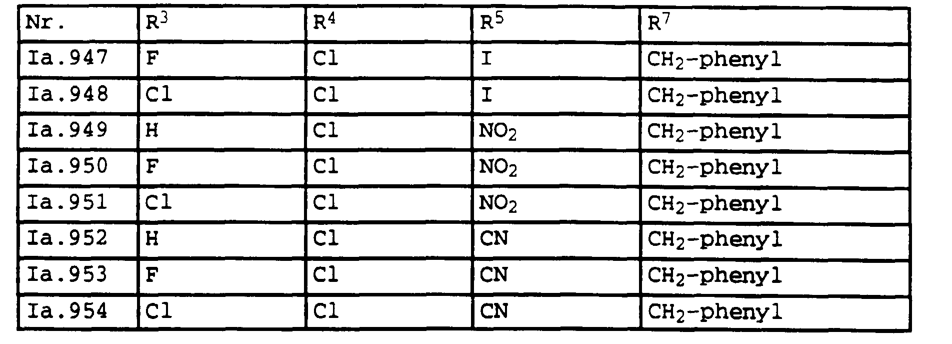

Des weiteren sind die folgenden substituierten Triazolinone I besonders bevorzugt:
- die Verbindungen Ib.001 bis 1b.954, die sich von den Verbindungen Ia.001 bis Ia.954 dadurch unterscheiden, daß X für Schwefel steht: 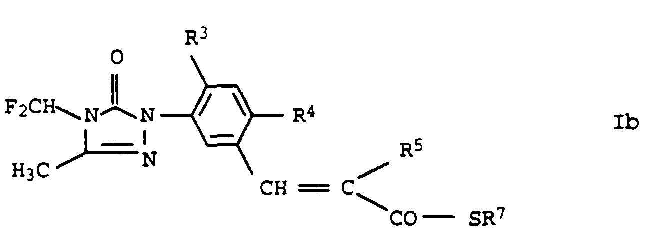
- die Verbindungen Ic.001 bis Ic.954, die sich von den Verbindungen Ia.001 bis Ia.954 dadurch unterscheiden, daß X für Amino steht: 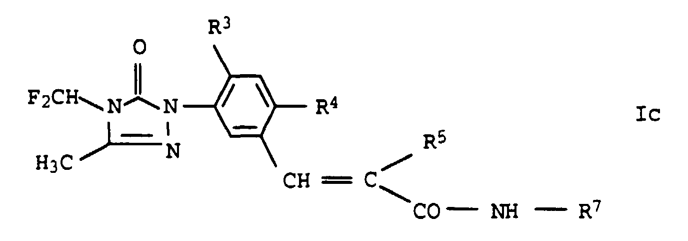
- die Verbindungen Id.001 bis Id.954, die sich von den Verbindungen Ia.001 bis Ia.954 dadurch unterscheiden, daß X für Aminomethyl steht:

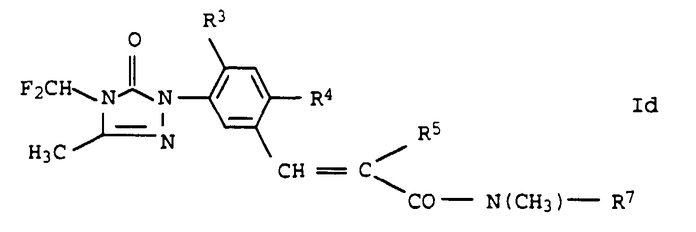

Die substituierten Triazolinone der Formel I sind auf verschiedene Weise erhältlich, beispielsweise nach einem der folgenden Verfahren:

### Verfahren A:

Diazotierung von substituierten Anilinen II und Umsetzung der hierbei erhaltenen Diazoniumsalze mit Alkinen III auf an sich bekannte Weise nach der Methode von Meerwein (vgl. z.B. N.I. Ganushchak et al., Zh. Organ. Ximii. 1980, (16) HO12, 2578-2581):

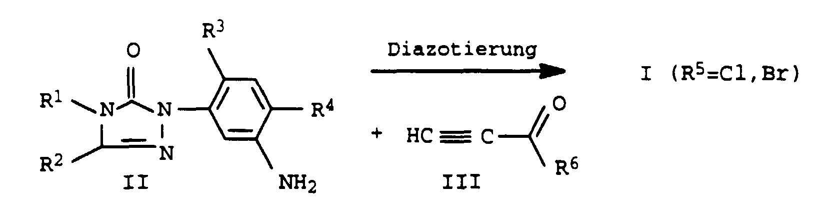

Bei diesem Reaktionstyp wird zuerst das substituierte Anilin II in ein Diazoniumsalz übergeführt. Dieses reagiert dann mit dem Alkin III in Gegenwart eines Kupfersalzes ab.

Das Phenyldiazoniumsalz wird vorteilhaft auf an sich bekannte Weise durch Umsetzung des substituierten Anilins II in einer wäßriger Säurelösung, z.B. in wäßriger Salzsäure, Bromwasserstoffsäure oder Schwefelsäure, mit einem Nitrit wie Natriumnitrit und Kaliumnitrit hergestellt und mit dem Alkin III in einem inerten Lösungsmittel in Gegenwart eines Kupferhalogenids wie Kugfer(I)chlorid, Kupfer(I)bromid, Kupfer(II)chlorid und Kupfer(II)bromid zur Reaktion gebracht.

Geeignete inerte Lösungsmittel sind z.B. Wasser, Acetonitril, Ketone wie Aceton, Diethylketon und Methylethylketon, Ether wie Dioxan und Tetrahydrofuran, ferner Alkohole wie Methanol und Ethanol.

Eine weitere Möglichkeit der Herstellung des Phenyldiazoniumsalzes besteht darin, das Anilin II in einem wasserfreien System, beispielsweise in Eisessig, der Chlorwasserstoff enthält, in Dioxan, absolutem Ethanol, Tetrahydrofuran, Acetonitril oder in Aceton, mit einem Salpetrigsäureeester wie tert.-Butylnitrit und Isopentylnitrit umzusetzen. In diesem Fall kann die Diazotierung in Gegenwart des Alkins III und des Kupferhalogenids stattfinden.

Die Reaktionstemperatur liegt normalerweise bei (-30) bis 80°C.

Üblicherweise werden die Komponenten der Diazotierungsreaktion in etwa stöchiometrischem Verhältnis eingesetzt, jedoch kann auch ein Überschuß einer der Komponenten, z.B. im Hinblick auf eine möglichst vollständige Umsetzung der anderen Komponente, vorteilhaft sein.

Das Alkin III kann in äquimolaren Mengen, im Über- oder Unterschuß, bezogen auf das Phenyldiazoniumsalz, eingesetzt werden. Im allgemeinen hat sich ein großer Überschuß an Alkin III, bezogen auf das Phenyldiazoniumsalz, als besonders vorteilhaft erwiesen. Das Kupferhalogenid wird normalerweise im stöchiometrischen Verhältnis eingesetzt, jedoch ist auch ein Über- oder Unterschuß möglich.

### Verfahren B:

Umsetzung eines substituierten Benzaldehyds IV mit einem Ylid der Formel V auf an sich bekannte Weise (vgl. z.B. R.S. Mali und V.J. Yadav, Synthesis (10), 862 (1984)):

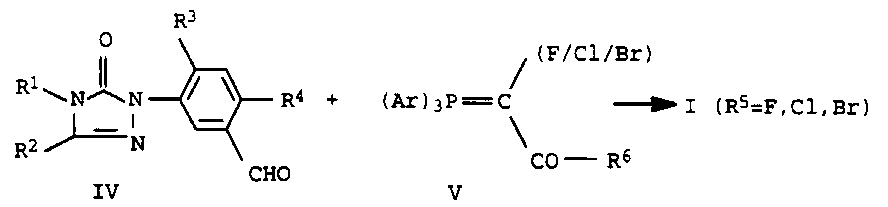

Ar steht für einen aromatischen Rest, der gewünschtenfalls substituiert sein kann, vorzugsweise für den Phenylrest.

Geeignete Lösungsmittel sind z.B. aromatische Kohlenwasserstoffe wie Benzol und Toluol, Ether wie Diethylether, Tetrahydrofuran und Dioxan, dipolar aprotische Lösungsmittel wie Dimethylsulfoxid und Dimethylformamid, oder protische Lösungsmittel wie Methanol und Ethanol. Auch Mischungen der genannten Solventien kommen in Betracht.

Normalerweise arbeitet man bei 0 °C bis zur Siedetemperatur des jeweiligen Reaktionsgemisches.

Üblicherweise werden substituierter Benzaldehyd und Ylid in etwa stöchiometrischen Mengen eingesetzt, jedoch besteht auch die Möglichkeit, eine der Komponenten im Überschuß einzusetzen.

### Verfahren C:

Umsetzung eines substituierten Benzaldehyds IV mit einer CH-aciden Verbindung NO₂-CH₂-COR⁶ oder NC-CH₂-COR⁶ auf an sich bekannte Weise (vgl. z.B. J. March, "Advanced Organic Chemistry", S. 835 ff.):

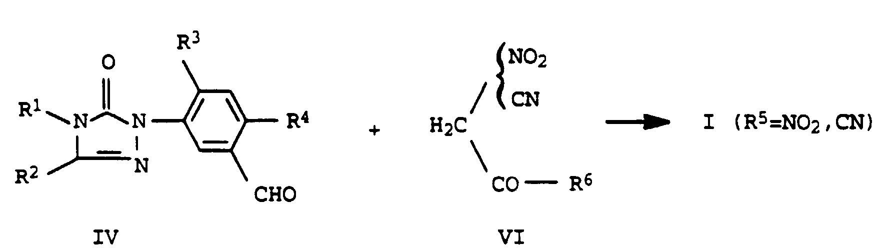

In Abhängigkeit von den jeweiligen Substituenten und den Reaktionsbedingungen kann es vorteilhaft sein, in Gegenwart einer katalytischen oder in etwa äquivalenten Menge Base, bezogen auf VI, oder in Gegenwart einer Säure zu arbeiten.

Als Basen kommen z.B. Metallalkoholate wie Natriummethanolat, Natriumethanolat und Kalium-tert.-butylat, aromatische oder aliphatische Stickstoffbasen wie Pyridin, Piperidin, Triethylamin, Ammoniumacetat und β-Alanin, ferner Metallhydride wie Natriumhydrid und Kaliumhydrid in Betracht.

Brauchbare Säuren sind insbesondere Essigsäure und Propionsäure.

Die Reaktion wird entweder lösungsmittelfrei oder in einem Uberschuß an Base oder Säure oder in einem inerten Lösungs- oder Verdünnungsmittel vorgenommen. Je nach Reaktionsbedingungen eignen sich beispielsweise Alkohole wie Methanol und Ethanol oder Ether wie Diethylether und Methyl-tert.-butylether als Solventien.

Die Reaktion wird allgemein bei 0 °C bis zu Siedetemperatur des Reaktionsgemisches vorgenommen.

Üblicherweise werden die Ausgangsverbindungen IV und VI in etwa äquivalenten Mengen eingesetzt, jedoch kann auch eine der Komponenten im Überschuß eingesetzt werden.

### Verfahren D:

Umsetzung einer aktivierten Carbonsäure VII mit einem Nukleophil VIII auf an sich bekannte Weise (vgl. z.B. J. March, "Advanced Organic Chemistry", S. 348 ff.):

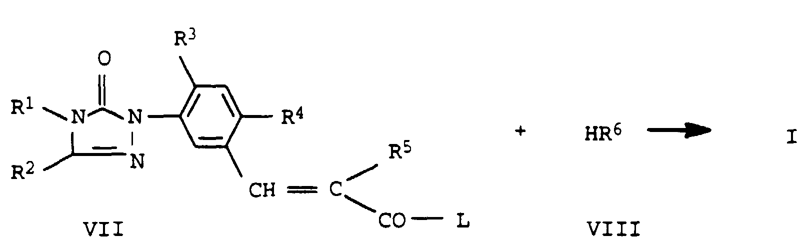

L steht für eine übliche Abgangsgruppe wie Chlor, Brom und 1-Imidazolyl.

Sofern L Chlor oder Brom bedeutet, kann die Anwesenheit einer Base wie Triethylamin und Pyridin empfehlenswert sein.

Als Lösungsmittel eignen sich z.B. halogenierte Kohlenwasserstoffe wie Methylenchlorid und 1,2-Dichlorethan, aromatische Kohlenwasserstoffe und Halogenkohlenwasserstoffe wie Benzol, Toluol und Chlorbenzol, Ether wie Diethylether, Tetrahydrofuran, Methyl-tert.-butylether und Dioxan, dipolar aprotische Lösungsmittel wie Dimethylsulfoxid, Dimethylformamid oder Mischungen derartiger Solventien.

Die Reaktion wird allgemein bei (- 20 °C) bis zur Siedetemperatur des Reaktionsgemisches vorgenommen.

Üblicherweise werden die Ausgangsverbindungen in etwa stöchiometrischen Mengen eingesetzt, sofern es sich nicht empfiehlt, eine der Komponenten im Überschuß einzusetzen.

Die aktivierten Carbonsäuren VII mit L = Chlor oder Brom sind ihrerseits in an sich bekannter Weise durch Umsetzung der entsprechenden Carbonsäuren mit einem Halogenierungsmittel, z.B. mit Thionylchlorid, Thionylbromid, Sulfurylchlorid, Sulfurylbromid, einem organischen Sulfonsäurechlorid wie Tosylchlorid, Phosphortrichlorid, Phosphortribromid, Phosphorpentachlorid, Phosphorpentabromid, Phorphoroxybromid oder mit einem binären Halogenierungssystem wie Tetrachlormethan/Triphenylphosphin und Tetrabrommethan/Triphenyl)phosphin erhältlich.

Die Halogenierung kann ohne Lösungsmittel oder in einem inerten Lösungsmittel durchgeführt werden. Geeignete Lösungsmittel sind - in Abhängigkeit vom Halogenierungsmittel - allgemein z.B. halogenierte Kohlenwasserstoffe wie Methylenchlorid, Chloroform und 1,2-Dichlorethan, aromatische Kohlenwasserstoffe und Halogenkohlenwasserstoffe wie Benzol, Toluol und Chorbenzol, dipolare aprotische Lösungsmittel wie Acetonitril, ferner Schwefelkohlenstoff, Ether wie Diethylether, Methyl-tert.-butylether und Tetrahydrofuran. Auch Mischungen dieser Solventien kommen in Betracht.

Die Halogenierung kann bei (-30) °C bis zur Siedetemperatur des Reaktionsgemisches erfolgen.

Je nach Halogenierungsmittel wird die zu halogenierende Säure zweckmäßigerweise in stöchiometrischer Menge oder im Unterschuß eingesetzt.

Eine Variante des Verfahrens besteht darin, die aktivierte Carbonsäure VII in situ herzustellen, insbesondere wenn L für 1-Imidazolyl steht oder wenn man unter Mitsunobu-Bedingungen arbeitet (vgl. O. Mitsunobu, Synthesis 1981, 1).

Die zu aktivierende Carbonsäure ist nach einem der Verfahren (A) - (C) erhältlich oder durch Verseifung der entsprechenden Ester der Formel I (mit R⁶ = -OR⁷), die ihrerseits nach Verfahren (A), (B) oder (C) hergestellt werden können.

Sofern nicht anders angegeben werden alle vorstehend beschriebenen Verfahren zweckmäßigerweise bei Atmosphärendruck oder unter dem Eigendruck des jeweiligen Reaktionsgemisches vorgenommen.

Die Aufarbeitung der Reaktionsgemische erfolgt in der Regel nach an sich bekannten Methoden, beispielsweise durch Entfernen des Lösungsmittels, Verteilen des Rückstandes in einem Gemisch aus Wasser und einem geeigneten organischen Lösungsmittel und Aufarbeiten der organischen Phase auf das Produkt hin.

Die substituierten Triazolinone der Formel I können ein oder mehrere Chiralitatszentren enthalten und fallen dann üblicherweise als Enantiomeren- oder Diastereomerengemische an. Die Mischungen können gewünschtenfalls nach den hierfür üblichen Methoden, z.B. mittels Kristallisation oder Chromatographie an einem optisch aktiven Adsorbat, in die weitgehend reinen Isomeren getrennt werden. Reine optisch aktive Isomere lassen sich beispielsweise auch aus entsprechenden optisch aktiven Ausgangsmaterialien herstellen.

Insbesondere diejenigen Verbindungen I, bei denen R¹ für Wasserstoff steht und/oder R⁶ Hydroxyl, Mercapto, -NHR⁸ oder -N(R⁸)-OH bedeutet, können auf an sich bekannte Weise in ihre Salze, vorzugsweise in ihre Alkalimetallsalze, übergeführt werden.

Salze der substituierten Triazolinone I, deren Metallion kein Alkalimetallion ist, können durch Umsalzen des entsprechenden Alkalimetallsalzes in üblicher Weise hergestellt werden, ebenso Ammonium- und Phosphoniumsalze mittels Ammoniak, Phosphonium-, Sulfonium- oder Sulfoxoniumhydroxiden.

Die Verbindungen I sowie deren landwirtschaftlich brauchbaren Salze eignen sich - sowohl als Isomerengemische als auch in Form der reinen Isomeren - als Herbizide. Sie können in Kulturen wie Weizen, Reis, Mais, Soja und Baumwolle Unkräuter und Schadgräser sehr gut bekämpfen, ohne die Kulturpflanzen nennenswert zu schädigen. Dieser Effekt tritt vor allem bei niedrigen Aufwandmengen auf.

In Abhängigkeit von der jeweiligen Applikationsmethode können die Verbindungen I bzw. sie enthaltenden herbiziden Mittel noch in einer weiteren Zahl von Kulturpflanzen zur Beseitigung unerwünschter Pflanzen eingesetzt werden. In Betracht kommen beispielsweise folgende Kulturen:

Allium cepa, Ananas comosus, Arachis hypogaea, Asparagus officinalis, Beta vulgaris spp. altissima, Beta vulgaris spp. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spp., Manihot esculenta, Medicago sativa, Musa spp., Nicotiana tabacum (N. rustica), Olea europaea, Oryza sativa , Phaseolus lunatus, Phaseolus vulgaris, Picea abies, Pinus spp., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera und Zea mays.

Darüber hinaus lassen sich die Verbindungen I in Kulturen, die durch Züchtung und/oder mittels gentechnischer Methoden gegen die Wirkung von I weitgehend resistent gemacht wurden, einsetzen.

Des weiteren eignen sich die substituierten Triazolinone I auch zur Desikkation und/oder Defoliation von Pflanzen.

Als Desikkantien eignen sie sich insbesondere zur Austrocknung der oberirdischen Teile von Kulturpflanzen wie Kartoffel, Raps, Sonnenblume und Sojabohne. Damit wird ein vollständig mechanisches Beernten dieser wichtigen Kulturpflanzen ermöglicht.

Von wirtschaftlichem Interesse ist ferner die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, das heißt die Förderung der Ausbildung von Trenngewebe zwischen Frucht- oder Blatt- und Sproßteil der Pflanzen ist auch für ein gut kontrollierbares Entblättern von Nutzpflanzen, insbesondere Baumwolle, wesentlich.

Außerdem führt die Verkürzung des Zeitintervalls, in dem die einzelnen Baumwollpflanzen reif werden, zu einer erhöhten Faserqualität nach der Ernte.

Die Verbindungen I bzw. die sie enthaltenden herbiziden Mittel können beispielsweise in Form von direkt versprühbaren wäßrigen Lösungen, Pulvern, Suspensionen, auch hochprozentigen wäßrigen, Öligen oder sonstigen Suspensionen oder Dispersionen, Emulsionen, Öldispersionen, Pasten, Stäubemitteln, Streumitteln oder Granulaten durch Versprühen, Vernebeln, Verstauben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Als inerte Hilfsstoffe für die Herstellung von direkt versprühbaren Lösungen, Emulsionen, Pasten oder Öldispersionen kommen im wesentlichen in Betracht: Mineralölfraktionen von mittlerem bis hohem Siedepunkt wie Kerosin und Dieselöl, ferner Kohlenteeröle sowie Öle pflanzlichen oder tierischen Ursprungs, aliphatische, cyclische und aromatische Kohlenwasserstoffe, z.B. Paraffine, Tetrahydronaphthalin, alkylierte Naphthaline und deren Derivate, alkylierte Benzole und deren Derivate, Alkohole wie Methanol, Ethanol, Propanol, Butanol und Cyclohexanol, Ketone wie Cyclohexanon, stark polare Lösungsmittel, z.B. Amine wie N-Methylpyrrolidon und Wasser.

Waßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittei und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylen- oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Konzentrationen der Wirkstoffe I in den anwendungsfertigen Zubereitungen können in weiten Bereichen variiert werden, etwa von 0,01 bis 95 Gew. %, vorzugsweise 0,5 bis 90 Gew.%. Die Wirkstoffe werden dabei in einer Reinheit von 90% bis 100%, vorzugsweise 95% bis 100% (nach NMR-Spektrum) eingesetzt.

Die folgenden Formulierungsbeispiele verdeutlichen die Herstellung solcher Zubereitungen:
I. 20 Gewichtsteile der Verbindung Nr. I.01 werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine waßrige Dispersion, die 0,02 Gew.% des Wirkstoffs enthält.
II. 20 Gewichtsteile der Verbindung Nr. I.02 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.
III. 20 Gewichtsteile des Wirkstoffs Nr. I.03 werden in einer Mischung gelöst, die aus 25 Gewichtsteilen Cyclohexanon, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine waßrige Dispersion, die 0,02 Gew.% des Wirkstoffs enthält.
IV. 20 Gewichtsteile des Wirkstoffs Nr. I.04 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser enthält man eine Spritzbrühe, die 0,1 Gew.% des Wirkstoffs enthält.
V. 3 Gewichtsteile des Wirkstoffs Nr. I.05 werden mit 97 Gewichtsteilen feinteiligem Kaolin vermischt. Man erhält auf diese Weise ein Stäubemittel, das 3 Gew.% des Wirkstoffs enthält.
VI. 20 Gewichtsteile des Wirkstoffs Nr. I.02 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

Die Applikation der Wirkstoffe I bzw. der herbiziden Mittel kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgerate so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff I betragen je nach Bekämpfungsziel, Jahreszeit, Zielpflanzen und Wachstumsstadium 0,001 bis 3,0, vorzugsweise 0,01 bis 1 kg/ha aktive Substanz (a.S.).

Zur Verbreiterung des Wirkungsspektrums und zur Erzielung synergistischer Effekte können die substituierten Triazolinone I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, die in 2-Stellung z.B. eine Carboxy- oder Carbimino-Gruppe tragen, Chinolincarbonsäurederivate, Imidazolinone, Sulfonamide, Sulfonylharnstoffe, Aryloxy-, Heteroaryloxyphenoxypropionsäuren sowie deren Salze, Ester und Amide und andere in Betracht.

Außerdem kann es von Nutzen sein, die Verbindungen I allein oder in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

### Herstellungsbeispiel

### 1-[4-Chlor-5-(2-chlor-2-ethoxycarbonyl-ethen-1-yl)-2-fluorphenyl)]-4-difluormethyl-4-methyl-triazolin-5-on (I.02)

Zu einer Suspension von 1,6 g tert.-Butylnitrit, 21,6 g Propiolsäureethylester und 1,6 g wasserfreien Kupfer(II)chlorid in 400 ml absolutem Acetonitril wurden 2,9 g 1-[5-Amino-4-chlor-2-fluorphenyl)]-4-difluormethyl-4-methyl-triazolin-5-on in 20 ml absolutem Acetonitril getropft. Nach 12 Std. Rühren bei 20-25 °C versetzte man die Reaktionsmischung mit verdünnter wäßriger Salzsäure. Anschließend extrahierte man das Produkt mit Methyl-tert.-butylether. Die vereinigten organischen Phasen wurden über Natriumsulfat getrocknet, wonach man das Lösungsmittel bei reduziertem Druck entfernte. Der Rückstand wurde chromatographisch an Kieselgel gereinigt (Eluent: Cyclohexan/Methyl-tert.-butylether = 9:1). Ausbeute: 1,5 g (37 %) [cis/trans-Gemisch]
¹H-NMR (in CDCl₃; Tetramethylsilan (TMS) als interner Standard): δ [ppm] = 1,14/1,40 (t,3H); 2,45/2,48 (s,3H); 4,16/4,34 (q,2H); 7,03/7,07 (t,lH); 7,31/7,40 (d,1H); 7,38/8,06 (s,1H); 7,48/8,18 (d,lH).

In Tabelle 3 sind noch weitere Triazolinone der Formel I aufgeführt, die auf die gleiche Weise hergestellt wurden oder herstellbar sind:

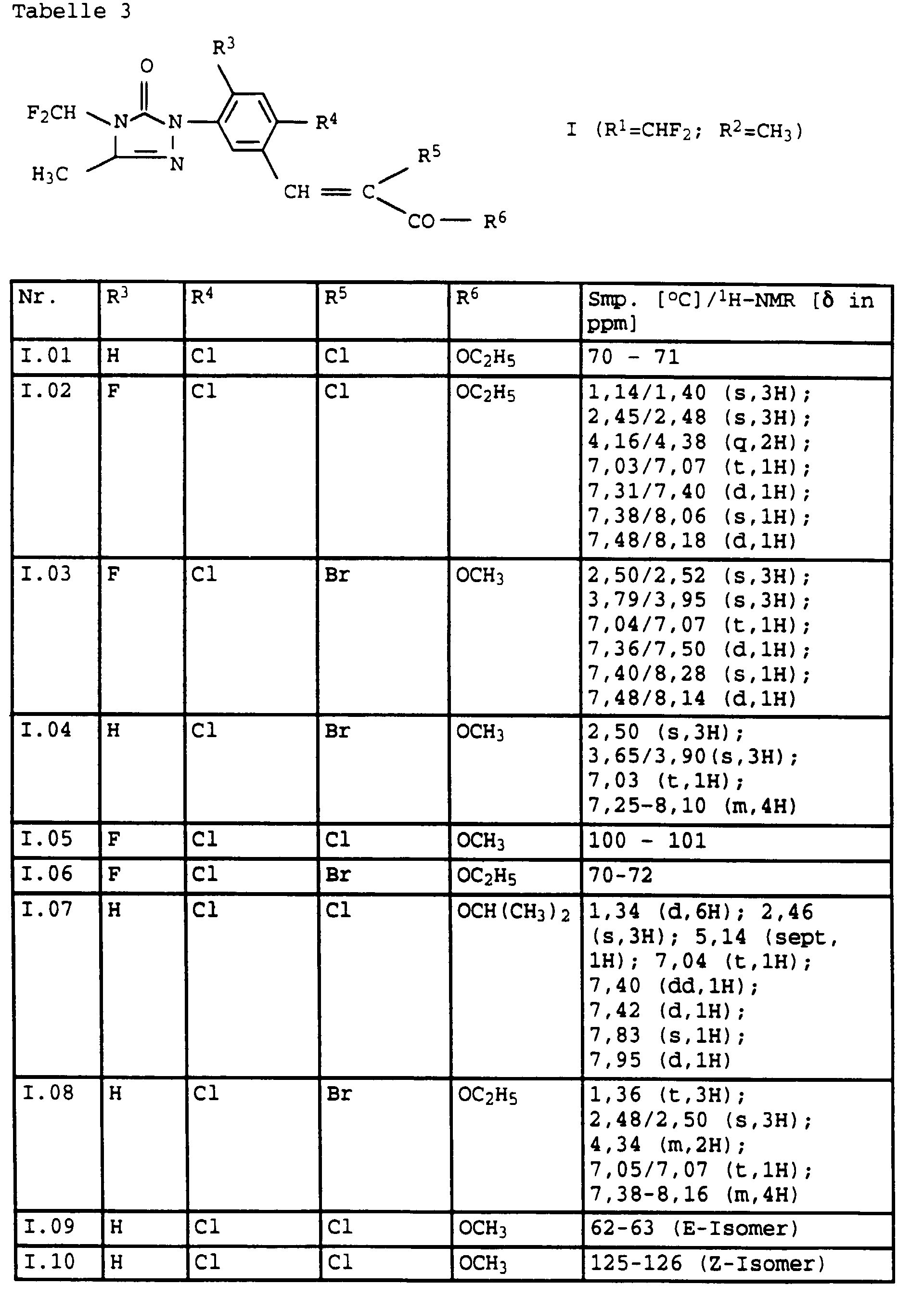

### Anwendungsbeispiele (herbizide Wirksamkeit)

Die herbizide Wirkung der substituierten Triazolinone I ließ sich durch die folgenden Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und erst dann mit den in Wasser suspendierten oder emulgierten Wirkstoffen behandelt. Die Testpflanzen wurden dafür entweder direkt gesät und in den gleichen Gefäßen aufgezogen oder sie werden erst als Keimpflanzen getrennt angezogen und einige Tage vor der Behandlung in die Versuchsgefäße verpflanzt. Die Aufwandmenge für die Nachauflaufbehandlung betrug 0,0156 oder 0,0078 kg/ha a.S. (aktive Substanz).

Die Pflanzen wurden artenspezifisch bei Temperaturen von 10 - 25°C bzw. 20 - 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewachshausversuchen verwendeten Pfianzen setzten sich aus folgenden Arten zusammen:

| | | |
|---|---|---|
| Lateinischer Name | Deutscher Name | Englischer Name |
| Amaranthus retroflexus | Zurückgekrümmter Ackerfuchsschwanz | redroot pigweed |
| Setaria faberii | Borstenhirse | giant foxtail |
| Setaria viridis | Grüne Borstenhirse | green foxtail |
| Veronica subspecies | Ehrenpreisarten | speedwell |

Bei einer Aufwandmenge von 0,0078 oder 0,0156 kg/ha a.S. zeigte die Verbindung Nr. I.02 im Nachauflaufverfahren eine sehr gute Wirkung gegen die o.g. Pflanzen. Die aus der WO 90/02120 bekannte und vergleichend ebenfalls auf ihre herbizide Wirkung hin getestete Verbindung 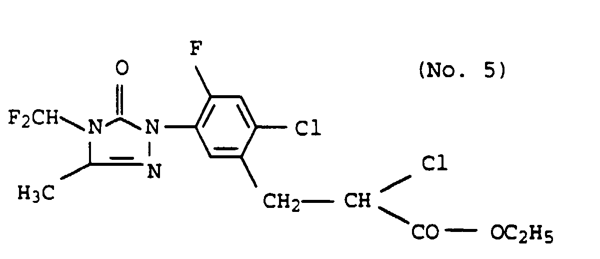 war dagegen viel weniger wirksam.

### Anwendungsbeispiele (desikkative/defoliante Wirksamkeit)

Als Testpflanzen dienten junge, 4-blättrige (ohne Keimblätter) Baumwollpflanzen, die unter Gewächshausbedingungen angezogen wurden (rel. Luftfeuchtigkeit 50 bis 70 %; Tag-/Nachttemperatur 27/20 °C).

Die jungen Baumwollpflanzen wurden tropfnaß mit wäßrigen Aufbereitungen der Wirkstoffe (unter Zusatz von 0,15 Gew.-% des Fettalkoholalkoxylats Plurafac LF 700, bezogen auf die Spritzbrühe) blattbehandelt. Die ausgebrachte Wassermenge betrug umgerechnet 1000 l/ha. Nach 13 Tagen wurde die Anzahl der abgeworfenen Blätter und der Grad der Entblätterung in % bestimmt.

Bei den unbehandelten Kontrollpflanzen trat kein Blattfall auf.

## Patentansprüche

1. Substituierte Triazolinone der allgemeinen Formel I 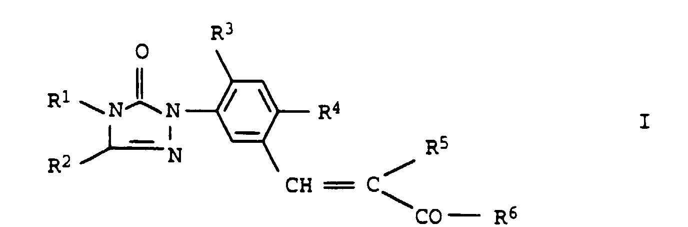 in der die Substituenten folgende Bedeutung haben:
R¹ Wasserstoff, C₁-C₆-Alkyl oder C₁-C₆-Halogenalkyl;
R² Wasserstoff, C₁-C₆-Alkyl oder C₁-C₆-Halogenalkyl;
R³ Wasserstoff oder Halogen;
R⁴ Cyano, Halogen, C₁-C₆-Alkyl, C₁-C₆-Halogenalkyl, C₁-C₆-Alkoxy oder C₁-C₆-Halogenalkoxy;
R⁵ Nitro, Cyano oder Halogen;
R⁶ -OR⁷, -SR⁷, -N(R⁸)-R⁹ oder -N(R⁸)-OR¹⁰, wobei
R⁷ für Wasserstoff, C₁-C₆-Alkyl, C₃-C₆-Alkenyl, C₃-C₆-Alkinyl, C₃-C₆-Cycloalkyl, C₁-C₆-Halogenalkyl, Cyano-C₁-C₆-alkyl, C₁-C₆-Alkoxy-C₁-C₆-alkyl, C₁-C₆-Alkylthio-C₁-C₆-alkyl, (C₁-C₆-Alkoxy)carbonyl-C₁-C₆-alkyl, (C₁-C₆-Alkylamino)carbonyl-C₁-C₆-alkyl, Di-(C₁-C₆-Alkyl)aminocarbonyl-C₁-C₆-alkyl, C₁-C₆-Alkyloximino-C₁-C₆-alkyl, Di-(C₁-C₆-alkoxy)-C₂-C₆-alkyl, Di-(C₁-C₆-alkylthio)-C₂-C₆-alkyl, C₃-C₆-Halogenalkenyl, Phenyl oder Benzyl, wobei die Phenylringe jeweils ein bis drei Reste tragen können, ausgewählt aus der Gruppe bestehend aus Cyano, Nitro, Halogen, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio und (C₁-C₆-Alkoxy)carbonyl;
R⁸ und R⁹ unabhängig voneinander für (C₁-C₆-Alkyl)carbonyl, (C₁-C₆-Halogenalkyl)carbonyl oder eine der für R⁷ genannten Bedeutungen, oder
R⁸ und R⁹ gemeinsam für eine vier- bis 6-gliedrige Kohlenstoffkette, in der eine nicht endständige Methyleneinheit durch Sauerstoff oder eine C₁-C₄-Alkyliminogruppe ersetzt sein kann und
R¹⁰ für (C₁-C₆-Alkyl)carbonyl, (C₁-C₆-Halogenalkyl)carbonyl oder eine der für R⁷ genannten Bedeutungen stehen,
sowie die landwirtschaftlich brauchbaren Salze von I.

2. Substituierte Triazolinone der Formel I nach Anspruch 1, wobei
R¹ Wasserstoff, Methyl oder C₁-C₆-Halogenalkyl und
R² Wasserstoff, C₁-C₆-Alkyl oder C₁-C₄-Halogenalkyl bedeuten.

3. Verfahren zur Herstellung von substituierten Trazolinonen der Formel I gemäß Anspruch 1, wobei R⁵ für Chlor oder Brom steht, dadurch gekennzeichnet, daß man ein Anilin der Formel II 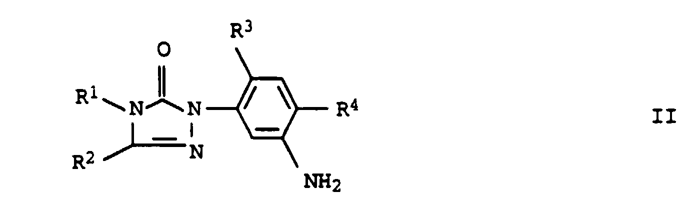 und ein Alkin III 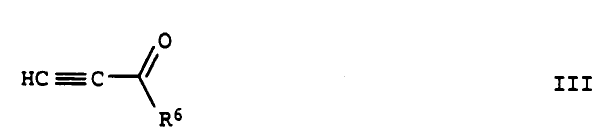 auf an sich bekannte Weise einer Arylierungsreaktion nach Meerwein unterwirft.

4. Verfahren zur Herstellung von substituierten Triazolinonen der Formel I gemaß Anspruch 1, wobei R⁵ für Chlor oder Brom steht, dadurch gekennzeichnet, daß man einen substituierten Benzaldehvd der Formel IV 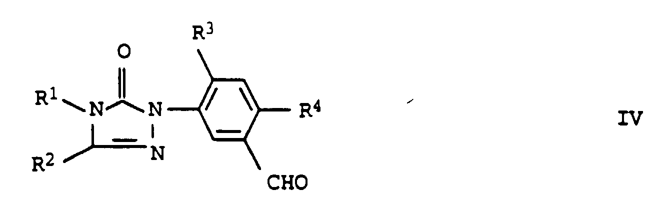 mit einem Ylid der Formel V 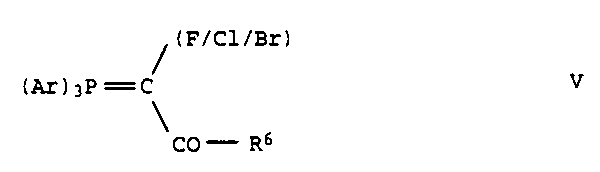 wobei Ar für einen aromatischen Rest steht, der gewünschtenfalls substituiert sein kann, umsetzt.

5. Verfahren zur Herstellung von substituierten Triazolinonen der Formel I gemäß Anspruch 1, wobei R⁵ für Nitro oder Cyano steht, dadurch gekennzeichnet, daß man einen substituierten Benzaldeyhd der Formel IV 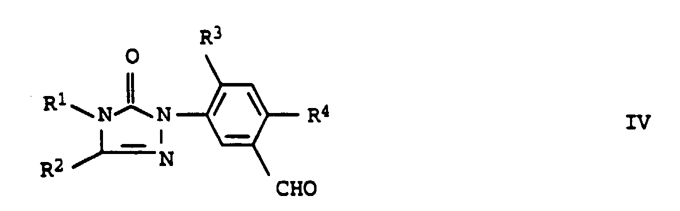 auf an sich bekannte Weise mit einer CH-aciden Verbindung NO₂-CH₂-CO-R⁶ oder NC-CH₂-CO-R⁶ (VI) umsetzt.

6. Verfahren zur Herstellung von substituierten Triazolinonen der Formel I gemäß Anspruch 1, dadurch gekennzeichnet, daß man eine aktivierte Carbonsäure der Formel VII, 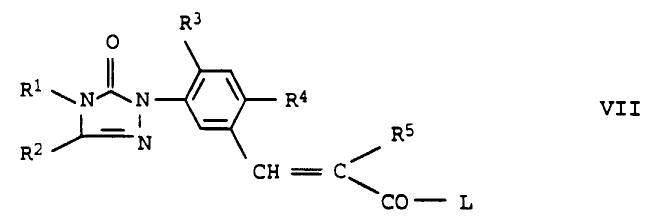 wobei L für eine übliche Abgangsgruppe steht, auf an sich bekannte Weise mit einem Nukleophil H-R⁶ (VIII) umsetzt.

7. Verwendung der substituierten Triazolinone der Formel I und deren landwirtschaftlich brauchbaren Salze von I, gemäß Anspruch 1, als Herbizide oder zur Desikkation und/oder Defoliation von Pflanzen.

8. Herbizides Mittel, enthaltend eine herbizid wirksame Menge mindestens eines substituierten Triazolinon der Formel I oder ein landwirtschaftlich brauchbares Salz von I, gemäß Anspruch 1, und mindestens einen inerten flüssigen und/oder festen Tragerstoff sowie gewünschtenfalls mindestens ein Adjuvans.

9. Mittel zur Desikkation und/oder Defoliation von Pflanzen, enthaltend eine desikkant und/oder defoliant wirksame Menge mindestens eines substituierten Triazolinons der Formel I oder ein landwirtschaftlich brauchbares Salz von I, gemäß Anspruch 1, und mindestens einen inerten flüssigen und/oder festen Trägerstoff sowie gewünschtenfalls mindestens ein Adjuvans.

10. Verfahren zur Herstellung von herbizid wirksamen Mitteln, dadurch gekennzeichnet, daß man eine herbizid wirksame Menge mindestens eines substituierten Triazolinons der Formel I oder ein landwirtschaftlich brauchbares Salz von I, gemäß Anspruch 1, und mindestens einen inerten flüssigen und/oder festen Trägerstoff sowie gewünschtenfalls mindestens ein Adjuvans mischt.

11. Verfahren zur Herstellung von desikkant und/oder defoliant wirksamen Mitteln, dadurch gekennzeichnet, daß man eine desikkant und/oder defoliant wirksame Menge mindestens eines substituierten Triazolinons der Formel I oder ein landwirtschaftlich brauchbares Salz von I, gemäß Anspruch 1, und mindestens einen inerten flüssigen und/oder festen Trägerstoff sowie gewünschtenfalls mindestens ein Adjuvans mischt.

12. Verfahren zur Bekämpfung von unerwünschten Pflanzenwuchs, dadurch gekennzeichnet, daß man eine herbizid wirksame Menge mindestens eines substituierten Triazolinons der Formel I oder eines landwirtschaftlich brauchbaren Salzes von I, gemäß Anspruch 1, auf Pflanzen, deren Lebensraum oder auf Saatgut einwirken laßt.

13. Verfahren zur Desikkation und/oder Defoliation von Pflanzen, dadurch gekennzeichnet, daß man eine desikkant und/oder defoliant wirksame Menge mindestens eines substituierten Triazolinons der Formel I oder eines landwirtschaftlich brauchbaren Salzes von I, gemaß Anspruch 1, auf Pflanzen einwirken laßt.

## Claims

1. A substituted triazolinone of the formula I 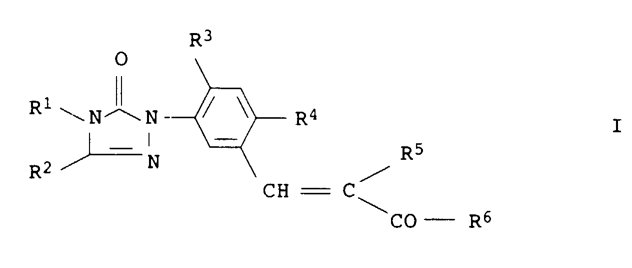 where
R¹ is hydrogen, C₁-C₆-alkyl or C₁-C₆-haloalkyl;
R² is hydrogen, C₁-C₆-alkyl or C₁-C₆-haloalkyl;
R³ is hydrogen or halogen;
R⁴ is cyano, halogen, C₁-C₆-alkyl, C₁-C₆-haloalkyl, C₁-C₆-alkoxy or C₁-C₆-haloalkoxy;
R⁵ is nitro, cyano or halogen;
R⁶ is -OR⁷, -SR⁷, -N(R⁸)-R⁹ or -N(R⁸)-OR¹⁰,
R⁷ is hydrogen, C₁-C₆-alkyl, C₃-C₆-alkenyl, C₃-C₆-alkynyl, C₃-C₆-cycloalkyl, C₁-C₆-haloalkyl, cyano-C₁-C₆-alkyl, C₁-C₆-alkoxy-C₁-C₆-alkyl, C₁-C₆-alkylthio-C₁-C₆-alkyl, (C₁-C₆-alkoxy)carbonyl-C₁-C₆-alkyl, (C₁-C₆-alkylamino)carbonyl-C₁-C₆-alkyl, di-(C₁-C₆-alkyl)aminocarbonyl-C₁-C₆-alkyl, C₁-C₆-alkyloximino-C₁-C₆-alkyl, di-(C₁-C₆-alkoxy)-C₂-C₆-alkyl, di-(C₁-C₆-alkylthio)-C₂-C₆-alkyl, C₃-C₆-haloalkenyl, phenyl or benzyl, where each of the phenyl rings may carry one to three radicals selected from the group consisting of cyano, nitro, halogen, C₁-C₆-alkyl, C₁-C₆-haloalkyl, C₁-C₆-alkoxy, C₁-C₆-haloalkoxy, C₁-C₆-alkylthio, C₁-C₆-haloalkylthio and (C₁-C₆-alkoxy)carbonyl;
R⁸ and R⁹, independently of one another, are each (C₁-C₆-alkyl)carbonyl, (C₁-C₆-haloalkyl)carbonyl or have one of the meanings stated for R⁷, or
R⁸ and R⁹ together form a four-membered to 6-membered carbon chain in which a nonterminal methylene unit may be replaced by oxygen or C₁-C₄-alkylimino and
R¹⁰ is (C₁-C₆-alkyl)carbonyl or (C₁-C₆-haloalkyl)carbonyl or has one of the meanings stated for R⁷,
and the agriculturally useful salts of I.

2. A substituted triazolinone of the formula I as claimed in claim 1, wherein
R¹ is hydrogen, methyl or C₁-C₆-haloalkyl and
R² is hydrogen, C₁-C₆-alkyl or C₁-C₄-haloalkyl.

3. A process for the preparation of a substituted triazolinone of the formula I as claimed in claim 1, where R⁵ is chlorine or bromine, wherein an aniline of the formula II 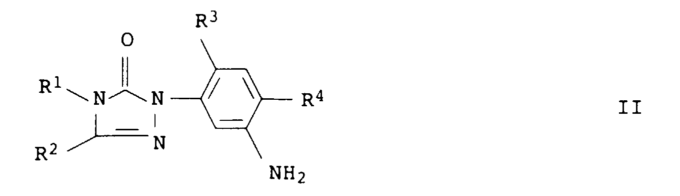 and an alkyne III 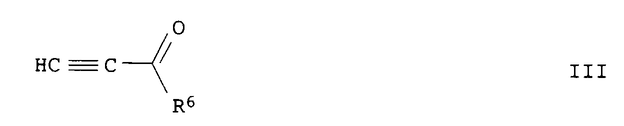 are subjected to a Meerwein alkylation reaction in a manner known per se.

4. A process for the preparation of a substituted triazolinone of the formula I as claimed in claim 1, where R⁵ is chlorine or bromine, wherein a substituted benzaldehyde of the formula IV 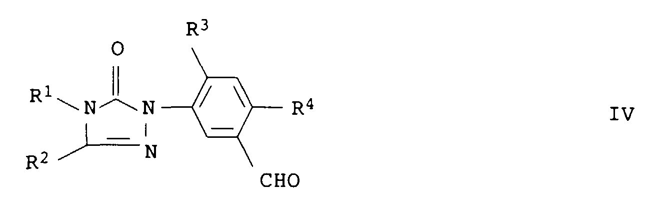 is reacted with an ylide of the formula V 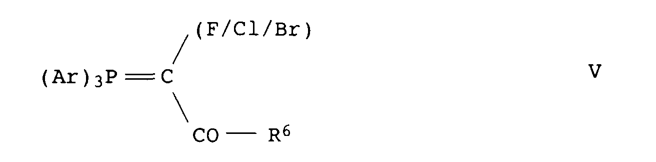 where Ar is an aromatic radical which, if desired, may be substituted.

5. A process for the preparation of a substituted triazolinone of the formula I as claimed in claim 1, where R⁵ is nitro or cyano, wherein a substituted benzaldehyde of the formula IV 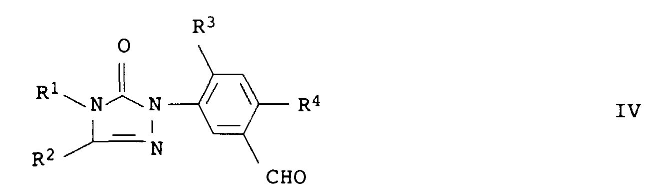 is reacted with a CH-acidic compound NO₂-CH₂-CO-R⁶ or NC-CH₂-CO-R⁶ (VI) in a manner known per se.

6. A process for the preparation of a substituted triazolinone of the formula I as claimed in claim 1, wherein an activated carboxylic acid of the formula VII 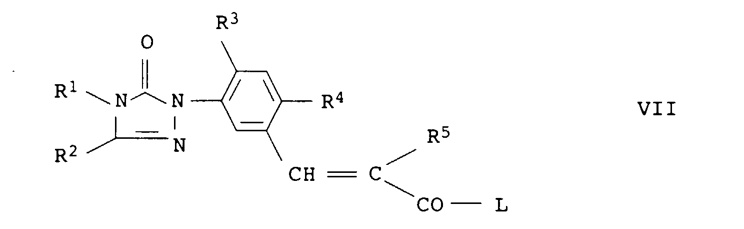 where L is a conventional leaving group, is reacted with a nucleophile H-R⁶ (VIII) in a manner known per se.

7. Use of a substituted triazolinone of the formula I or of an agriculturally useful salt of I, as claimed in claim 1, as a herbicide or for the desiccation or defoliation of plants.

8. A herbicide containing a herbicidal amount of at least one substituted triazolinone of the formula I or an agriculturally useful salt of I, as claimed in claim 1, and at least one inert liquid or solid carrier and, if desired, at least one adjuvant.

9. A plant desiccant or defoliant containing an amount, having a desiccant or defoliant action, of at least one substituted triazolinone of the formula I or an agriculturally useful salt of I, as claimed in claim 1, and at least one inert liquid or solid carrier and, if desired, at least one adjuvant.

10. A process for the preparation of a herbicide, wherein a herbicidal amount of at least one substituted triazolinone of the formula I or an agriculturally useful salt of I, as claimed in claim 1, and at least one inert liquid or solid carrier and, if desired, at least one adjuvant are mixed.

11. A process for the preparation of a desiccant or defoliant, wherein an amount, having a desiccant or defoliant action, of at least one substituted triazolinone of the formula I or an agriculturally useful salt of I, as claimed in claim 1, and at least one inert liquid or solid carrier and, if desired, at least one adjuvant are mixed.

12. A method for controlling undesirable plant growth, wherein a herbicidal amount of at least one substituted triazolinone of the formula I or of an agriculturally useful salt of I, as claimed in claim 1, is allowed to act on plants or on their habitat or on seed.

13. A method for desiccation or defoliation of plants, wherein an amount, having a desiccant or defoliant action, of at least one substituted triazolinone of the formula I or of an agriculturally useful salt of I, as claimed in claim 1, is allowed to act on plants.

## Revendications

1. Triazolinones substituées répondant à la formule générale I 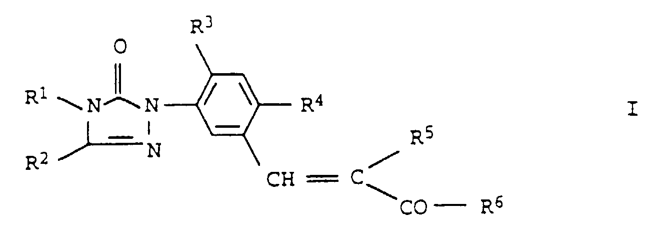 dans laquelle les symboles ont les significations suivantes :
R¹ : l'hydrogène, un groupe alkyle en C1-C6 ou halogénoalkyle en C1-C6;
R² : l'hydrogène, un groupe alkyle en C1-C6 ou halogénoalkyle en C1-C6;
R³ : l'hydrogène ou un halogène ;
R⁴ : un groupe cyano, un halogène, un groupe alkyle en C1-C6, halogénoalkyle en C1-C6, alcoxy en C1-C6 ou halogénoalcoxy en C1-C6;
R⁵ : un groupe nitro, cyano ou un halogène ;
R⁶ : -OR⁷, -SR⁷, -N(R⁸)-R⁹ ou -N(R⁸)-OR¹⁰ dans lesquels
R⁷ : représente l'hydrogène, un groupe alkyle en C1-C6, alcényle en C3-C6, alcynyle en C3-C6, cycloalkyle en C3-C6, halogénoalkyle en C1-C6, cyano-alkyle en C1-C6, (alcoxy en C1-C6)alkyle en C1-C6, (alkylthio en C1-C6)alkyle en C1-C6, (alcoxy en C1-C6)carbonyl-alkyle en C1-C6, (alkylamino en C1-C6)carbonyl-alkyle en C1-C6, di-(alkyle en C1-C6)aminocarbonyl-alkyle en C1-C6, (alkyloximino en C1-C6)alkyle en C1-C6, di-(alcoxy en C1-C6)alkyle en C2-C6, di-(alkylthio en C1-C6)alkyle en C2-C6, halogénoalcényle en C3-C6, phényle ou benzyle, chacun des noyaux phényle pouvant porter un à trois substituants choisis parmi les groupes cyano, nitro, les halogènes, les groupes alkyle en C1-C6, halogénoalkyle en C1-C6, alcoxy en C1-C6, halogénoalcoxy en C1-C6, alkylthio en C1-C6, halogénoalkylthio en C1-C6 et (alcoxy en C1-C6)carbonyle ;
R⁸ et R⁹ représentent chacun, indépendamment l'un de l'autre, un groupe (alkyle en C1-C6)carbonyle, (halogénoalkyle en C1-C6)carbonyle ou a l'une des significations indiquées pour R⁷, ou bien
R⁸ et R⁹, ensemble, représentent une chaîne carbonée de quatre à six chaînons dans laquelle un motif méthylène non terminal peut être remplacé par l'oxygène ou un groupe alkylimino en C1-C4 et
R¹⁰ représente un groupe (alkyle en C1-C6)carbonyle, (halogénoalkyle en C1-C6)carbonyle ou a l'une des significations indiquées R⁷,
et les sels de I acceptables pour les applications agricoles.

2. Triazolinones substituées de formule I de la revendication 1 dans laquelle
R¹ représente l'hydrogène, un groupe méthyle ou halogénoalkyle en C1-C6 et
R² représente l'hydrogène, un groupe alkyle en C1-C6 ou halogénoalkyle en C1-C4.

3. Procédé de préparation des triazolinones substituées de formule I de la revendication 1 dans laquelle R⁵ représente le chlore ou le brome, caractérisé par le fait que l'on soumet une aniline de formule II 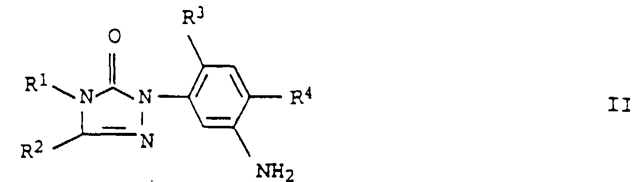 et un alcyne III 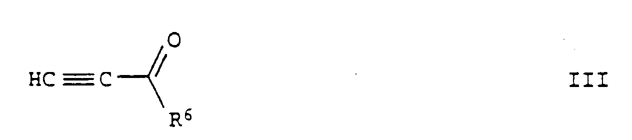 de manière connue en soi, à une réaction d'arylation selon Meerwein.

4. Procédé de préparation des triazolinones substituées de formule I de la revendication 1 dans laquelle R⁵ représente le chlore ou le brome, caractérisé par le fait que l'on fait réagir un benzaldéhyde substitué de formule IV 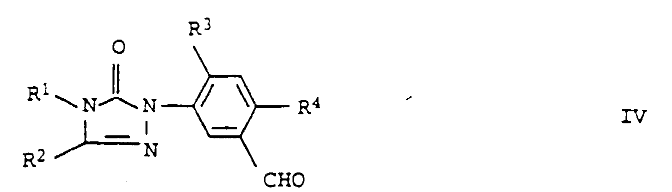 avec un ylide de formule V 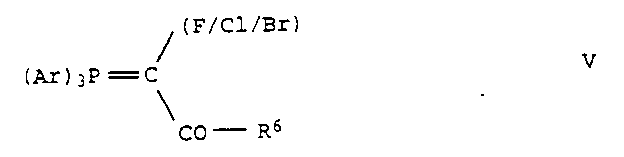 dans laquelle Ar représente un radical aromatique qui peut le cas échéant être substitué.

5. Procédé de préparation des triazolinones substituées de formule I de la revendication 1 dans laquelle R⁵ représente un groupe nitro ou cyano, caractérisé par le fait que l'on fait réagir un benzaldéhyde substitué de formule IV 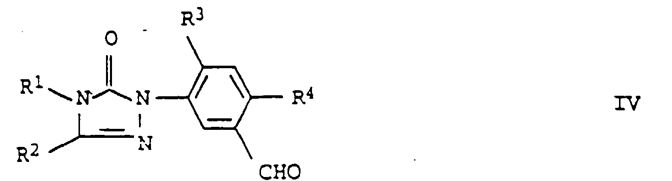 de manière connue en soi, avec un composé à CH acide NO₂-CH₂-CO-R⁶ ou NC-CH₂-CO-R⁶ (VI).

6. Procédé de préparation des triazolinones substituées de formule I de la revendication 1, caractérisé par le fait que l'on fait réagir un acide carboxylique activé de formule VII 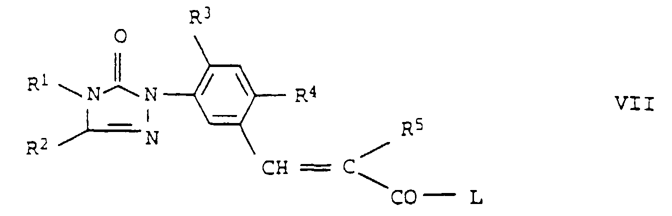 dans laquelle L représente un groupe éliminable usuel, de manière connue en soi, dans un nucléophile H-R⁶ (VIII).

7. Utilisation des triazolinones substituées de formule I et de leurs sels acceptables pour les applications agricoles selon la revendication 1 en tant qu'herbicides ou pour la dessiccation et/ou la défoliation de végétaux.

8. Produit herbicide contenant une quantité herbicide efficace d'au moins une triazolinone substituée de formule I ou de l'un de ses sels acceptables pour des applications agricoles selon la revendication 1 et au moins un véhicule liquide et/ou solide inerte ainsi que, le cas échéant, au moins un adjuvant.

9. Produit pour la dessiccation et/ou la défoliation de végétaux, contenant une quantité dessiccante et/ou défoliante efficace d'au moins une triazolinone substituée de formule I ou de l'un de ses sels acceptables pour les applications agricoles selon la revendication 1 et au moins un véhicule liquide et/ou solide inerte ainsi que, le cas échéant, au moins un adjuvant.

10. Procédé de préparation de produits herbicides, caractérisé par le fait que l'on mélange une quantité herbicide efficace d'au moins une triazolinone substituée de formule I ou de l'un de ses sels acceptable pour les applications agricoles selon la revendication 1 et au moins un véhicule liquide et/ou solide inerte ainsi que, le cas échéant, au moins un adjuvant.

11. Procédé de préparation de produits dessiccants et/ou défoliants, caractérisé par le fait que l'on mélange une quantité dessiccante et/ou défoliante efficace d'au moins une triazolinone substituée de formule I ou de l'un de ses sels acceptable pour des applications agricoles selon la revendication 1 et au moins un véhicule liquide et/ou solide inerte ainsi que, le cas échéant, au moins un adjuvant.

12. Procédé pour combattre les croissances de végétaux indésirables, caractérisé par le fait que l'on fait agir sur les végétaux, leur habitat ou leurs semences une quantité herbicide efficace d'au moins une triazolinone substituée de formule I ou de l'un de ses sels acceptable pour les applications agricoles selon la revendication 1.

13. Procédé pour la dessiccation et/ou la défoliation des végétaux, caractérisé par le fait que l'on fait agir sur les végétaux une quantité dessiccante et/ou défoliante efficace d'au moins une triazolinone substituée de formule I ou de l'un de ses sels acceptable pour les applications agricoles selon la revendication 1.
